# EUROPEAN PATENT SPECIFICATION

(11) **EP 3 784 232 B1**
(45) Date of publication and mention of the grant of the patent: **11.02.2026**
(21) Application number: 19791528.3
(22) Date of filing: 23.04.2019
(51) Int. Cl.: A61K 31/404, A61K 31/428, A61K 31/4375, A61K 9/06

(54) **IMPROVED METHODS FOR INDUCING TISSUE REGENERATION AND SENOLYSIS IN MAMMALIAN CELLS**
VERBESSERTE VERFAHREN ZUR INDUZIERUNG DER GEWEBEREGENERATION UND DER SENOLYSE BEI SÄUGETIERZELLEN
MÉTHODES AMÉLIORÉS POUR INDUIRE UNE RÉGÉNÉRATION TISSULAIRE ET UNE SÉNOLYSE DANS DES CELLULES DE MAMMIFÈRE

(30) Priority: 23.04.2018 US 201862661322 P
(43) Date of publication of application: 03.03.2021
(73) Proprietor: AgeX Therapeutics, Inc., Alameda, CA 94501 (US)
(72) Inventor: WEST, Michael, D., Mill Valley, CA 94941 (US); STERNBERG, Hal, Berkeley, CA 94703 (US)
(74) Representative: Harrison IP Limited
(86) International application number: PCT/US2019/028816
(87) International publication number: WO 2019/209892

(56) References cited:
- WO-A1-2007/058671
- WO-A1-2012/065065
- WO-A1-2017/214342
- US-A1- 2015 017 718
- US-B1- 6 667 176
- QIN HUA ET AL: "Small molecules for reprogramming and transdifferentiation", CMLS CELLULAR AND MOLECULAR LIFE SCIENCES, BIRKHAUSER VERLAG, HEIDELBERG, DE, vol. 74, no. 19, 11 July 2017 (2017-07-11), pages 3553 - 3575, XP036314164, ISSN: 1420-682X, [retrieved on 20170711], DOI: 10.1007/S00018-017-2586-X
- DHRUBA BISWAS ET AL: "Chemically Induced Reprogramming of Somatic Cells to Pluripotent Stem Cells and Neural Cells", INTERNATIONAL JOURNAL OF MOLECULAR SCIENCES, vol. 17, no. 2, 6 February 2016 (2016-02-06), pages 226, XP055484588, DOI: 10.3390/ijms17020226

## Description

### FIELD OF THE INVENTION

The present invention relates to compositions and methods for screening for novel therapeutic formulations for treating medical conditions including aging, degenerative disease, and cancer through the modulation of molecular pathways regulating regeneration and senolysis by means of altering the embryonic-fetal and prenatal/postnatal transitional states of mammalian cells.

### BACKGROUND

Advances in stem cell technology, such as the isolation and propagation *in vitro* of human pluripotent stem (hPS), including but not limited to human embryonic stem (hES) and human induced pluripotent stem (hiPS) cells, constitute an important new area of medical research. hPS cells have a demonstrated potential to be propagated in the undifferentiated state or alternatively to be induced to differentiate into any and all of the cell types in the human body (Thomson et al., Science 282:1145-1147 (1998)). The unique intrinsic capacity of hPS cells to differentiate into all somatic cell types logically provides a platform for the manufacture of transplantable hPS-derived cells of similar diversity for the treatment for a wide variety of degenerative diseases. While this pluripotency of hES and hiPS cells is currently widely recognized, less recognized, and rarely studied, is the unique capacity of hPS cells cultured *in vitro* to generate relatively undifferentiated embryonic anlagen.

Even more rarely studied is the potential of hPS cell-derived cells to differentiate into recognized cell types such as cardiomyocytes or osteochondral cells that nevertheless display subtle prenatal, or even prefetal patterns of gene expression that distinguish them from fetal or adult counterparts. Immediately prior to the embryonic-fetal transition (EFT) *in vivo,* mammalian differentiated cells and tissues such as the skin, heart, and spinal cord show a profound scarless regenerative potential that is progressively lost subsequent to the EFT. In the case of some tissues, such as the human heart, potential for scarless regeneration is detectable for approximately a week past the prenatal-postnatal transition (PPT) period. Given the importance of understanding and modulating tissue regeneration and tissue growth for the fields of regenerative medicine and oncology, improved methods for modelling and modulating the biology *in vitro* and *in vivo* have significant potential utility in research and clinical practice.

Small molecules for reprogramming and transdifferentiation of pluripotency induced by transcription factors can generate induced pluripotent stem cells, adult stem cells or specialized cells (Qin Hua et al. CMLS Cellular and Molecular Life Sciences, Birkhuaser Verlag, Heidelberg, DE, Vol 74, no 19, July 2017, pages 3553-3575.

The ability to generate transplantable neural cells in a large quantity in the laboratory is a critical step in the field of developing stem cell regenerative medicine for neural repair (Dhruba Biswas et al. International Journal of Molecular Sciences, Vol 17, no 2, 6 February 2016, page 226).

The possibility to alter the genetic profile of mammalian cells is disclosed in WO2107/214342 and WO2012/065065.

The potential of pluripotent stem cells and derived embryoid bodies for *in vitro* self-assembly into 3-dimensional organoids has generated interest as a potential pathway for both obtaining tissue for transplantation (Singh et al, Stem Cells Dev. 2015. 24(23): 2778-95) as well as modeling human embryonic development. The present invention teaches that said organoid formation is a reflection of the intrinsic potential of cells prior to the EFT to undergo tissue generation and/or regeneration. In contrast to embryonic cells, fetal and adult-derived cells often show reduced potential for organogenesis *in vitro* and epimorphic regeneration *in vivo.* Epimorphic regeneration, sometimes referred to as "epimorphosis," refers to a type of tissue regeneration wherein a blastema of relatively undifferentiated mesenchyme proliferates at the site of injury and then the cells differentiate to restore the original tissue histology. The developmental timing of the loss of epimorphic potential cannot be fixed precisely, and likely varies with tissue type, nevertheless, the EFT which occurs at about the end of eight weeks of human development (Carnegie Stage 23; O'Rahilly, R., F. Miller (1987) Developmental Stages in Human Embryos, Including a Revision of Streeter's 'Horizons' and a Survey of the Carnegie Collection. Washington, Carnegie Institution of Washington) appears to temporally correspond to the loss of skin regeneration in placental mammals (Walmsley, G.G. et al 2015. Scarless Wound Healing: Chasing the Holy Grail Plast Reconstr Surg. 135(3):907-17). Correlations between species show increased regenerative potential in the embryonic or larval state (reviewed in Morgan, T.H. (1901). Regeneration (New York: The MacMillan Company); also Sanchez Alvarado, A., and Tsonis, P.A. (2006) Bridging the regeneration gap: genetic insights from diverse animal models. Nat. Rev. Genet. 7, 873-884). This suggests that tissue regeneration, as opposed to scarring, reflects the presence of an embryonic as opposed to fetal or adult phenotype, though there is currently no consensus in the scientific community that epimorphic tissue regeneration is a result of an embryonic (pre-natal, more specifically, pre-fetal) pattern of gene expression. In the case of some species, a change in developmental timing (heterochrony) correlates with profound regenerative potential such as is the case in the developmental arrest in larval development (heterochrony) and limb regeneration observed in the Mexican salamander axolotl (*A. mexicanum*) (Voss, S.R. et al, Thyroid hormone responsive QTL and the evolution of paedomorphic salamanders. Heredity (2012) 109, 293-298.

Despite these observations, there are limited markers of the EFT and methods to test the role of specific molecules in regulating the EFT for the treatment of degenerative disease or cancer. We previously disclosed compositions and methods related to markers of the EFT in mammalian species and their use in modulating tissue regeneration and cancer diagnosis described in "Compositions and Methods for Induced Tissue Regeneration in Mammalian Species" (international patent application publication number WO 2014/197421) and "Improved Methods for Detecting and Modulating the Embryonic-Fetal Transition in Mammalian Species" (international patent application publication number WO 2017/214342). The aforementioned compositions and methods were based in part on the methods allowing the clonal expansion of hPS cell-derived embryonic progenitor cell lines which provide a means to propagate novel diverse and highly purified cell lineages with a pre-natal pattern of gene expression useful for regenerating tissues such as skin in a scarless manner. Such cell types have important applications in research, and for the manufacture of cell-based therapies (see PCT application Ser. No. PCT/US2006/013519 filed on April 11, 2006 and titled "Novel Uses of Cells With Prenatal Patterns of Gene Expression"; U.S. patent application Ser. No. 11/604,047 filed on November 21, 2006 and titled "Methods to Accelerate the Isolation of Novel Cell Strains from Pluripotent Stem Cells and Cells Obtained Thereby"; and U.S. patent application Ser. No. 12/504,630 filed on July 16, 2009 and titled "Methods to Accelerate the Isolation of Novel Cell Strains from Pluripotent Stem Cells and Cells Obtained Thereby). Nevertheless, additional and improved methods and compositions for modulating the EFT for the treatment of degenerative disease and cancer are needed. The present invention teaches compositions and methods related to the detection and modulation of EFT for scarless mammalian regenerative therapy for diverse applications including but not limited to aging and age-related degenerative disease and diverse types of malignancy.

### SUMMARY

The inventors of the present invention teach that the potential for scarless tissue regeneration is present throughout the lifespan of some primitive species such as hydra and planaria, but is restricted to early development in most mammalian species. The inventors of the present invention further teach that sequential molecular alterations occur following the completion of embryogenesis in mammals that benefit the organism by providing tumor suppression, but the aforementioned alterations have the deleterious effect of suppressing the potential for later scarless regeneration and senolysis in numerous tissues (antagonistic pleiotropy). Therefore, it is taught that the repression of regeneration after embryogenesis interferes with tumor growth in the adult. Senolysis on the other hand (being defined as the programmed apoptosis of cells that have significant DNA damage such as that associated with dysfunctional telomeres) is taught to be repressed following embryogenesis for the simple reason that tissues no longer capable of regeneration, cannot replace cells that have undergone senolysis. Therefore tissues that can regenerate tend to be susceptible to apoptosis while those than cannot regenerate tend to inhibit apoptosis. Therefore, the inventors of the present invention teach that the intrinsic capacity of cells to regenerate or undergo senolysis are regulated by the molecular mechanisms associated with normal development in mammals, more specifically those alterations associated with the EFT.

Furthermore, the inventors of the present invention teach that altering the molecular regulation of the EFT in adult mammalian tissues so as to restore an embryonic pattern of gene expression (referred to herein as "induced Tissue Regeneration", or "iTR" are capable of inducing increased scarless tissue regeneration throughout the body, and are simultaneously capable of inducing senolysis.

Further, the inventors of the present invention teach that while the majority of malignant cancer cells revert to an embryonic pattern of gene expression (referred to herein as the "embryoonco phenotype" or "EOP" (characterized for example by repressing the expression of *COX7A1),* the EOP is labile with subsets of tumor cells shifting to an adult pattern of gene expression. As a result, when tumors are exposed to genotoxic stimuli, such as when patients are treated with chemotherapeutic agents or radiation therapy, the majority of cancer cells with an EOP being relatively sensitive to apoptosis are destroyed, while a residual number of cells that are relatively adult in the pattern of gene expression (for instance in expressing *COX7A1),* are resistant to apoptosis and can seed further growth and resistance of the cancer to the chemo or radiation therapy. Therefore, surprisingly, the inventors teach the counterintuitive interpretation that what are often thought of as "cancer stem cells" or "CSCs" are in actuality not immature progenitors, but surprisingly the opposite is the case, CSCs are more mature adult-like cells (expressing, for example, more *COX7A1).* This understanding provides novel diagnostics and therapeutic strategies in monitoring for the presence of markers of embryonic or adult markers in tumors or in the blood as cancer diagnostics and the companion therapeutic strategy of treating cancers with iTR factors to revert cancer cells back to an embryonic pattern of gene expression such that they are again capable of senolysis in the presence of chemotherapy or radiation therapy.

According to a first aspect, the present invention provides a set of compositions for altering the genetic profile of post-natal mammalian cells comprising:
a. a first composition comprising: 0.5 - 5.0mM valproic acid, 7 - 10µM 6- [[2-[[4-(2,4-Dichlorophenyl)-5-(5- methyl- lH-imidazol-2-yl)-2- pyrimidinyl] amino] ethyl] amino] -3 -pyridinecarbonitrile (CHIR99021), 4.0 - 10µM 2 -(3- (6-Methylpyridine-2-yl)-lH-pyrazol-4-yl)-1,5-naphthyridine (RepSox), 2.0 - 10µM Tranylpromine (Parnate), 0.5 - 50µM Forskolin and 1.0 - 5µM 4-[(E)-2-(5, 5,8,8- tetramethyl-6,7-dihydronaphthalen-2-yl)prop-l-enyl]benzoic acid (TTNPB);
b. a second composition comprising: 0.5 - 5.0mM valproic acid, 7 - l0uM CHIR99021, 4.0 - 10µM RepSox, 2.0 - 10µM Tranylpromine (Parnate), 0.5 - 50µM Forskolin, 1.0 - 5µM TTNPB and 50nM - 240nM 3-Deazaneplanocin A (DZNep); and,
c. a third composition comprising: 0.1 - 1.0 µM N-[(2R)-2,3- dihydroxypropoxy] -3 ,4-difluoro-2-(2-fluoro-4-iodoanilino)benzamide (PD0325901) and 7.0 -10 µM 6-[[2-[[4-(2,4-Dichlorophenyl)-5-(5- methyl- lH-imidazol-2-yl)-2- pyrimidinyl] amino] ethyl] amino] -3 -pyridinecarbonitrile (CHIR99021 ).

The present invention further provides A set of compositions for altering the genetic profile of post-natal mammalian cells comprising:
a. a first composition comprising: 0.5 - 5.0mM valproic acid, 7 - 10µM 6- [ [2-[ [4-(2,4-Dichlorophenyl)-5 -(5- methyl- 1 H-imidazol-2-yl)-2- pyrimidinyl] amino] ethyl] amino] -3 -pyridinecarbonitrile (CHIR99021), 4.0 - 10µM 2 -(3- (6-Methylpyridine-2-yl)-lH-pyrazol-4-yl)-1,5-naphthyridine (RepSox), 2.0 - 10µM Tranylpromine (Parnate), 0.5 - 50µM Forskolin and 1.0 - 5µM 4-[(E)-2-(5, 5,8,8- tetramethyl-6,7-dihydronaphthalen-2-yl)prop-l-enyl]benzoic acid (TTNPB);
b. a second composition comprising: 0.5 - 5.0mM valproic acid, 7 - 10µM CHIR99021, 4.0 - 10µM RepSox, 2.0 - 10µM Tranylpromine (Parnate), 0.5 - 50µM Forskolin, 1.0 - 5µM TTNPB and 50nM - 240nM 3-Deazaneplanocin A (DZNep);
c. a third composition comprising: 0.1 - 1.0 µM N-[(2R)-2,3- dihydroxypropoxy] -3 ,4-difluoro-2-(2-fluoro-4-iodoanilino)benzamide (PD0325901) and 7.0 -10 µM 6-[[2-[[4-(2,4-Dichlorophenyl)-5-(5- methyl- lH-imidazol-2-yl)-2- pyrimidinyl] amino] ethyl] amino] -3 -pyridinecarbonitrile (CHIR99021 );
d. a fourth composition comprising an expression vector for expressing the gene TERT in mammalian cells.

In another aspect of the present invention, methods are provided to identify agents capable of iTR comprised of the steps of 1) culturing post-natal mammalian cells in a format that facilitates the exposure of said cells to one or more exogenous agents suspected of inducing tissue regeneration, 2) contacting said cells with said one or more exogenous agents and, 3) measuring the expression of genetic markers of embryonic-fetal transition (EFT) in said cells, wherein alteration of said expression is indicative of the ability of the agent to cause iTR.

The invention further provides a method to identify agents capable of inducing tissue regeneration (iTR), the method comprising: 1) culturing post-natal mammalian cells in a format that facilitates the exposure of said cells to one or more exogenous agents suspected of inducing tissue regeneration, 2) contacting said cells with said one or more exogenous agents and, 3) preparing RNA from said cells and measuring the levels of embryonic-fetal transition (EFT) transcripts in said cells to determine whether said one or more agents cause iTR.

In a further aspect, the invention provides a method of altering the genetic profile of post-natal cells, the method comprising:
a. contacting one or more post-natal mammalian cells with a first composition comprising 0.5 - 5.0 mM valproic acid, 7 - 10µM CHIR99021, 4 - 10µM RepSox, 2.0 - 10µM Tranylpromine (Parnate), 0.5 - 50µM Forskolin, 1.0 - 5µM TTNPB for approximately 17 days;
b. contacting the one or more cells of step (a) with a second composition comprising 0.5 - 5.0 mM valproic acid, 7.0 - 10µM CHIR99021, 4 - 10µM RepSox, 2.0 - 10µM Tranylpromine (Parnate), 0.5 - 50µM Forskolin, 1.0 - 5µM TTNPB and 50 - 240nM 3- Deazaneplanocin A (DZNep) for 14 days; and
c. contacting the one or more cells of step (b) with a third composition comprising 0.1 - 1.0 µM PD0325901 and 7.0 - 10 µM CHIR99021 for approximately 7 days.

Certain conventional techniques of cell biology, cell culture, molecular biology, microbiology, recombinant nucleic acid (e.g., DNA) technology, immunology, etc., which are within the skill of the art, may be of use in aspects of the invention. Non-limiting descriptions of certain of these techniques are found in the following publications: Ausubel, F., et al., (eds.), Current Protocols in Molecular Biology, Current Protocols in Immunology, Current Protocols in Protein Science, and Current Protocols in Cell Biology, all John Wiley & Sons, N.Y., editions as of 2008; Sambrook, Russell, and Sambrook, Molecular Cloning: A Laboratory Manual, 3.sup.rd ed., Cold Spring Harbor Laboratory Press, Cold Spring Harbor, 2001; Harlow, E. and Lane, D., Antibodies--A Laboratory Manual, Cold Spring Harbor Laboratory Press, Cold Spring Harbor, 1988; Burns, R., Immunochemical Protocols (Methods in Molecular Biology) Humana Press; 3rd ed., 2005, Monoclonal antibodies: a practical approach (P. Shepherd and C Dean, eds., Oxford University Press, 2000); Freshney, R. I., "Culture of Animal Cells, A Manual of Basic Technique", 5th ed., John Wiley & Sons, Hoboken, N J, 2005).

### BRIEF DESCRIPTION OF THE DRAWINGS

FIG. 1 shows *COX7A1* expression in hES cells, diverse hEP lines, human arm fibroblasts during the EFT through postnatal development, adult arm fibroblasts before and after reprogramming to iPS cells, and adult arm fibroblasts before and after inducing iTR with AGEX1547. Error bars show St. Dev.
FIG. 2 shows *ADIRF* expression in hES cells, diverse hEP lines, human arm fibroblasts during the EFT through postnatal development, adult arm fibroblasts before and after reprogramming to iPS cells, and adult arm fibroblasts before and after inducing iTR with AGEX1547. Error bars show St. Dev.
FIG. 3 shows *TNFRSF11B* expression in hES cells, diverse hEP lines, human arm fibroblasts during the EFT through postnatal development, adult arm fibroblasts before and after reprogramming to iPS cells, and adult arm fibroblasts before and after inducing iTR with AGEX1547. Error bars show St. Dev.
FIG. 4 shows *AMH* expression in hES cells, diverse hEP lines, human arm fibroblasts during the EFT through postnatal development, adult arm fibroblasts before and after reprogramming to iPS cells, and adult arm fibroblasts before and after inducing iTR with AGEX1547. Error bars show St. Dev.
FIG. 5 shows *COL1A1* expression in hES cells, diverse hEP lines, human arm fibroblasts during the EFT through postnatal development, adult arm fibroblasts before and after reprogramming to iPS cells, and adult arm fibroblasts before and after inducing iTR with AGEX1547. Error bars show St. Dev.
FIG. 6 shows *POU5F1 (OCT4)* expression in hES cells, diverse hEP lines, human arm fibroblasts during the EFT through postnatal development, adult arm fibroblasts before and after reprogramming to iPS cells, and adult arm fibroblasts before and after inducing iTR with AGEX1547. Error bars show St. Dev.
FIG. 7 shows TUNEL assay results comparing the hES cell-derived clonal embryonic progenitor cell lines 4D20.8 and 30MV2-6 to adult counterparts MSC and HAEC cells exposed to o nM, 0.037 nM and 3.7 nM Thapsigargin.
FIG. 8 shows a list of candidate small molecule iTR factors, TR inhibitor genes, and TR activator genes useful in screening.
FIG. 9 shows COX7A1 expression for the first set of samples in Example 4.
FIG. 10 shows COX 7A1 expression for the second set of samples in Example 5.

### DETAILED DESCRIPTION

The invention is set out in the appended set of claims. The embodiments and/or examples of the following description which are not covered by the appended claims are not considered to be part of the present invention.

### Abbreviations

AC - Adult-derived cells
AMH - Anti-Mullerian Hormone
ASC - Adult stem cells
cGMP - Current Good Manufacturing Processes
CM - Cancer Maturation
CNS - Central Nervous System
DMEM - Dulbecco's modified Eagle's medium
DMSO - Dimethyl sulphoxide
DNAm - Changes in the methylation of DNA that provide a marker or "clock" of the age of cells and tissue.
DNN - Deep Neural Network
DPBS - Dulbecco's Phosphate Buffered Saline
ED Cells - Embryo-derived cells; hED cells are human ED cells
EDTA - Ethylenediamine tetraacetic acid
EFT - Embryonic-Fetal Transition
EG Cells - Embryonic germ cells; hEG cells are human EG cells
EP - Embryonic progenitors
ES Cells - Embryonic stem cells; hES cells are human ES cells
ESC - Embryonic Stem Cells
FACS - Fluorescence activated cell sorting
FBS - Fetal bovine serum
FPKM - Fragments Per Kilobase of transcript per Million mapped reads from RNA sequencing.
GFER - Growth Factor, Augmenter of Liver Regeneration (ALR)
GFP - Green fluorescent protein
GMP - Good Manufacturing Practices
HAEC - Human Aortic Endothelial Cell
hED Cells - Human embryo-derived cells
hEG Cells - "Human embryonic germ cells" are stem cells derived from the primordial germ cells of fetal tissue.
HESC - Human Embryonic Stem Cells
hiPS Cells - "Human induced pluripotent stem cells" are cells with properties similar to hES cells obtained from somatic cells after exposure to hES-specific transcription factors such as *SOX2, KLF4, OCT4, MYC,* or *NANOG, LIN28, OCT4,* and *SOX2.*
HSE - "Human skin equivalents" are mixtures of cells and biological or synthetic matrices manufactured for testing purposes or for therapeutic application in promoting wound repair.
iCM - Induced Cancer Maturation.
iPS Cells - "Induced pluripotent stem cells" are cells with properties similar to hES cells obtained from somatic cells after exposure to ES-specific transcription factors such as *SOX2, KLF4, OCT4, MYC,* or *NANOG, LIN28, OCT4,* and *SOX2, SOX2, KLF4, OCT4, MYC,* and (*LIN28A* or *LIN28B*), or other combinations of *OCT4, SOX2, KLF4, NANOG, ESRRB, NR5A2, CEBPA, MYC, LIN28A* and *LIN28B.*
iS-CSC - "Induced Senolysis of Cancer Stem Cells" refers to the treatment of cells in malignant tumors that are refractory to ablation by chemotherapeutic agents or radiation therapy wherein said iS-CSC treatment causes said refractory cells to revert to a pre-fetal pattern of gene expression and become sensitive to chemotherapeutic agents or radiation therapy.
iTM - Induced Tissue Maturation
iTR - Induced Tissue Regeneration
MEM - Minimal essential medium
MSC - Mesenchymal stem cell
NT - Nuclear Transfer
PBS - Phosphate buffered saline
PPT - "Prenatal-Postnatal Transition" refers to the molecular alterations that occur in cells of placental mammals at or within a week of birth.
PS fibroblasts - "Pre-scarring fibroblasts" are fibroblasts derived from the skin of early gestational skin or derived from ED cells that display a prenatal pattern of gene expression in that they promote the rapid healing of dermal wounds without scar formation.
RFU - Relative Fluorescence Units
RNA-seq - RNA sequencing
SFM - Serum-Free Medium
St. Dev. - Standard Deviation
TR - Tissue Regeneration

### Definitions

The term "analytical reprogramming technology" refers to a variety of methods to reprogram the pattern of gene expression of a somatic cell to that of a more pluripotent state, such as that of an iPS, ES, ED, EC or EG cell, wherein the reprogramming occurs in multiple and discrete steps and does not rely simply on the transfer of a somatic cell into an oocyte and the activation of that oocyte (see U.S. application nos. 60/332,510, filed November 26, 2001; 10/304,020, filed November 26, 2002; PCT application no. PCT/US02/37899, filed November 26, 2003; U.S. application no. 60/705625, filed August 3, 2005; U.S. application no. 60/729173, filed August 20, 2005; U.S. application no. 60/818813, filed July 5, 2006, PCT/US06/30632, filed August 3, 2006).

The term "induced Senolysis of Cancer Stem Cells" (iS-CSC) refers to the treatment of cells in malignant tumors that are refractory to ablation by chemotherapeutic agents or radiation therapy wherein said iS-CSC treatment causes said refractory cells to revert to a pre-fetal pattern of gene expression and become sensitive to chemotherapeutic agents or radiation therapy.

The term "cell expressing gene X", "gene X is expressed in a cell" (or cell population), or equivalents thereof, means that analysis of the cell using a specific assay platform provided a positive result. The converse is also true (i.e., by a cell not expressing gene X, or equivalents, is meant that analysis of the cell using a specific assay platform provided a negative result). Thus, any gene expression result described herein is tied to the specific probe or probes employed in the assay platform (or platforms) for the gene indicated.

The term "cell line" refers to a mortal or immortal population of cells that is capable of propagation and expansion *in vitro.*

The term "clonal" refers to a population of cells obtained the expansion of a single cell into a population of cells all derived from that original single cells and not containing other cells.

The term "differentiated cells" when used in reference to cells made by methods of this invention from pluripotent stem cells refer to cells having reduced potential to differentiate when compared to the parent pluripotent stem cells. The differentiated cells of this invention comprise cells that could differentiate further (i.e., they may not be terminally differentiated).

The term "embryonic" or "embryonic stages of development" refers to prenatal stages of development of cells, tissues or animals, specifically, the embryonic phases of development of cells compared to fetal and adult cells. In the case of the human species, the transition from embryonic to fetal development occurs at about 8 weeks of prenatal development, in mouse it occurs on or about 16 days, and in the rat species, at approximately 17.5 days post coitum. (http://php.med.unsw.edu.au/embryology/index.php?title=Mouse_Timeline_Detailed).

The term "embryonic stem cells" (ES cells) refers to cells derived from the inner cell mass of blastocysts, blastomeres, or morulae that have been serially passaged as cell lines while maintaining an undifferentiated state (e.g. expressing *TERT, OCT4,* and SSEA and TRA antigens specific for ES cells of the species). The ES cells may be derived from fertilization of an egg cell with sperm or DNA, nuclear transfer, parthenogenesis, or by means to generate hES cells with hemizygosity or homozygosity in the MHC region. While ES cells have historically been defined as cells capable of differentiating into all of the somatic cell types as well as germ line when transplanted into a preimplantation embryo, candidate ES cultures from many species, including human, have a more flattened appearance in culture and typically do not contribute to germ line differentiation, and are therefore called "ES-like cells." It is commonly believed that human ES cells are in reality "ES-like", however, in this application we will use the term ES cells to refer to both ES and ES-like cell lines.

The term "global modulator of TR" or "global modulator of iTR" refers to agents capable of modulating a multiplicity of iTR genes or iTM genes including, but not limited to, agents capable of downregulating *COX7A1, ADIRF, or TNFRSF11B* while simultaneously up-regulating *AMH* in cells derived from fetal or adult sources and are capable of inducing a pattern of gene expression leading to increased scarless tissue regeneration in response to tissue damage or degenerative disease.

The term "human embryonic stem cells" (hES cells) refers to human ES cells.

The term "human induced pluripotent stem cells" refers to cells with properties similar to hES cells, including the ability to form all three germ layers when transplanted into immunocompromised mice wherein said iPS cells are derived from cells of varied somatic cell lineages following exposure to de-differentiation factors, for example hES cell-specific transcription factor combinations: *KLF4, SOX2, MYC; OCT4* or *SOX2, OCT4, NANOG,* and *LIN28;* or various combinations of *OCT4, SOX2, KLF4, NANOG, ESRRB, NR5A2, CEBPA, MYC, LIN28A* and *LIN28B* or other methods that induce somatic cells to attain a pluripotent stem cell state with properties similar to hES cells. However, the reprogramming of somatic cells by somatic cell nuclear transfer (SCNT) are typically referred to as NT-ES cells as opposed to iPS cells.

The term "induced Cancer Maturation" refers to methods resulting in a change in the phenotype of premalignant or malignant cells such that subsequent to said induction, the cells express markers normally expressed in that cell type in fetal or adult stages of development as opposed to the embryonic stages.

The term "induced tissue regeneration" refers to the use of the methods of the present invention to alter the molecular composition of fetal or adult mammalian cells such that said cells are capable or regenerating functional tissue following damage to that tissue wherein said regeneration would not be the normal outcome in animals of that species. While functionally iTR is intended to generate new tissue formation at the sights of injury or degenerative disease or to induce senolysis in CSCs or aged cells, the inventors of the present invention teach that in iTR is in fact reversing many aspects of aging in cells including markers such as DNAm but not restoring telomerase activity. The addition of telomerase activity together with iTR is also defined in the present invention as "iTR".

The term "iTR-related Senolysis" refers to the induction of apoptosis in cells of aged tissues that have significant DNA damage including but not limited to that from cell aging (telomere shortening) through the reprogramming of said damaged cells to an embryonic pattern of gene expression.

The term "isolated" refers to a substance that is (i) separated from at least some other substances with which it is normally found in nature, usually by a process involving the hand of man, (ii) artificially produced (e.g., chemically synthesized), and/or (iii) present in an artificial environment or context (i.e., an environment or context in which it is not normally found in nature).

The term "iS-CSC factors" refers to molecules that alter the levels of TR activators and TR inhibitors in a manner leading to TR and associated increase in sensitivity to apoptosis of cancer cells exposed to chemotherapeutic or radiation therapy.

The term "iTR factor" refers to molecules that alter the levels of TR activators and TR inhibitors in a manner leading to TR in a tissue not naturally capable of TR. Said iTR factor also refers to combinations of individual factors. Therefore, cocktails of factors decribed herein including but not limited to the cocktail designated AgeX1547 is considered an "iTR factor" in the present application.

The term "iTR genes" refers to genes that when altered in expression can cause induced tissue regeneration in tissues not normally capable of such regeneration.
The term "nucleic acid" is used interchangeably with "polynucleotide" and encompasses in various embodiments naturally occurring polymers of nucleosides, such as DNA and RNA, and non-naturally occurring polymers of nucleosides or nucleoside analogs. In some embodiments a nucleic acid comprises standard nucleosides (abbreviated A, G, C, T, U). In other embodiments, a nucleic acid comprises one or more non-standard nucleosides. In some embodiments, one or more nucleosides are non-naturally occurring nucleosides or nucleotide analogs. A nucleic acid can comprise modified bases (for example, methylated bases), modified sugars (2'-fluororibose, arabinose, or hexose), modified phosphate groups or other linkages between nucleosides or nucleoside analogs (for example, phosphorothioates or 5'-N-phosphoramidite linkages), locked nucleic acids, or morpholinos. In some embodiments, a nucleic acid comprises nucleosides that are linked by phosphodiester bonds, as in DNA and RNA. In some embodiments, at least some nucleosides are linked by non-phosphodiester bond(s). A nucleic acid can be single-stranded, double-stranded, or partially double-stranded. An at least partially double-stranded nucleic acid can have one or more overhangs, e.g., 5' and/or 3' overhang(s). Nucleic acid modifications (e.g., nucleoside and/or backbone modifications, including use of non-standard nucleosides) known in the art as being useful in the context of RNA interference (RNAi), aptamer, or antisense-based molecules for research or therapeutic purposes are contemplated for use in various embodiments of the instant invention. See, e.g., Crooke, S T (ed.) Antisense drug technology: principles, strategies, and applications, Boca Raton: CRC Press, 2008; Kurreck, J. (ed.) Therapeutic oligonucleotides, RSC biomolecular sciences. Cambridge: Royal Society of Chemistry, 2008. In some embodiments, a modification increases half-life and/or stability of a nucleic acid, e.g., *in vivo,* relative to RNA or DNA of the same length and strandedness. In some embodiments, a modification decreases immunogenicity of a nucleic acid relative to RNA or DNA of the same length and strandedness. In some embodiments, between 5% and 95% of the nucleosides in one or both strands of a nucleic acid is modified. Modifications may be located uniformly or nonuniformly, and the location of the modifications (e.g., near the middle, near or at the ends, alternating, etc.) can be selected to enhance desired property(ies). A nucleic acid may comprise a detectable label, e.g., a fluorescent dye, radioactive atom, etc. "Oligonucleotide" refers to a relatively short nucleic acid, e.g., typically between about 4 and about 60 nucleotides long. Where reference is made herein to a polynucleotide, it is understood that both DNA, RNA, and in each case both single- and double-stranded forms (and complements of each single-stranded molecule) are provided. "Polynucleotide sequence" as used herein can refer to the polynucleotide material itself and/or to the sequence information (i.e. the succession of letters used as abbreviations for bases) that biochemically characterizes a specific nucleic acid. A polynucleotide sequence presented herein is presented in a 5' to 3' direction unless otherwise indicated.

The term "oligoclonal" refers to a population of cells that originated from a small population of cells, typically 2-1000 cells, that appear to share similar characteristics such as morphology or the presence or absence of markers of differentiation that differ from those of other cells in the same culture. Oligoclonal cells are isolated from cells that do not share these common characteristics, and are allowed to proliferate, generating a population of cells that are essentially entirely derived from the original population of similar cells.

The term "pluripotent stem cells" refers to animal cells capable of differentiating into more than one differentiated cell type. Such cells include hES cells, blastomere/morula cells and their derived hED cells, hiPS cells, hEG cells, hEC cells, and adult-derived cells including mesenchymal stem cells, neuronal stem cells, and bone marrow-derived stem cells. Pluripotent stem cells may be genetically modified or not genetically modified. Genetically modified cells may include markers such as fluorescent proteins to facilitate their identification within the egg.

The term "polypeptide" refers to a polymer of amino acids. The terms "protein" and "polypeptide" are used interchangeably herein. A peptide is a relatively short polypeptide, typically between about 2 and 60 amino acids in length. Polypeptides used herein typically contain the standard amino acids (i.e., the 20 L-amino acids that are most commonly found in proteins). However, a polypeptide can contain one or more non-standard amino acids (which may be naturally occurring or non-naturally occurring) and/or amino acid analogs known in the art in certain embodiments. One or more of the amino acids in a polypeptide may be modified, for example, by the addition of a chemical entity such as a carbohydrate group, a phosphate group, a fatty acid group, a linker for conjugation, functionalization, etc. A polypeptide that has a nonpolypeptide moiety covalently or noncovalently associated therewith is still considered a "polypeptide". Polypeptides may be purified from natural sources, produced using recombinant DNA technology, synthesized through chemical means such as conventional solid phase peptide synthesis, etc. The term "polypeptide sequence" or "amino acid sequence" as used herein can refer to the polypeptide material itself and/or to the sequence information (i.e., the succession of letters or three letter codes used as abbreviations for amino acid names) that biochemically characterizes a polypeptide. A polypeptide sequence presented herein is presented in an N-terminal to C-terminal direction unless otherwise indicated. A polypeptide may be cyclic or contain a cyclic portion. Where a naturally occurring polypeptide is discussed herein, it will be understood that the invention encompasses embodiments that relate to any isoform thereof (e.g., different proteins arising from the same gene as a result of alternative splicing or editing of mRNA or as a result of different alleles of a gene, e.g., alleles differing by one or more single nucleotide polymorphisms (typically such alleles will be at least 95%, 96%, 97%, 98%, 99%, or more identical to a reference or concensus sequence). A polypeptide may comprise a sequence that targets it for secretion or to a particular intracellular compartment (e.g., the nucleus) and/or a sequence targets the polypeptide for post-translational modification or degradation. Certain polypeptides may be synthesized as a precursor that undergoes post-translational cleavage or other processing to become a mature polypeptide. In some instances, such cleavage may only occur upon particular activating events. Where relevant, the invention provides embodiments relating to precursor polypeptides and embodiments relating to mature versions of a polypeptide.

The term "prenatal" refers to a stage of embryonic development of a placental mammal prior to which an animal is not capable of viability apart from the uterus.

The term "primordial stem cells" refers collectively to pluripotent stem cells capable of differentiating into cells of all three primary germ layers: endoderm, mesoderm, and ectoderm, as well as neural crest. Therefore, examples of primordial stem cells would include but not be limited by human or non-human mammalian ES cells or cell lines, blastomere/morula cells and their derived ED cells, iPS, and EG cells.

The term "purified" refers to agents or entities (e.g., compounds) that have been separated from most of the components with which they are associated in nature or when originally generated. In general, such purification involves action of the hand of man. Purified agents or entities may be partially purified, substantially purified, or pure. Such agents or entities may be, for example, at least 50%, 60%, 70%, 75%, 80%, 85%, 90%, 95%, 96%, 97%, 98%, 99%, or more than 99% pure. In some embodiments, a nucleic acid or polypeptide is purified such that it constitutes at least 75%, 80%, 855%, 90%, 95%, 96%, 97%, 98%, 99%, or more, of the total nucleic acid or polypeptide material, respectively, present in a preparation. Purity can be based on, e.g., dry weight, size of peaks on a chromatography tracing, molecular abundance, intensity of bands on a gel, or intensity of any signal that correlates with molecular abundance, or any art-accepted quantification method. In some embodiments, water, buffers, ions, and/or small molecules (e.g., precursors such as nucleotides or amino acids), can optionally be present in a purified preparation. A purified molecule may be prepared by separating it from other substances (e.g., other cellular materials), or by producing it in such a manner to achieve a desired degree of purity. In some embodiments, a purified molecule or composition refers to a molecule or composition that is prepared using any art-accepted method of purification. In some embodiments "partially purified" means that a molecule produced by a cell is no longer present within the cell, e.g., the cell has been lysed and, optionally, at least some of the cellular material (e.g., cell wall, cell membrane(s), cell organelle(s)) has been removed.

The term "RNA interference" (RNAi) is used herein consistently with its meaning in the art to refer to a phenomenon whereby double-stranded RNA (dsRNA) triggers the sequence-specific degradation or translational repression of a corresponding mRNA having complementarity to a strand of the dsRNA. It will be appreciated that the complementarity between the strand of the dsRNA and the mRNA need not be 100% but need only be sufficient to mediate inhibition of gene expression (also referred to as "silencing" or "knockdown"). For example, the degree of complementarity is such that the strand can either (i) guide cleavage of the mRNA in the RNA-induced silencing complex (RISC); or (ii) cause translational repression of the mRNA. In certain embodiments the double-stranded portion of the RNA is less than about 30 nucleotides in length, e.g., between 17 and 29 nucleotides in length. In certain embodiments a first strand of the dsRNA is at least 80%, 85%, 90%, 95%, or 100% complementary to a target mRNA and the other strand of the dsRNA is at least 80%, 85%, 90%, 95%, or 100% complementary to the first strand. In mammalian cells, RNAi may be achieved by introducing an appropriate double-stranded nucleic acid into the cells or expressing a nucleic acid in cells that is then processed intracellularly to yield dsRNA therein. Nucleic acids capable of mediating RNAi are referred to herein as "RNAi agents". Exemplary nucleic acids capable of mediating RNAi are a short hairpin RNA (shRNA), a short interfering RNA (siRNA), and a microRNA precursor.

These terms are well known and are used herein consistently with their meaning in the art. siRNAs typically comprise two separate nucleic acid strands that are hybridized to each other to form a duplex. They can be synthesized *in vitro,* e.g., using standard nucleic acid synthesis techniques. siRNAs are typically double-stranded oligonucleotides having 16-30, e.g., 16, 17, 18, 19, 20, 21, 22, 23, 24, 25, 26, 27, 28, 29, or 30 nucleotides (nt) in each strand, wherein the double-stranded oligonucleotide comprises a double-stranded portion between 15 and 29 nucleotides long and either or both of the strands may comprise a 3' overhang between, e.g., 1-5 nucleotides long, or either or both ends can be blunt. In some embodiments, an siRNA comprises strands between 19 and 25 nt, e.g., between 21 and 23 nucleotides long, wherein one or both strands comprises a 3' overhang of 1-2 nucleotides. One strand of the double-stranded portion of the siRNA (termed the "guide strand" or "antisense strand") is substantially complementary (e.g., at least 80% or more, e.g., 85%, 90%, 95%, or 100%) complementary to (e.g., having 3, 2, 1, or 0 mismatched nucleotide(s)) a target region in the mRNA, and the other double-stranded portion is substantially complementary to the first double-stranded portion. In many embodiments, the guide strand is 100% complementary to a target region in an mRNA and the other passenger strand is 100% complementary to the first double-stranded portion (it is understood that, in various embodiments, the 3' overhang portion of the guide strand, if present, may or may not be complementary to the mRNA when the guide strand is hybridized to the mRNA). In some embodiments, a shRNA molecule is a nucleic acid molecule comprising a stem-loop, wherein the double-stranded stem is 16-30 nucleotides long and the loop is about 1-10 nucleotides long. siRNA can comprise a wide variety of modified nucleosides, nucleoside analogs and can comprise chemically or biologically modified bases, modified backbones, etc. Without limitation, any modification recognized in the art as being useful for RNAi can be used. Some modifications result in increased stability, cell uptake, potency, etc. Some modifications result in decreased immunogenicity or clearance. In certain embodiments the siRNA comprises a duplex about 19-23 (e.g., 19, 20, 21, 22, or 23) nucleotides in length and, optionally, one or two 3' overhangs of 1-5 nucleotides in length, which may be composed of deoxyribonucleotides. shRNA comprise a single nucleic acid strand that contains two complementary portions separated by a predominantly non-selfcomplementary region. The complementary portions hybridize to form a duplex structure and the non-selfcomplementary region forms a loop connecting the 3' end of one strand of the duplex and the 5' end of the other strand. shRNAs undergo intracellular processing to generate siRNAs. Typically, the loop is between 1 and 8, e.g., 2-6 nucleotides long.

MicroRNAs (miRNAs) are small, naturally occurring, non-coding, single-stranded RNAs of about 21-25 nucleotides (in mammalian systems) that inhibit gene expression in a sequence-specific manner. They are generated intracellularly from precursors (pre-miRNA) having a characteristic secondary structure comprised of a short hairpin (about 70 nucleotides in length) containing a duplex that often includes one or more regions of imperfect complementarity which is in turn generated from a larger precursor (pri-miRNA). Naturally occurring miRNAs are typically only partially complementary to their target mRNA and often act via translational repression. RNAi agents modelled on endogenous miRNA or miRNA precursors are of use in certain embodiments of the invention. For example, an siRNA can be designed so that one strand hybridizes to a target mRNA with one or more mismatches or bulges mimicking the duplex formed by a miRNA and its target mRNA. Such siRNA may be referred to as miRNA mimics or miRNA-like molecules. miRNA mimics may be encoded by precursor nucleic acids whose structure mimics that of naturally occurring miRNA precursors.

In certain embodiments an RNAi agent is a vector (e.g., a plasmid or virus) that comprises a template for transcription of an siRNA (e.g., as two separate strands that can hybridize to each other), shRNA, or microRNA precursor. Typically the template encoding the siRNA, shRNA, or miRNA precursor is operably linked to expression control sequences (e.g., a promoter), as known in the art. Such vectors can be used to introduce the template into vertebrate cells, e.g., mammalian cells, and result in transient or stable expression of the siRNA, shRNA, or miRNA precursor. Precurors (shRNA or miRNA precursors) are processed intracellularly to generate siRNA or miRNA.

In general, small RNAi agents such as siRNA can be chemically synthesized or can be transcribed *in vitro* or *in vivo* from a DNA template either as two separate strands that then hybridize, or as an shRNA which is then processed to generate an siRNA. Often RNAi agents, especially those comprising modifications, are chemically synthesized. Chemical synthesis methods for oligonucleotides are well known in the art.

The term "small molecule" as used herein, is an organic molecule that is less than about 2 kilodaltons (KDa) in mass. In some embodiments, the small molecule is less than about 1.5 KDa, or less than about 1 KDa. In some embodiments, the small molecule is less than about 800 daltons (Da), 600 Da, 500 Da, 400 Da, 300 Da, 200 Da, or 100 Da. Often, a small molecule has a mass of at least 50 Da. In some embodiments, a small molecule contains multiple carbon-carbon bonds and can comprise one or more heteroatoms and/or one or more functional groups important for structural interaction with proteins (e.g., hydrogen bonding), e.g., an amine, carbonyl, hydroxyl, or carboxyl group, and in some embodiments at least two functional groups. Small molecules often comprise one or more cyclic carbon or heterocyclic structures and/or aromatic or polyaromatic structures, optionally substituted with one or more of the above functional groups. In some embodiments, a small molecule is non-polymeric. In some embodiments, a small molecule is not an amino acid. In some embodiments, a small molecule is not a nucleotide. In some embodiments, a small molecule is not a saccharide. The term "subject" can be any multicellular animal. Often a subject is a vertebrate, e.g., a mammal or avian. Exemplary mammals include, e.g., humans, non-human primates, rodents (e.g., mouse, rat, rabbit), ungulates (e.g., ovine, bovine, equine, caprine species), canines, and felines. Often, a subject is an individual to whom a compound is to be delivered, e.g., for experimental, diagnostic, and/or therapeutic purposes or from whom a sample is obtained or on whom a diagnostic procedure is performed (e.g., a sample or procedure that will be used to assess tissue damage and/or to assess the effect of a compound of the invention). The term "tissue damage" is used herein to refer to any type of damage or injury to cells, tissues, organs, or other body structures. The term encompasses, in various embodiments, degeneration due to disease, damage due to physical trauma or surgery, damage caused by exposure to deleterious substance, and other disruptions in the structure and/or functionality of cells, tissues, organs, or other body structures.

The term "tissue regeneration" or "TR" refers to at least partial regeneration, replacement, restoration, or regrowth of a tissue, organ, or other body structure, or portion thereof, following loss, damage, or degeneration, where said tissue regeneration but for the methods described in the present invention would not take place. Examples of tissue regeneration include the regrowth of severed digits or limbs including the regrowth of cartilage, bone, muscle, tendons, and ligaments, the scarless regrowth of bone, cartilage, skin, or muscle that has been lost due to injury or disease, with an increase in size and cell number of an injured or diseased organ such that the tissue or organ approximates the normal size of the tissue or organ or its size prior to injury or disease. Depending on the tissue type, tissue regeneration can occur via a variety of different mechanisms such as, for example, the rearrangement of pre-existing cells and/or tissue (e.g., through cell migration), the division of adult somatic stem cells or other progenitor cells and differentiation of at least some of their descendants, and/or the dedifferentiation, transdifferentiation, and/or proliferation of cells.

The term "TR activator genes" refers to genes whose lack of expression in fetal and adult cells but whose expression in embryonic phases of development facilitate TR.

The term "TR inhibitor genes" refers to genes whose expression in fetal and adult animals inhibit TR.

The term "treat", "treating", "therapy", "therapeutic" and similar terms in regard to a subject refer to providing medical and/or surgical management of the subject. Treatment can include, but is not limited to, administering a compound or composition (e.g., a pharmaceutical composition) to a subject. Treatment of a subject according to the instant invention is typically undertaken in an effort to promote regeneration, e.g., in a subject who has suffered tissue damage or is expected to suffer tissue damage (e.g., a subject who will undergo surgery). The effect of treatment can generally include increased regeneration, reduced scarring, and/or improved structural or functional outcome following tissue damage (as compared with the outcome in the absence of treatment), and/or can include reversal or reduction in severity or progression of a degenerative disease.

The term "variant" as applied to a particular polypeptide refers to a polypeptide that differs from such polypeptide (sometimes referred to as the "original polypeptide") by one or more amino acid alterations, e.g., addition(s), deletion(s), and/or substitution(s). Sometimes an original polypeptide is a naturally occurring polypeptide (e.g., from human or non-human animal) or a polypeptide identical thereto. Variants may be naturally occurring or created using, e.g., recombinant DNA techniques or chemical synthesis. An addition can be an insertion within the polypeptide or an addition at the N- or C-terminus. In some embodiments, the number of amino acids substituted, deleted, or added can be for example, about 1 to 30, e.g., about 1 to 20, e.g., about 1 to 10, e.g., about 1 to 5, e.g., 1, 2, 3, 4, or 5. In some embodiments, a variant comprises a polypeptide whose sequence is homologous to the sequence of the original polypeptide over at least 50 amino acids, at least 100 amino acids, at least 150 amino acids, or more, up to the full length of the original polypeptide (but is not identical in sequence to the original polypeptide), e.g., the sequence of the variant polypeptide is at least 50%, 60%, 70%, 75%, 80%, 85%, 90%, 95%, 96%, 97%, 98%, 99%, or more identical to the sequence of the original polypeptide over at least 50 amino acids, at least 100 amino acids, at least 150 amino acids, or more, up to the full length of the original polypeptide. In some embodiments, a variant comprises a polypeptide at least 50%, 60%, 70%, 75%, 80%, 85%, 90%, 91%, 92%, 93%, 94%, 95%, 96%, 97%, 98%, 99%, 99.5% or more identical to an original polypeptide over at least 50%, 60%, 70%, 80%, 85%, 90%, 91%, 92%, 93%, 94%, 95%, 96%, 97%, 98%, 99%, or 100% of the length of the original polypeptide. In some embodiments, a variant comprises at least one functional or structural domain, e.g., a domain identified as such in the Conserved Domain Database (CDD) of the National Center for Biotechnology Information (www.ncbi.nih.gov), e.g., an NCBI-curated domain.

In some embodiments one, more than one, or all biological functions or activities of a variant or fragment is substantially similar to that of the corresponding biological function or activity of the original molecule. In some embodiments, a functional variant retains at least 10%, 20%, 30%, 40%, 50%, 60%, 70%, 80%, 90%, 95%, 96%, 97%, 98%, 99%, or more of the activity of the original polypeptide, e.g., about equal activity. In some embodiments, the activity of a variant is up to approximately 100%, approximately 125%, or approximately 150% of the activity of the original molecule. In other nonlimiting embodiments, an activity of a variant or fragment is considered substantially similar to the activity of the original molecule if the amount or concentration of the variant needed to produce a particular effect is within 0.5 to 5-fold of the amount or concentration of the original molecule needed to produce that effect.

In some embodiments amino acid "substitutions" in a variant are the result of replacing one amino acid with another amino acid having similar structural and/or chemical properties, i.e., conservative amino acid replacements. "Conservative" amino acid substitutions may be made on the basis of similarity in any of a variety or properties such as side chain size, polarity, charge, solubility, hydrophobicity, hydrophilicity, and/or amphipathicity of the residues involved. For example, the non-polar (hydrophobic) amino acids include alanine, leucine, isoleucine, valine, glycine, proline, phenylalanine, tryptophan and methionine. The polar (hydrophilic), neutral amino acids include serine, threonine, cysteine, tyrosine, asparagine, and glutamine. The positively charged (basic) amino acids include arginine, lysine and histidine. The negatively charged (acidic) amino acids include aspartic acid and glutamic acid. Within a particular group, certain substitutions may be of particular interest, e.g., replacements of leucine by isoleucine (or vice versa), serine by threonine (or vice versa), or alanine by glycine (or vice versa). Of course, non-conservative substitutions are often compatible with retaining function as well. In some embodiments, a substitution or deletion does not alter or delete an amino acid important for activity. Insertions or deletions may range in size from about 1 to 20 amino acids, e.g., 1 to 10 amino acids. In some instances, larger domains may be removed without substantially affecting function. In certain embodiments of the invention the sequence of a variant can be obtained by making no more than a total of 5, 10, 15, or 20 amino acid additions, deletions, or substitutions to the sequence of a naturally occurring enzyme. In some embodiments no more than 1%, 5%, 10%, or 20% of the amino acids in a polypeptide are insertions, deletions, or substitutions relative to the original polypeptide. Guidance in determining which amino acid residues may be replaced, added, or deleted without eliminating or substantially reducing activities of interest, may be obtained by comparing the sequence of the particular polypeptide with that of homologous polypeptides (e.g., from other organisms) and minimizing the number of amino acid sequence changes made in regions of high homology (conserved regions) or by replacing amino acids with those found in homologous sequences since amino acid residues that are conserved among various species are more likely to be important for activity than amino acids that are not conserved.

In some embodiments, a variant of a polypeptide comprises a heterologous polypeptide portion. The heterologous portion often has a sequence that is not present in or homologous to the original polypeptide. A heterologous portion may be, e.g., between 5 and about 5,000 amino acids long, or longer. Often it is between 5 and about 1,000 amino acids long. In some embodiments, a heterologous portion comprises a sequence that is found in a different polypeptide, e.g., a functional domain. In some embodiments, a heterologous portion comprises a sequence useful for purifying, expressing, solubilizing, and/or detecting the polypeptide. In some embodiments, a heterologous portion comprises a polypeptide "tag", e.g., an affinity tag or epitope tag. For example, the tag can be an affinity tag (e.g., HA, TAP, Myc, 6xHis, Flag, GST), fluorescent or luminescent protein (e.g., EGFP, ECFP, EYFP, Cerulean, DsRed, mCherry), solubility-enhancing tag (e.g., a SUMO tag, NUS A tag, SNUT tag, or a monomeric mutant of the Ocr protein of bacteriophage T7). See, e.g., Esposito D and Chatterjee D K. Curr Opin Biotechnol.; 17(4):353-8 (2006). In some embodiments, a tag can serve multiple functions. A tag is often relatively small, e.g., ranging from a few amino acids up to about 100 amino acids long. In some embodiments a tag is more than 100 amino acids long, e.g., up to about 500 amino acids long, or more. In some embodiments, a polypeptide has a tag located at the N- or C-terminus, e.g., as an N- or C-terminal fusion. The polypeptide could comprise multiple tags. In some embodiments, a 6.times.His tag and a NUS tag are present, e.g., at the N-terminus. In some embodiments, a tag is cleavable, so that it can be removed from the polypeptide, e.g., by a protease. In some embodiments, this is achieved by including a sequence encoding a protease cleavage site between the sequence encoding the portion homologous to the original polypeptide and the tag. Exemplary proteases include, e.g., thrombin, TEV protease, Factor Xa, PreScission protease, etc. In some embodiments, a "self-cleaving" tag is used. See, e.g., PCT/US05/05763. Sequences encoding a tag can be located 5' or 3' with respect to a polynucleotide encoding the polypeptide (or both). In some embodiments, a tag or other heterologous sequence is separated from the rest of the polypeptide by a polypeptide linker. For example, a linker can be a short polypeptide (e.g., 15-25 amino acids). Often a linker is composed of small amino acid residues such as serine, glycine, and/or alanine. A heterologous domain could comprise a transmembrane domain, a secretion signal domain, etc.

In certain embodiments of the invention a fragment or variant, optionally excluding a heterologous portion, if present, possesses sufficient structural similarity to the original polypeptide so that when its 3-dimensional structure (either actual or predicted structure) is superimposed on the structure of the original polypeptide, the volume of overlap is at least 70%, preferably at least 80%, more preferably at least 90% of the total volume of the structure of the original polypeptide. A partial or complete 3-dimensional structure of the fragment or variant may be determined by crystallizing the protein, which can be done using standard methods. Alternately, an NMR solution structure can be generated, also using standard methods. A modeling program such as MODELER (Sali, A. and Blundell, T L, J. Mol. Biol., 234, 779-815, 1993), or any other modeling program, can be used to generate a predicted structure. If a structure or predicted structure of a related polypeptide is available, the model can be based on that structure. The PROSPECT-PSPP suite of programs can be used (Guo, J T, et al., Nucleic Acids Res. 32 (Web Server issue):W522-5, Jul. 1, 2004). Where embodiments of the invention relate to variants of a polypeptide, it will be understood that polynucleotides encoding the variant are provided.

The term "vector" is used herein to refer to a nucleic acid or a virus or portion thereof (e.g., a viral capsid or genome) capable of mediating entry of, e.g., transferring, transporting, etc., a nucleic acid molecule into a cell. Where the vector is a nucleic acid, the nucleic acid molecule to be transferred is generally linked to, e.g., inserted into, the vector nucleic acid molecule. A nucleic acid vector may include sequences that direct autonomous replication (e.g., an origin of replication), or may include sequences sufficient to allow integration of part or all of the nucleic acid into host cell DNA. Useful nucleic acid vectors include, for example, DNA or RNA plasmids, cosmids, and naturally occurring or modified viral genomes or portions thereof or nucleic acids (DNA or RNA) that can be packaged into viral) capsids. Plasmid vectors typically include an origin of replication and one or more selectable markers. Plasmids may include part or all of a viral genome (e.g., a viral promoter, enhancer, processing or packaging signals, etc.). Viruses or portions thereof that can be used to introduce nucleic acid molecules into cells are referred to as viral vectors. Useful viral vectors include adenoviruses, adeno-associated viruses such as AAV8, retroviruses, lentiviruses, vaccinia virus and other poxviruses, herpesviruses (e.g., herpes simplex virus), and others. Viral vectors may or may not contain sufficient viral genetic information for production of infectious virus when introduced into host cells, i.e., viral vectors may be replication-defective, and such replication-defective viral vectors may be preferable for therapeutic use. Where sufficient information is lacking it may, but need not be, supplied by a host cell or by another vector introduced into the cell. The nucleic acid to be transferred may be incorporated into a naturally occurring or modified viral genome or a portion thereof or may be present within the virus or viral capsid as a separate nucleic acid molecule. It will be appreciated that certain plasmid vectors that include part or all of a viral genome, typically including viral genetic information sufficient to direct transcription of a nucleic acid that can be packaged into a viral capsid and/or sufficient to give rise to a nucleic acid that can be integrated into the host cell genome and/or to give rise to infectious virus, are also sometimes referred to in the art as viral vectors. Vectors may contain one or more nucleic acids encoding a marker suitable for use in the identifying and/or selecting cells that have or have not been transformed or transfected with the vector. Markers include, for example, proteins that increase or decrease either resistance or sensitivity to antibiotics (e.g., an antibiotic-resistance gene encoding a protein that confers resistance to an antibiotic such as puromycin, hygromycin or blasticidin) or other compounds, enzymes whose activities are detectable by assays known in the art (e.g., beta.-galactosidase or alkaline phosphatase), and proteins or RNAs that detectably affect the phenotype of transformed or transfected cells (e.g., fluorescent proteins). Expression vectors are vectors that include regulatory sequence(s), e.g., expression control sequences such as a promoter, sufficient to direct transcription of an operably linked nucleic acid. Regulatory sequences may also include enhancer sequences or upstream activator sequences. Vectors may optionally include 5' leader or signal sequences. Vectors may optionally include cleavage and/or polyadenylations signals and/or a 3' untranslated regions. Vectors often include one or more appropriately positioned sites for restriction enzymes, to facilitate introduction into the vector of the nucleic acid to be expressed. An expression vector comprises sufficient cis-acting elements for expression; other elements required or helpful for expression can be supplied by the host cell or *in vitro* expression system.

Various techniques may be employed for introducing nucleic acid molecules into cells. Such techniques include chemical-facilitated transfection using compounds such as calcium phosphate, cationic lipids, cationic polymers, liposome-mediated transfection, non-chemical methods such as electroporation, particle bombardment, or microinjection, and infection with a virus that contains the nucleic acid molecule of interest (sometimes termed "transduction"). Markers can be used for the identification and/or selection of cells that have taken up the vector and, typically, express the nucleic acid. Cells can be cultured in appropriate media to select such cells and, optionally, establish a stable cell line.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number. Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

### Methods

In addition to the methods described below, methods that find use in the production and use of cells with an embryonic pattern of gene expression corresponding with scarless regenerative potential can be found in the following: PCT application Ser. No. PCT/US2006/013519 filed on April 11, 2006 and titled "Novel Uses of Cells With Prenatal Patterns of Gene Expression"; U.S. patent application Ser. No. 11/604,047 filed on November 21, 2006 and titled "Methods to Accelerate the Isolation of Novel Cell Strains from Pluripotent Stem Cells and Cells Obtained Thereby"; and U.S. patent application Ser. No. 12/504,630 filed on July 16, 2009 and titled "Methods to Accelerate the Isolation of Novel Cell Strains from Pluripotent Stem Cells and Cells Obtained Thereby", (See, e.g. U.S. provisional patent application no. 61/831,421, filed June 5, 2013, PCT patent application PCT/US2014/040601, filed June 3, 2014 and U.S. patent application no. 14/896,664, filed on December 7, 2015), "Compositions and Methods for Induced Tissue Regeneration in Mammalian Species" (international patent application publication number WO 2014/197421) and "Improved Methods for Detecting and Modulating the Embryonic-Fetal Transition in Mammalian Species" (international patent application publication number WO 2017/214342.

### RNAi

By way of nonlimiting example, dsRNA is prepared from *in vitro* transcription reactions (Promega) using PCR-generated templates with flanking T7 promoters, purified by phenol extraction and ethanol precipitation, and annealed after resuspension in water. Intact experimental animals are injected with 4 x 30 nL dsRNA on three consecutive days following induced tissue injury beginning with the first injection two hours after surgery.

### TR Modulation and iTR Modulators

The present disclosure provides novel iTR modulators and methods of use thereof. In some aspects, the invention provides novel methods of enhancing regeneration comprising administering an agent that alters the concentration of said iTR modulators to a multicellular organism in need thereof.

The applicants teach that primitive animals that display the potential for profound TR such as the regeneration of amputated limbs in axolotls, the regeneration of skin in MRL or the African Spiny Mouse, or the regeneration of whole body segments in planaria, do so by simply recapitulating normal embryonic development of the respective tissues. Furthermore, the applicants teach that the cause of inability to regenerate damaged tissue in TR-resistant mammals such as most murine species and humans is that certain embryonic gene transcription is altered in the EFT in these TR-resistant animals. The applicants further teach that the restoration of certain of these embryo-specific patterns of gene expression altered in the EFT in TR-resistant animals can induce competency for regeneration in any tissue, including responsiveness to organizing center factors, leading to complex tissue regeneration and a comcommitant reduction in scar formation. Lastly, the applicants teach novel agents and associated methods of inducing TR in mammalian species. Said methods facilitate TR in mammalian species *in vivo,* particularly in the species *Homo sapiens.*

Genes whose expression in fetal and adult animals inhibit TR are herein designated "TR inhibitors", and genes whose lack of expression in fetal and adult cells but whose expression in embryonic phases of development facilitate TR are herein designated "TR activators." Collectively, TR inhibitor genes and TR activator genes are herein designated iTR genes. Molecules that alter the levels of TR activators and TR inhibitors in a manner leading to TR are herein designated "iTR factors". iTR genes and, the protein products of iTR genes, are often conserved in animals ranging from sea anemones to mammals. The gene-encoded protein sequences, and sequences of nucleic acids (e.g., mRNA) encoding genes referred to herein, including those from a number of different non-human animal species are known in the art and can be found, e.g., in publicly available databases such as those available at the National Center for Biotechnology Information (NCBI) (www.ncbi.nih.gov).

The TR inhibitory gene *COX7A1* was observed to be expressed primarily in stromal as opposed to epithelial cells in normal tissue, though it was also expressed at lower levels in epithelial cultures. In the case of neoplasms, the gene was observed to be down-regulated in many stromal cancers such as osteosarcoma, chondrosarcoma, rhabdomyosarcoma, as well as some gliomas, carcinomas, and adenocarcinomas. This is consistent with the observation of increased glycolysis in cancer known as the Warburg effect, though the absence of *COX7A1* expression has not previously been implicated in the Warburg effect. Since the applicants propose that TR genes are altered in the transition from embryonic to fetal development in part to prevent cancer in the adult, the repression of *COX7A1* in stromal and some CNS and epithelial tumors would revert a stromal cell to an embryonic state which thereby facilitating oncogenesis. The exogenous induction of expression of *COX7A1* in such tumors lacking expression would therefore have a therapeutic effect, in part by altering the activity of p53 and HIF1alpha, and HIF1alpha, and thereby inhibiting cell proliferation and increasing apoptosis in cancer cells.

In another embodiment, the present invention provides a means of detecting cancer cells. Rarely have researchers identified a marker of an abnormality associated with a majority of cancer cell types. As described herein, the markers distinguishing embryonic from their fetal and adult counterparts can be used to distinguish normal cells displaying an adult pattern of expression from malignant cells, which display an embryonic pattern. Said detection methods, including but not limited to the expression of *COX7A1, NAALADL1, AMH,* and genes from the alpha, beta, and gamma clustered protocadherin genes including but not limited to *PCDHA4, PCDHB2,* and *PCDHGA12* in an embryonic as opposed to fetal/adult pattern are useful not only in identifying malignant cells (with the exception of blood cells), but are also useful in identifying tumors that will be resistant to commonly-used chemotherapeutic agents which are characterized by their expression of a fetal/adult pattern.

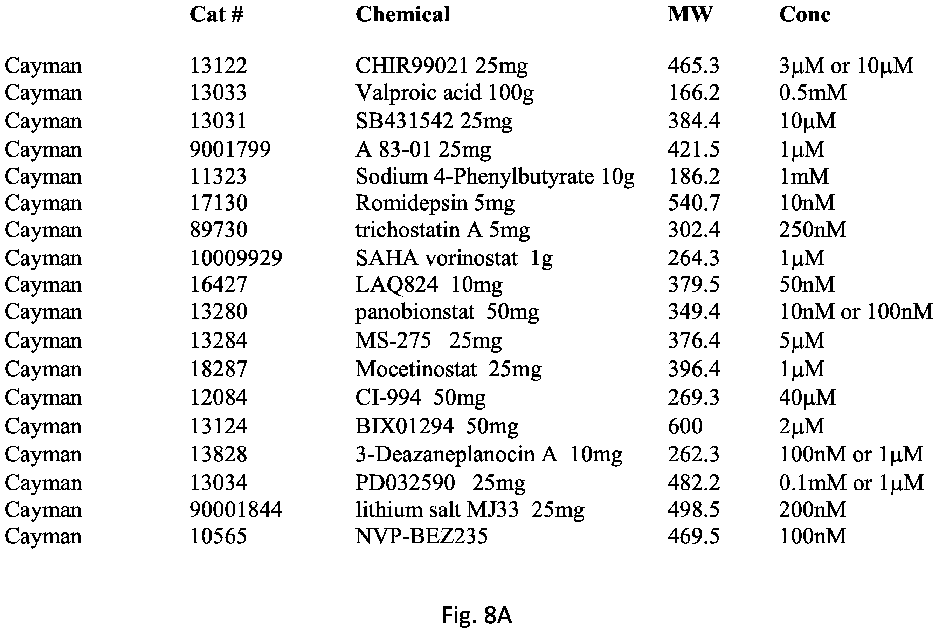

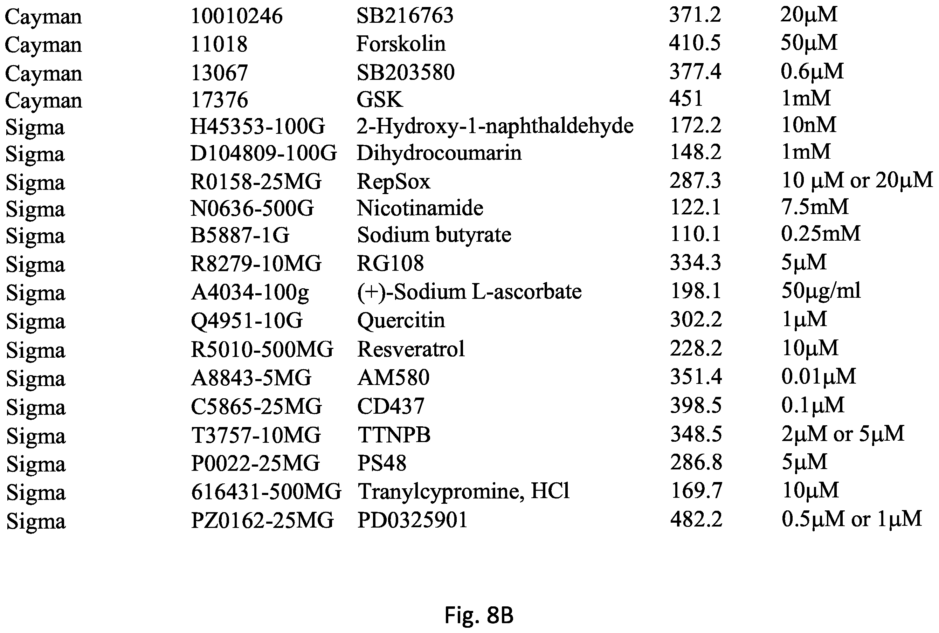

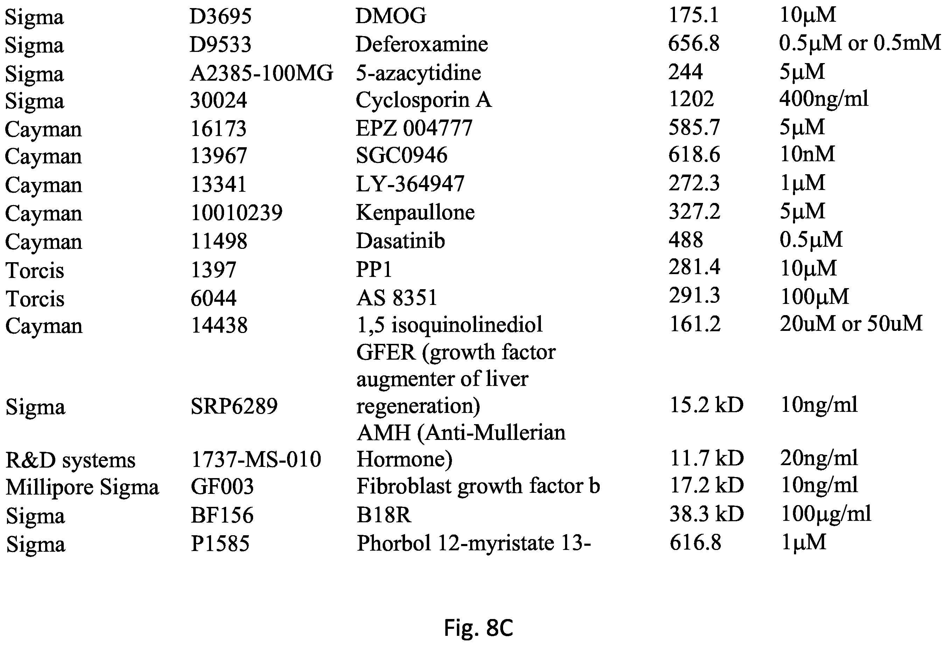

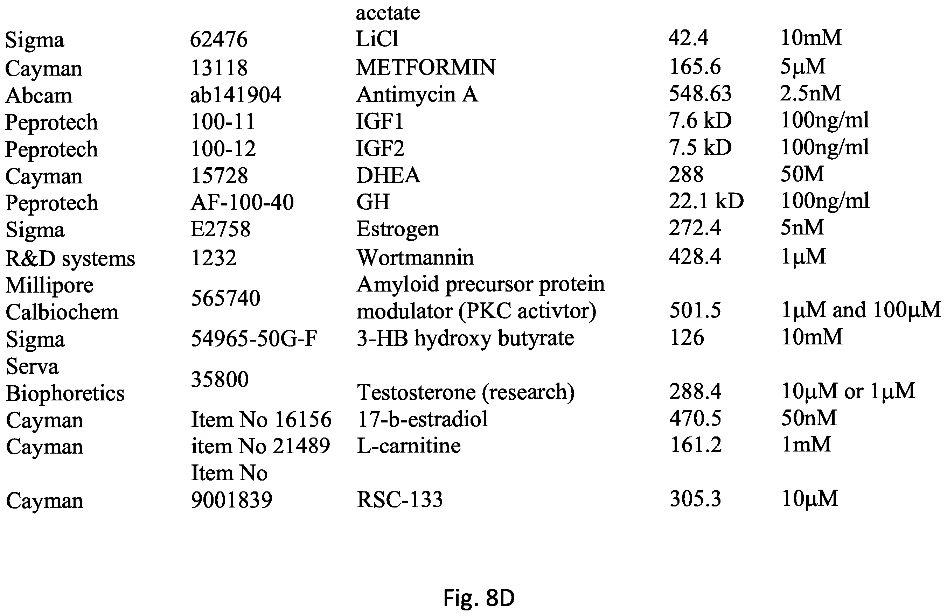

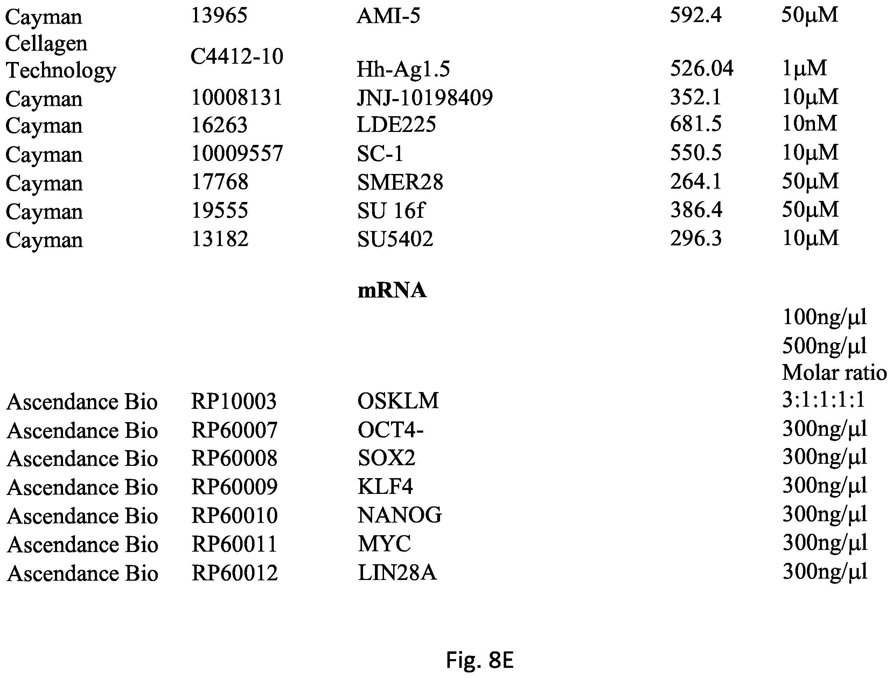

The disclosure provides a number of different methods of modulating iTR genes and a variety of different compounds useful for modulating iTR genes. In general, an iTR factor can be, e.g., a small molecule, nucleic acid, oligonucleotide, polypeptide, peptide, lipid, carbohydrate, etc. Said iTR factor can be one or a combination of TR factors listed in Figure 8. The concentration and optimum combination of factors can be determined by screening the combinations of factors against their effect on mammalian cells, preferably human cells that correspond to fetal or adult stages of development such as those that express *COX7A1,* and determining iTR formulations that lead to markedly decreased *COX7A1, ADIRF, TNFRSF11B,* or increased *AMH* expressions.

In some embodiments of the invention, iTR factors inhibit by decreasing the amount of TR inhibitor RNA produced by cells and/or by decreasing the level of activity of TR inhibitor genes. In the case of targeting TR inhibitors, factors are identified and used in research and therapy that reduce the levels of the product of the TR inhibitor gene. Said TR inhibitor gene can be any one or combination of TR inhibitor genes previously disclosed, for example, in WO 2017/214342 or WO 2014/197421. Said TR inhibitor gene can be any one or combination of TR inhibitor genes. The amount of TR inhibitor gene RNA can be decreased by inhibiting synthesis of TR inhibitor RNA synthesis by cells (also referred to as "inhibiting TR inhibitor gene expression"), e.g., by reducing the amount of mRNA encoding TR inhibitor genes or by reducing translation of mRNA encoding TR inhibitor genes. Said factor can be by way of nonlimiting example, RNAi targeting a sequence within the TR inhibitor genes.

In some embodiments, TR inhibitor gene expression is inhibited by RNA interference (RNAi). As known in the art, RNAi is a process in which the presence in a cell of double-stranded RNA that has sequence correspondence to a gene leads to sequence-specific inhibition of the expression of the gene, typically as a result of cleavage or translational repression of the mRNA transcribed from the gene. Compounds useful for causing inhibition of expression by RNAi ("RNAi agents") include short interfering RNAs (siRNAs), short hairpin RNAs (shRNAs), microRNAs (miRNAs), and miRNA-like molecules.

One of skill in the art can readily design sequences for RNAi agents, e.g., siRNAs, useful for inhibiting expression of mammalian TR inhibitor genes, e.g., human TR inhibitor genes once one has identified said TR inhibitor genes. In some embodiments, such sequences are selected to minimize "off-target" effects. For example, a sequence that is complementary to a sequence present in TR inhibitor gene mRNA and not present in other mRNAs expressed in a species of interest (or not present in the genome of the species of interest) may be used. Position-specific chemical modifications may be used to reduce potential off-target effects. In some embodiments, at least two different RNAi agents, e.g., siRNAs, targeted to TR inhibitor gene mRNA are used in combination. In some embodiments, a microRNA (which may be an artificially designed microRNA) is used to inhibit TR inhibitor gene expression.

In some embodiments of the invention, TR inhibitor gene expression is inhibited using an antisense molecule comprising a single-stranded oligonucleotide that is perfectly or substantially complementary to mRNA encoding TR inhibitor genes. The oligonucleotide hybridizes to TR inhibitor gene mRNA leading, e.g., to degradation of the mRNA by RNase H or blocking of translation by steric hindrance. In other embodiments of the invention, TR inhibitor gene expression is inhibited using a ribozyme or triplex nucleic acid.

In some embodiments, of the invention, a TR inhibitor inhibitor inhibits at least one activity of an TR inhibitor protein. TR inhibitor activity can be decreased by contacting the TR inhibitor protein with a compound that physically interacts with the TR inhibitor protein. Such a compound may, for example, alter the structure of the TR inhibitor protein (e.g., by covalently modifying it) and/or block the interaction of the TR inhibitor protein with one or more other molecule(s) such as cofactors or substrates. In some embodiments, inhibition or reduction may be a decrease of at least about 5%, 10%, 15%, 20%, 25%, 30%, 35%, 40%, 45%, 50%, 55%, 60%, 65%, 70%, 75%, 80%, 85%, 90%, 95%, or 99% of a reference level (e.g., a control level). A control level may be the level of the TR inhibitor that occurs in the absence of the factor. For example, an TR factor may reduce the level of the TR inhibitor protein to no more than 95%, 90%, 85%, 80%, 75%, 70%, 65%, 60%, 55%, 50%, 40%, 30%, 25%, 20%, 10%, or 5% of the level that occurs in the absence of the factor under the conditions tested. In some embodiments, levels of the TR inhibitor are reduced to 75% or less of the level that occurs in the absence of the factor, under the conditions tested. In some embodiments, levels of the TR inhibitor are reduced to 50% or less of the level that occurs in the absence of the TR factor, under the conditions tested. In some embodiments, levels of the TR inhibitor are reduced to 25% or less of the level that occurs in the absence of the iTR factor, under the conditions tested. In some embodiments, levels of the TR inhibitor are reduced to 10% or less of the level that occurs in the absence of the iTR factor, under the conditions tested. In some cases the level of modulation (e.g., inhibition or reduction) as compared with a control level is statistically significant. As used herein, "statistically significant" refers to a p-value of less than 0.05, e.g., a p-value of less than 0.025 or a p-value of less than 0.01, using an appropriate statistical test (e.g, ANOVA, t-test, etc.).

In some embodiments of the invention, a compound directly inhibits TR inhibitor proteins, i.e., the compound inhibits TR inhibitor proteins by a mechanism that involves a physical interaction (binding) between the TR inhibitor and the iTR factor. For example, binding of a TR inhibitor to an iTR factor can interfere with the TR inhibitor's ability to catalyze a reaction and/or can occlude the TR inhibitors active site. A variety of compounds can be used to directly inhibit TR inhibitors. Exemplary compounds that directly inhibit TR inhibitors can be, e.g., small molecules, antibodies, or aptamers.

In some embodiments of the invention, an iTR factor binds covalently to the TR inhibitor. For example, the compound may modify amino acid residue(s) that are needed for enzymatic activity. In some embodiments, an iTR factor comprises one or more reactive functional groups such as an aldehyde, haloalkane, alkene, fluorophosphonate (e.g., alkyl fluorophosphonate), Michael acceptor, phenyl sulfonate, methylketone, e.g., a halogenated methylketone or diazomethylketone, fluorophosphonate, vinyl ester, vinyl sulfone, or vinyl sulfonamide, that reacts with an amino acid side chain of TR inhibitors. In some embodiments, an iTR factor inhibitor comprises a compound that physically interacts with a TR inhibitor, wherein the compound comprises a reactive functional group. In some embodiments, the structure of a compound that physically interacts with the TR inhibitor is modified to incorporate a reactive functional group. In some embodiments, the compound comprises a TR inhibitor substrate analog or transition state analog. In some embodiments, the compound interacts with the TR inhibitor in or near the TR inhibitor active site.

In other embodiments, an iTR factor binds non-covalently to a TR inhibitor and/or to a complex containing the TR inhibitor and a TR inhibitor substrate. In some embodiments, an iTR factor binds non-covalently to the active site of a TR inhibitor and/or competes with substrate(s) for access to the TR inhibitor active site. In some embodiments, an iTR factor binds to the TR inhibitor with a K_{d} of approximately 10⁻³ M or less, e.g., 10⁻⁴ M or less, e.g., 10⁻⁵ M or less, e.g., 10⁻⁶ M or less, 10⁻⁷ M or less, 10⁻⁸ M or less, or 10 M or less under the conditions tested, e.g., in a physiologically acceptable solution such as phosphate buffered saline. Binding affinity can be measured, e.g., using surface plasmon resonance (e.g., with a Biacore system), isothermal titration calorimetry, or a competitive binding assay, as known in the art. In some embodiments, the inhibitor comprises a TR inhibitor substrate analog or transition state analog.

In the case of increasing the activity of TR activators, any one of combination of the TR activator genes listed in Figure 8 may be used. The levels of the products of these genes may be introduced using the vectors described herein.

In other embodiments, the iTR factors are constructs that introduce RNA into cells either directly or through gene expression constructs that are capable of inducing pluripotency if allowed to react with cells for a sufficient period of time, but for lesser times can cause iTR. Preferably, the RNAs do not include all of the RNAs needed for reprogramming to pluripotency and instead include only *LIN28A* or *LIN28B* optionally together with an agent to increase telomere length such as RNA for the catalytic component of telomerase (*TERT*)*.* Most preferably, the agents to induce iTR are genes/factors induced by *LIN28A* or *LIN28B*-encoded proteins such as GFER, optionally in combination with an agent that increases telomere length such as the RNA or gene encoding TERT, and/or in combination with the factors disclosed herein important for iTR such as 0.05-5mM valproic acid, preferably 0.5 mM valproic acid, 1-100 ng/mL AMH, preferably 10 ng/mL AMH, and 2-200 ng/mL GFER, preferably 20 ng/mL. When administered in vivo, such factors are preferably administered in a slow-release hydrogel matrix such as one comprised of chemically modified and crosslinked hyaluronic acid and collagen such as HyStem matrices.

### Reporter-Based Screening Assays for iTR Factors

The invention provides methods for identifying iTR factors using (a) a reporter molecule comprising a readily-detectable marker such as GFP or beta galactosidase whose expression is driven by the promoter of one of the TR activator genes described herein such as that for *COX7A1.* The invention provides screening assays that involve determining whether a test compound affects the expression of TR activator genes and/or inhibits the expression of TR inhibitory genes. The invention further provides reporter molecules and compositions useful for practicing the methods. In general, compounds identified using the inventive methods can act by any of mechanism that results in increased or decreased TR activator or inhibitor genes respectively. In the case of the *COX7A1* promoter, a promoter sequence flanking the 5' end of the human gene has been characterized to the position of -756 bases to the ATG translation start codon (Yu, M., et al. Biochimica and Biophysica Acta 1574 (2002) 345-353). Transcription start site of the most cDNAs were observed to be at -55 bases of the translation start codon. The promoter, as well as the rest of the gene sequence, lays in a CpG island, similarly to the promoters of many housekeeping genes, although the expression of *COX7A1* is tissue specific. CpG islands are characterized by the abundance of CG dinucleotides that surpasses that of the average, expected content for the genome, over the span of at least 200 bases. The promoter comprises several regulatory binding site sequences: MEF2 at position -524, as well as three E boxes (characterized as E1, E2, and E3), at, respectively - positions -58, -279 and -585; E box is a DNA binding site (CAACTG) that binds members of the myogenic family of regulatory proteins. Additionally, in the region approximately -95 to -68 bases, there are multiple CG rich segments similar to the one recognized by the transcription factor Sp1.

The gene itself, as characterized in GRCh38.p7 primary assembly, occupies 1948 bases between positions 36150922 and 36152869 on Human chromosome 18, and comprises 4 exons interspersed by three introns. Gene sequence, with the promoter sequence is curated at NCBI under locus identifier AF037372.

### Reporter Molecules, Cells, and Membranes

In general, detectable moieties useful in the reporter molecules of the invention include light-emitting or light-absorbing compounds that generate or quench a detectable fluorescent, chemiluminescent, or bioluminescent signal. In some embodiments, activation of TR activator genes or inhibition of TR inhibitory genes causes release of the detectable moiety into a liquid medium, and the signal generated or quenched by the released detectable moiety present in the medium (or a sample thereof) is detected. In some embodiments, the resulting signal causes an alteration in a property of the detectable moiety, and such alteration can be detected, e.g., as an optical signal. For example, the signal may alter the emission or absorption of electromagnetic radiation (e.g., radiation having a wavelength within the infrared, visible or UV portion of the spectrum) by the detectable moiety. In some embodiments, a reporter molecule comprises a fluorescent or luminescent moiety, and a second molecule serves as quencher that quenches the fluorescent or luminescent moiety. Such alteration can be detected using apparatus and methods known in the art.

In many embodiments of the invention, the reporter molecule is a genetically encodable molecule that can be expressed by a cell, and the detectable moiety comprises, e.g., a detectable polypeptide. Thus in some embodiments, the reporter molecule is a polypeptide comprising a fluorescent polypeptides such as green, blue, sapphire, yellow, red, orange, and cyan fluorescent proteins and derivatives thereof (e.g., enhanced GFP); monomeric red fluorescent protein and derivatives such as those known as "mFruits", e.g., mCherry, mStrawberry, mTomato, etc., and luminescent proteins such as aequorin. (It will be understood that in some embodiments, the fluorescence or luminescence occurs in the presence of one or more additional molecules, e.g., an ion such as a calcium ion and/or a prosthetic group such as coelenterazine.) In some embodiments, the detectable moiety comprises an enzyme that acts on a substrate to produce a fluorescent, luminescent, colored, or otherwise detectable product. Examples of enzymes that may serve as detectable moieties include luciferase; beta-galactosidase; horseradish peroxidase; alkaline phosphatase; etc. (It will be appreciated that the enzyme is detected by detecting the product of the reaction.) In some embodiments, the detectable moiety comprises a polypeptide tag that can be readily detected using a second agent such as a labeled (e.g., fluorescently labeled) antibody. For example, fluorescently labeled antibodies that bind to the HA, Myc, or a variety of other peptide tags are available. Thus the invention encompasses embodiments in which a detectable moiety can be detected directly (i.e., it generates a detectable signal without requiring interaction with a second agent) and embodiments in which a detectable moiety interacts (e.g., binds and/or reacts) with a second agent and such interaction renders the detectable moiety detectable, e.g., by resulting in generation of a detectable signal or because the second agent is directly detectable. In embodiments in which a detectable moiety interacts with a second agent to produce a detectable signal, the detectable moiety may react with the second agent is acted on by a second agent to produce a detectable signal. In many embodiments, the intensity of the signal provides an indication of the amount of detectable moiety present. e.g., in a sample being asssessed or in area being imaged. In some embodiments, the amount of detectable moiety is optionally quantified, e.g., on a relative or absolute basis, based on the signal intensity.

The invention provides nucleic acids comprising a sequence that encodes a reporter polypeptide of the invention. In some embodiments, a nucleic acid encodes a precursor polypeptide of a reporter polypeptide of the invention. In some embodiments, the sequence encoding the polypeptide is operably linked to expression control elements (e.g., a promoter or promoter/enhancer sequence) appropriate to direct transcription of mRNA encoding the polypeptide. The invention further provides expression vectors comprising the nucleic acids. Selection of appropriate expression control elements may be based, e.g., on the cell type and species in which the nucleic acid is to be expressed. One of ordinary skill in the art can readily select appropriate expression control elements and/or expression vectors. In some embodiments, expression control element(s) are regulatable, e.g., inducible or repressible. Exemplary promoters suitable for use in bacterial cells include, e.g., Lac, Trp, Tac, araBAD (e.g., in a pBAD vectors), phage promoters such as T7 or T3. Exemplary expression control sequences useful for directing expression in mammalian cells include, e.g., the early and late promoters of SV40, adenovirus or cytomegalovirus immediate early promoter, or viral promoter/enhancer sequences, retroviral LTRs, promoters or promoter/enhancers from mammalian genes, e.g., actin, EF-1 alpha, phosphoglycerate kinase, etc. Regulatable (e.g., inducible or repressible) expression systems such as the Tet-On and Tet-Off systems (regulatable by tetracycline and analogs such as doxycycline) and others that can be regulated by small molecules such as hormones receptor ligands (e.g., steroid receptor ligands, which may or may not be steroids), metal-regulated systems (e.g., metallothionein promoter), etc.

The invention further provides cells and cell lines that comprise such nucleic acids and/or vectors. In some embodiments, the cells are eukaryotic cells, e.g., fungal, plant, or animal cells. In some embodiments, the cell is a vertebrate cell, e.g., a mammalian cell, e.g., a human cell, non-human primate cell, or rodent cell. Often a cell is a member of a cell line, e.g., an established or immortalised cell line that has acquired the ability to proliferate indefinitely in culture (e.g., as a result of mutation or genetic manipulation such as the constitutive expression of the catalytic component of telomerase). Numerous cell lines are known in the art and can be used in the instant invention. Mammalian cell lines include, e.g., HEK-293 (e.g., HEK-293T), CHO, NIH-3T3, COS, and HeLa cell lines. In some embodiments, a cell line is a tumor cell line. In other embodiments, a cell is non-tumorigenic and/or is not derived from a tumor. In some embodiments, the cells are adherent cells. In some embodiments, non-adherent cells are used. In some embodiments, a cell is of a cell type or cell line is used that has been shown to naturally have a subset of TR activator genes expressed or TR inhibitor genes not expressed. If a cell lacks one or more TR activator or inhibitor genes, the cell can be genetically engineered to express such protein(s). In some embodiments, a cell line of the invention is descended from a single cell. For example, a population of cells can be transfected with a nucleic acid encoding the reporter polypeptide and a colony derived from a single cell can be selected and expanded in culture. In some embodiments, cells are transiently transfected with an expression vector that encodes the reporter molecule. Cells can be co-transfected with a control plasmid, optionally expressing a different detectable polypeptide, to control for transfection efficiency (e.g., across multiple runs of an assay).

### TR Activator and TR Inhibitor Polypeptides and Nucleic Acids

TR activator and TR inhibitor genes are listed in Figure 8. Under the headings "Embryonic Markers" and "Fetal/Adult Markers", respectively. TR activator and TR inhibitor polypeptides useful in the inventive methods may be obtained by a variety of methods. In some embodiments, the polypeptides are produced using recombinant DNA techniques. Standard methods for recombinant protein expression can be used. A nucleic acid encoding a TR activator or TR inhibitor gene can readily be obtained, e.g., from cells that express the genes (e.g., by PCR or other amplification methods or by cloning) or by chemical synthesis or *in vitro* transcription based on the cDNA sequence polypeptide sequence. One of ordinary skill in the art would know that due to the degeneracy of the genetic code, the genes can be encoded by many different nucleic acid sequences. Optionally, a sequence is codon-optimized for expression in a host cell of choice. The genes could be expressed in bacterial, fungal, animal, or plant cells or organisms. The genes could be isolated from cells that naturally express it or from cells into which a nucleic acid encoding the protein has been transiently or stably introduced, e.g., cells that contain an expression vector encoding the genes. In some embodiments, the gene is secreted by cells in culture and isolated from the culture medium.

In some embodiments of the invention, the sequence of a TR activator or TR inhibitor polypeptide is used in the inventive screening methods. A naturally occurring TR activator or TR inhibitor polypeptide can be from any species whose genome encodes a TR activator or TR inhibitor polypeptide, e.g., human, non-human primate, rodent, etc. A polypeptide whose sequence is identical to naturally occurring TR activator or TR inhibitor is sometimes referred to herein as "native TR activator/inhibitor". A TR activator or TR inhibitor polypeptide of use in the invention may or may not comprise a secretion signal sequence or a portion thereof. For example, mature TR activator or TR inhibitor comprising or consisting of amino acids 20-496 of human TR activator or TR inhibitor (or corresponding amino acids of TR activator or TR inhibitor of a different species) may be used.

In some embodiments, a polypeptide comprising or consisting of a variant or fragment of TR activator or TR inhibitor is used. TR activator or TR inhibitor variants include polypeptides that differ by one or more amino acid substitutions, additions, or deletions, relative to TR activator or TR inhibitor. In some embodiments, a TR activator or TR inhibitor variant comprises a polypeptide at least 70%, 75%, 80%, 85%, 90%, 95%, 96%, 97%, 98%, 99%, or more identical to at least amino acids 20-496 of TR activator or TR inhibitor (e.g., from human or mouse) over at least 50%, 60%, 70%, 80%, 85%, 90%, 95%, 96%, 97%, 98%, 99%, or 100% of at least amino acids 20-496 of human TR activator or TR inhibitor or amino acids 20-503 of mouse TR activator or TR inhibitor. In some embodiments, a TR activator or TR inhibitor variant comprises a polypeptide at least 80%, 85%, 90%, 95%, 96%, 97%, 98%, 99%, or more identical to at least amino acids 20-496 of human TR activator or TR inhibitor or amino acids 20-503 of mouse TR activator or TR inhibitor. In some embodiments, a TR activator or TR inhibitor polypeptide comprises a polypeptide at least 80%, 85%, 90%, 95%, 96%, 97%, 98%, 99%, or more identical to at least amino acids 20-496 of human TR activator or TR inhibitor or amino acids 20-503 of mouse TR activator or TR inhibitor. A nucleic acid that encodes a TR activator or TR inhibitor variant or fragment can readily be generated, e.g., by modifying the DNA that encodes native TR activator or TR inhibitor using, e.g., site-directed mutagenesis, or by other standard methods, and used to produce the TR activator or TR inhibitor variant or fragment. For example, a fusion protein can be produced by cloning sequences that encode TR activator or TR inhibitor into a vector that provides the sequence encoding the heterologous portion. In some embodiments, a tagged TR activator or TR inhibitor is used. For example, in some embodiments a TR activator or TR inhibitor polypeptide comprising a 6xHis tag, e.g., at its C terminus, is used.

### Test Compounds

A wide variety of test compounds can be used in the inventive methods for identifying iTR factors and global modulators of iTR. For example, a test compound can be a small molecule, polypeptide, peptide, nucleic acid, oligonucleotide, lipid, carbohydrate, antibody, or hybrid molecule including but not limited to those described herein, including mRNA for the genes *OCT4, SOX2, KLF4, NANOG, ESRRB, NR5A2, CEBPA, MYC, LIN28A* and *LIN28B* alone and in diverse combinations, and in diverse combinations with small molecule compounds such as combinations of the following compounds: inhibitors of glycogen synthase 3 (GSK3) including but not limited to CHIR99021; inhibitors of TGF-beta signaling including but not limited to SB431542, A-83-01, and E616452; HDAC inhibitors including but not limited to aliphatic acid compounds including but not limited to: valproic acid, phenylbutyrate, and n-butyrate; cyclic tetrapeptides including trapoxin B and the depsipeptides; hydroxamic acids such as trichostatin A, vorinostat (SAHA), belinostat (PXD101), LAQ824, panobinostat (LBH589), and the benzamides entinostat (MS-275), CI994, mocetinostat (MGCD0103); those specifically targeting Class I (*HDAC1, HDAC2, HDAC3,* and *HDAC8*), IIA (*HDAC4, HDAC5, HDAC7,* and *HDAC9),* IIB (*HDAC6* and *HDAC10*), III (*SIRT1, SIRT2, SIRT3, SIRT4, SIRT5, SIRT6,* or *SIRT7)* including the sirtuin inhibitors nicotinomide, diverse derivatives of NAD, dihydrocoumarin, naphthopyranone, and 2-hydroxynaphthaldehydes, or IV (*HDAC11*) deacetylases; inhibitors of H3K4/9 histone demethylase LSD1 including but not limited to parnate; inhibitors of Dot1L including but not limited to EPZ004777; inhibitors of G9a including but not limited to Bix01294; inhibitors of *EZH2* including but not limited to DZNep, inhibitors of DNA methyltransferase including but not limited to RG108; 5-aza-2'deoxycytidine (trade name Vidaza and Azadine); vitamin C which can inhibit DNA methylation, increase Tet1 which increases 5hmC which is a first step of demethylation; activators of 3' phosphoinositide-dependent kinase 1 including but not limited to PS48; promoters of glycolysis including but not limited to Quercetin and fructose 2, 6-bisphosphate (an activator of phosphofructokinase 1); agents that promote the activity of the HIF1 transcription complex including but not limited to Quercetin; RAR agonists including but not limited to AM580, CD437, and TTNPB; agents that mimic hypoxia including but not limited to Resveratrol; agents that increase telomerase activity including but not limited to the exogenous expression of the catalytic component of telomerase *(TERT),* agents that promote epigenetic modifications via downregulation of LSD1, a H3K4-specific histone demethylase including but not limited to lithium; or inhibitors of the MAPK/ERK pathway including but not limited to PD032590. Additional compounds include histone deacetylase inhibitor MS-275, histone methyltransferase inhibitor BIX01294, DNA methyltransferase inhibitor RG108, analog of retinoic acid AM580 or CD437 (a retinoic acid receptor agonist), and historne methyltransferase inhibitor EPZ004777 (inhibitor of DOT1L and disruptor of telomeric silencing 1-like) and inhibitor of DNA methyltransferase and HDAC RSC133. Such compounds may be administered in diverse combinations, concentrations, and for differing periods of time, to optimize the effect of iTR on cells cultured *in vitro* using markers of global iTR such as by assaying for decreased expression of *COX7A1, ADIRF, TNFRSF11B,* or other inhibitors of iTR as described herein, and/or assaying for increased expresson of *AMH* or other activators or iTR as described herein, or in injured or diseased tissues *in vivo,* or in modulating the lifespan of animals *in vivo.*

*In vitro* assays for iTR patterns of expression of the genes *COX7A1, ADIRF, TNFRSF11B,* or *AMH* as well as gene expression or protein markers of pluripotency including *OCT4,* and *HELLS* or Tra-1-60, Tra-1-81, and SSEA4 respectively are performed to optimize global patterns of iTR gene expression without reverting the target cells to pluripotency. Examples of individual agents and combinations of agents screened are: *OCT4, SOX2, KLF4, MYC and LIN28A; OCT4; KLF4; OCT4, KLF4; OCT4, KLF4, LIN28A; OCT4, KLF4, LIN28B; SOX2; MYC; NANOG; ESRRB; NT5A2; OCT4, SOX2, KLF4,* and *LIN28A; OCT4, SOX2, KLF4,* and *LIN28B; OCT4, KLF4, MYC* and *LIN28A;* and each of the preceeding combinations of agents together with 0.25 mM NaB, 5µM PS48 and 0.5 µM A-83-01 during the first four weeks, followed by treatment with 0.25mM sodium butyrate, 5 µM PS48, 0.5 µM A-83-01 and 0.5 µM PD0325901 each of which is assayed at 0, 1, 2, 4, 7, 10, and 14 days for markers of global modulation of iTR gene expression.

### Improved iTR Factor Screen

The screen for compounds or combinations of compounds useful in producing an iTR effect in fetal or adult mammalian cells may be implemented *in vitro* or *in vivo* (such as animal models). Preferably, said screen is performed in vitro in low- or high-throughput. The screen assays for increase or decrease of embryonic or fetal/adult markers respectively. Said screen may employ the use of antibodies, the assay of RNA, metabolic markers, or other markers described herein or described in "Compositions and Methods for Induced Tissue Regeneration in Mammalian Species" (international patent application publication number WO 2014/197421), and "Improved Methods for Detecting and Modulating the Embryonic-Fetal Transition in Mammalian Species" (international patent application publication number WO 2017/214342). Preferably, said screens assay the levels of mRNA markers, most preferably a decrease in the expression of *COX7A1* as an indicator of iTR. Most preferably said screen is performed in multi-well format and the expression is assays through the use of a reporter gene such as eGFP knowed-in to the *COX7A1* locus in the genome.

Compounds can be obtained from natural sources or produced synthetically. Compounds can be at least partially pure or may be present in extracts or other types of mixtures whose components are at least in part unknown or uncharacterized. Extracts or fractions thereof can be produced from, e.g., plants, animals, microorganisms, marine organisms, fermentation broths (e.g., soil, bacterial or fungal fermentation broths), etc. In some embodiments, a compound collection ("library") is tested. The library may comprise, e.g., between 100 and 500,000 compounds, or more. Compounds are often arrayed in multwell plates (e.g., 384 well plates, 1596 well plates, etc.). They can be dissolved in a solvent (e.g., DMSO) or provided in dry form, e.g., as a powder or solid. Collections of synthetic, semi-synthetic, and/or naturally occurring compounds can be tested. Compound libraries can comprise structurally related, structurally diverse, or structurally unrelated compounds. Compounds may be artificial (having a structure invented by man and not found in nature) or naturally occurring. In some embodiments, a library comprises at least some compounds that have been identified as "hits" or "leads" in other drug discovery programs and/or derivatives thereof. A compound library can comprise natural products and/or compounds generated using non-directed or directed synthetic organic chemistry. Often a compound library is a small molecule library. Other libraries of interest include peptide or peptoid libraries, cDNA libraries, antibody libraries, and oligonucleotide libraries. A library can be focused (e.g., composed primarily of compounds having the same core structure, derived from the same precursor, or having at least one biochemical activity in common).

Compounds chosen for screening may be chosen from a library of synthetic or natural product-derived small molecules with a variety of chemical structures known to provide potential bioactive motifs. to valproic acid at a concentration of 0.05-5.0 mM, preferably 1.0 mM; the GSK-3 inhibitor 6-[[2-[[4-(2,4-Dichlorophenyl)-5-(5- methyl-1H-imidazol-2-yl)-2-pyrimidinyl]amino]ethyl]amino]-3-pyridinecarbonitrile also known as CHIR99021 at a concentration of 7.0 nM -10 uM, preferably 10uM; the inhibitor of the TGFβR-1/ALK5 2 -(3-(6-Methylpyridine-2-yl)-1H-pyrazol-4-yl)-1,5-naphthyridine also known as RepSox at a concentration of 4.0 nM-10 uM, preferably 10 uM; the inhibitor of lysine-specific demethylase 1 Parnate (also named tranylcypromine) at a concentration of 2.0-10 uM, preferably 10 uM; the activator of adenylyl cyclase Forskolin at a concentration of 0.5-50 uM, preferably 50uM; the retinoid receptor agonist arotinoid acid, otherwise known as 4-[(E)-2-(5,5,8,8-tetramethyl-6,7-dihydronaphthalen-2-yl)prop-1-enyl]benzoic acid, or TTNPB at a concentration of 1.0nM-5uM, preferably 5.0 uM; the EZH2 inhibitor (1S,2R,5R)-5-(4-aminoimidazo[4,5-c]pyridin-1-yl)-3-(hydroxymethyl)cyclopent-3-ene-1,2-diol also known as 3-Deazaneplanocin A (DZNep) at a concentration of 0.05-0.24 uM, preferably 0.1 uM; the inhibitor of the MEK/ERK pathway N-[(2R)-2,3-dihydroxypropoxy]-3,4-difluoro-2-(2-fluoro-4-iodoanilino)benzamide also known as PD0325901 is applied at a concentration of 0.1 nM-1.0 uM, preferably 1.0 uM; SB431542 at a concentration of preferably 10.0 uM; A 83-01 at a concentration of preferably 1.0 uM; Sodium 4-Phenylbutyrate at a concentration of preferably 1.0 uM; Romidepsin at a concentration of preferably 10 nM; trichostatin A at a concentration of preferably 250 nM; SAHA also known as vorinostat at a concentration of preferably 1.0 uM; LAQ824 at a concentration of preferably 50 nM; panobionstat at a concentration of preferably 10-100 nM; MS-275 at a concentration of preferably 5.0 uM; Mocetinostat at a concentration of preferably 1.0 uM; CI-994 at a concentration of preferably 40 uM; BIX01294 at a concentration of preferably 2.0 uM; PD032590 at a concentration of preferably 0.1-1.0 uM; lithium salt at a concentration of preferably 200 nM; NVP-BEZ235 at a concentration of preferably 100 nM; SB216763 at a concentration of preferably 20 uM; SB203580 at a concentration of preferably 0.6 uM; the protein kinase R-like ER kinase (PERK) inhibitor GSK2606414 at a concentration of preferably 0.1 nM-1.0 mM, preferably 100 nM; 2-Hydroxy-1-naphthaldehyde at a concentration of preferably 10 nM; dihydrocoumarin at a concentration of preferably 1.0 mM; nicotinamide at a concentration of preferably 7.5 mM; sodium butyrate at a concentration of preferably 0.25 mM; RG108 at a concentration of preferably 5.0 uM; (+)-Sodium L-ascorbate at a concentration of preferably 50 ug/mL; Quercitin at a concentration of preferably 1.0 uM; Resveratrol at a concentration of preferably 10 uM; AM580 at a concentration of preferably 10 nM; CD437 at a concentration of preferably 0.1 uM; PS48 at a concentration of preferably 5.0 uM; DMOG at a concentration of preferably 10 uM; Deferoxamine at a concentration of preferably 0.5 uM - 0.5 mM; 5-azacytidine at a concentration of preferably 5.0 uM; Cyclosporin A at a concentration of preferably 400 ng/mL; EPZ 004777 at a concentration of preferably 5.0 uM; SGC0946 at a concentration of preferably 10 nM; LY-364947 at a concentration of preferably 1.0 uM; Kenpaullone at a concentration of preferably 5.0 uM; Dasatinib at a concentration of preferably 0.5 uM; PP1 at a concentration of preferably 10 uM; 2-Hydroxy-1-naphthaldehyde isonicotinoylhydrazone also known as AS 8351 at a concentration of preferably 100 uM; 1,5 isoquinolinediol at a concentration of preferably 20-50 uM; the protein factor GFER (growth factor augmenter of liver regeneration) at a concentration of preferably 10 ng/mL; the protein factor AMH (Anti-Mullerian Hormone) at a concentration of preferably 20 ng/mL; the protein factor fibroblast growth factor beta at a concentration of preferably 10 ng/mL; B18R at a concentration of preferably 100 ug/mL; Phorbol 12-myristate 13-acetate at a concentration of preferably 1.0 uM; LiC at a concentration of preferably 10 mM; metformin at a concentration of preferably 5.0 uM; Antimycin A at a concentration of preferably 2.5 nM; the protein insulin-like growth factor 1 (IGF1) at a concentration of preferably 100 ng/mL; the protein insulin-like growth factor 2 (IGF2) at a concentration of preferably 100 ng/mL; dehydroepiandrosterone (DHEA) at a concentration of preferably 50 uM; the protein growth hormone (GH) at a concentration of preferably 100 ng/mL; the steroid 17-b-estradiol at a concentration of preferably 5.0 nM; Wortmannin at a concentration of preferably 1.0 uM; the PKC activator (2S,5S)-(E,E)-8-(5-(4-(Trifluoromethyl)phenyl)-2,4-pentadienoylamino)benzolactam, PKC Activator V at a concentration of preferably 1.0-100 uM; the ketone body 3- hydroxybutyrate (3-HB) at a concentration of preferably 10 mM; the steroid testosterone at a concentration of preferably 1.0-10 uM; L-carnitine at a concentration of preferably 1.0 mM; RSC-133 at a concentration of preferably 10 uM; arginine N-methyltransferase inhibitor-1 (AMI-5) at a concentration of preferably 50 uM; the hedgehog pathway activator Hh-Ag1.5 at a concentration of preferably 1.0 uM; JNJ10198409 at a concentration of preferably 10 uM; LDE225 at a concentration of preferably 10 nM; the inhibitor of RasGAP and ERK1 SC-1 at a concentration of preferably 100 nM-10 uM; the small molecule enhancer of autophagy SMER28 at a concentration of preferably 50 uM; the PDGFR Tyrosine Kinase Inhibitor VII SU 16f at a concentration of preferably 50 uM; the inhibitor of the tyrosine kinase domains of VEGFR2, FGFR1, and PDGFRβ SU5402 at a concentration of preferably 10 uM; and mRNA for the reprogramming factors *OCT4, NANOG, LIN28A, LIN28B, SOX2, MYC, KLF4,* or *DNMT3B,* at a concentration of preferably 300 ng/mL individually or in combination with the aforementioned factors.

Compound libraries are available from a number of commercial vendors such as Tocris BioScience, Nanosyn, BioFocus, and from government entities. For example, the Molecular Libraries Small Molecule Repository (MLSMR), a component of the U.S. National Institutes of Health (NIH) Molecular Libraries Program is designed to identify, acquire, maintain, and distribute a collection of >300,000 chemically diverse compounds with known and unknown biological activities for use, e.g., in high-throughput screening (HTS) assays (see https://mli.nih.gov/mli/). The NIH Clinical Collection (NCC) is a plated array of approximately 450 small molecules that have a history of use in human clinical trials. These compounds are highly drug-like with known safety profiles. In some embodiments, a collection of compounds comprising "approved human drugs" is tested. An "approved human drug" is a compound that has been approved for use in treating humans by a government regulatory agency such as the US Food and Drug Administration, European Medicines Evaluation Agency, or a similar agency responsible for evaluating at least the safety of therapeutic agents prior to allowing them to be marketed. The test compound may be, e.g., an antineoplastic, antibacterial, antiviral, antifungal, antiprotozoal, antiparasitic, antidepressant, antipsychotic, anesthetic, antianginal, antihypertensive, antiarrhythmic, antiinflammatory, analgesic, antithrombotic, antiemetic, immunomodulator, antidiabetic, lipid- or cholesterol-lowering (e.g., statin), anticonvulsant, anticoagulant, antianxiety, hypnotic (sleep-inducing), hormonal, or anti-hormonal drug, etc. In some embodiments, a compound is one that has undergone at least some preclinical or clinical development or has been determined or predicted to have "drug-like" properties. For example, the test compound may have completed a Phase I trial or at least a preclinical study in non-human animals and shown evidence of safety and tolerability.

In some embodiments, a test compound is substantially non-toxic to cells of an organism to which the compound may be administered and/or to cells with which the compound may be tested, at the concentration to be used or, in some embodiments, at concentrations up to 10-fold, 100-fold, or 1,000-fold higher than the concentration to be used. For example, there may be no statistically significant effect on cell viability and/or proliferation, or the reduction in viability or proliferation can be no more than 1%, 5%, or 10% in various embodiments. Cytotoxicity and/or effect on cell proliferation can be assessed using any of a variety of assays. For example, a cellular metabolism assay such as AlamarBlue, MTT, MTS, XTT, and CellTitre Glo assays, a cell membrane integrity assay, a cellular ATP-based viability assay, a mitochondrial reductase activity assay, a BrdU, EdU, or H3-Thymidine incorporation assay could be used. In some embodiments, a test compound is not a compound that is found in a cell culture medium known or used in the art, e.g., culture medium suitable for culturing vertebrate, e.g., mammalian cells or, if the test compound is a compound that is found in a cell culture medium known or used in the art, the test compound is used at a different, e.g., higher, concentration when used in a method of the present invention.

### Assays for Global modulators of iTR

### Aspects of Assay Implementation and Controls

Various inventive screening assays described above involve determining whether a test compound inhibits the levels of active TR inhibitors or increases the levels of active TR activators. Suitable cells for expression of a reporter molecule are described above. In performing an inventive assay, assay components (e.g., cells, TR activator or TR inhibitor polypeptide, and test compounds) are typically dispensed into multiple vessels or other containers. Any type of vessel or article capable of containing cells can be used. In many embodiments of the invention, the vessels are wells of a multi-well plate (also called a "microwell plate", "microtiter plate", etc. For purposes of description, the term "well" will be used to refer to any type of vessel or article that can be used to perform an inventive screen, e.g., any vessel or article that can contain the assay components. It should be understood that the invention is not limited to use of wells or to use of multi-well plates. In some embodiments, any article of manufacture in which multiple physically separated cavities (or other confining features) are present in or on a substrate can be used. For example, assay components can be confined in fluid droplets, which may optionally be arrayed on a surface and, optionally, separated by a water-resistant substance that confines the droplets to discrete locations, in channels of a microfluidic device, etc.

In general, assay components can be added to wells in any order. For example, cells can be added first and maintained in culture for a selected time period (e.g., between 6 and 48 hours) prior to addition of a test compound and target TR activator or TR inhibitor polypeptides or cells with express constructs to a well. In some embodiments, compounds are added to wells prior to addition of polypeptides of cells. In some embodiments, expression of a reporter polypeptide is induced after plating the cells, optionally after addition of a test compound to a well. In some embodiments, expression of the reporter molecule is achieved by transfecting the cells with an expression vector that encodes the reporter polypeptide. In some embodiments, the cells have previously been genetically engineered to express the reporter polypeptide. In some embodiments, expression of the reporter molecule is under control of regulatable expression control elements, and induction of expression of the reporter molecule is achieved by contacting the cells with an agent that induces (or derepresses) expression.

The assay composition comprising cells, test compound, or polypeptide is maintained for a suitable time period during which test compound may (in the absence of a test compound that inhibits its activity) cause an increase or decrease of the level or activity of the target TR activator or TR inhibitor. The number of cells, amount of TR activator or TR inhibitor polypeptide, and amount of test compound to be added will depend, e.g., on factors such as the size of the vessel, cell type, and can be determined by one of ordinary skill in the art. In some embodiments, the ratio of the molar concentration of TR activator or TR inhibitor polypeptide to test compound is between 1: 10 and 10: 1. In some embodiments, the number of cells, amount of test compound, and length of time for which the composition is maintained can be selected so that a readily detectable level signal after a selected time period in the absence of a test compound. In some embodiments, cells are at a confluence of about 25%-75%, e.g., about 50%, at the time of addition of compounds. In some embodiments, between 1,000 and 10,000 cells/well (e.g., about 5,000 cells/well) are plated in about 100 µl medium per well in 96-well plates. In other exemplary embodiments, cells are seeded in about 30 µl-50 µl of medium at between 500 and 2,000 (e.g., about 1000) cells per well into 384-well plates. In some embodiments, compounds are tested at multiple concentrations (e.g., 2-10 different concentrations) and/or in multiple replicates (e.g., 2-10 replicates). Multiple replicates of some or all different concentrations can be performed. In some embodiments, candidate TR factors are used at a concentration between 0.1 µg/ml and 100 µg/ml, e.g., 1 µg/ml and 10 µg/ml. In some embodiments, candidate TR factors are used at multiple concentrations. In some embodiments, compounds are added to cells between 6 hours and one day (24 hr) after seeding. In some aspects of any of the inventive compound screening and/or characterization methods, a test compound is added to an assay composition in an amount sufficient to achieve a predetermined concentration. In some embodiments, the concentration is up to about 1 nM. In some embodiments, the concentration is between about 1 nM and about 100 nM. In some embodiments, the concentration is between about 100 nM and about 10 µM. In some embodiments the concentration is at least 10 µM, e.g., between 10 µM and 100 µM. The assay composition can be maintained for various periods of time following addition of the last component thereof. In certain embodiments the assay composition is maintained for between about 10 minutes and about 4 days, e.g., between 1 hour and 3 days, e.g., between 2 hours and 2 days, or any intervening range or particular value, e.g., about 4-8 hours, after addition of all components. Multiple different time points can be tested. Additional aliquots of test compound can be added to the assay composition within such time period. In some embodiments, cells are maintained in cell culture medium appropriate for culturing cells of that type. In some embodiments, a serum-free medium is used. In some embodiments, the assay composition comprises a physiologically acceptable liquid that is compatible with maintaining integrity of the cell membrane and, optionally, cell viability, instead of cell culture medium. Any suitable liquid could be used provided it has the proper osmolarity and is otherwise compatible with maintaining reasonable integrity of the cell membrane and, optionally, cell viability, for at least a sufficient period of time to perform an assay. One or more measurements indicative of an increase in the level of active TR activator or decrease in TR inhibitor can be made during or following the incubation period.

In some embodiments, the compounds screened for potential to be global modulators of iTR are chosen from agents capable in other conditions of inducing pluripotency in somatic cell types. Such agents include the following compounds individually or in combination: the genes *OCT4, SOX2, KLF4, NANOG, ESRRB, NR5A2, CEBPA, MYC, LIN28A* and *LIN28B* alone and in combination with small molecule compounds such as combinations of the following compounds: inhibitors of glycogen synthase 3 (GSK3) including but not limited to CHIR99021; inhibitors of TGF-beta signaling including but not limited to SB431542, A-83-01, and E616452; HDAC inhibitors including but not limited to aliphatic acid compounds including but not limited to: valproic acid, phenylbutyrate, and n-butyrate; cyclic tetrapeptides including trapoxin B and the depsipeptides; hydroxamic acids such as trichostatin A, vorinostat (SAHA), belinostat (PXD101), LAQ824, panobinostat (LBH589), and the benzamides entinostat (MS-275), CI994, mocetinostat (MGCD0103); those specifically targeting Class I *(HDAC1, HDAC2, HDAC3,* and *HDAC8),* IIA (*HDAC4, HDAC5, HDAC7,* and *HDAC9*), IIB (*HDAC6* and *HDAC10*), III (*SIRT1, SIRT2, SIRT3, SIRT4, SIRT5, SIRT6,* or *SIRT7*) including the sirtuin inhibitors nicotinomide, diverse derivatives of NAD, dihydrocoumarin, naphthopyranone, and 2-hydroxynaphthaldehydes, or IV (*HDAC11*) deacetylases; inhibitors of H3K4/9 histone demethylase LSD1 including but not limited to parnate; inhibitors of Dot1L including but not limited to EPZ004777; inhibitors of G9a including but not limited to Bix01294; inhibitors of *EZH2* including but not limited to DZNep, inhibitors of DNA methyltransferase including but not limited to RG108; 5-aza-2'deoxycytidine (trade name Vidaza and Azadine); vitamin C which can inhibit DNA methylation, increase Tet1 which increases 5hmC which is a first step of demethylation; activators of 3' phosphoinositide-dependent kinase 1 including but not limited to PS48; promoters of glycolysis including but not limited to Quercetin and fructose 2, 6-bisphosphate (an activator of phosphofructokinase 1); agents that promote the activity of the HIF1 transcription complex including but not limited to Quercetin; RAR agonists including but not limited to AM580, CD437, and TTNPB; agents that mimic hypoxia including but not limited to Resveratrol; agents that increase telomerase activity including but not limited to the exogenous expression of the catalytic component of telomerase *(TERT)*, agents that promote epigenetic modifications via downregulation of LSD1, a H3K4-specific histone demethylase including but not limited to lithium; or inhibitors of the MAPK/ERK pathway including but not limited to PD032590. Such compounds may be administered in diverse combinations, concentrations, and for differing periods of time, to optimize the effect of iTR on cells cultured *in vitro* using markers of global iTR such as by assaying for decreased expression of *COX7A1* or *NAALADL1,* or other inhibitors of iTR as described herein, and/or assaying for increased expresson of *PCDHB2* or *AMH* or other activators or iTR as described herein, or in injured or diseased tissues *in vivo,* or in modulating the lifespan of animals *in vivo.*

In some embodiments, individual compounds, each typically of known identity (e.g., structure and/or sequence), are added to each of a multiplicity of wells. In some embodiments, two or more compounds may be added to one or more wells. In some embodiments, one or more compounds of unknown identity may be tested. The identity may be determined subsequently using methods known in the art.

In various embodiments, foregoing assay methods of the invention are amenable to high-throughput screening (HTS) implementations. In some embodiments, the screening assays of the invention are high throughput or ultra high throughput (see, e.g., Fernandes, P. B., Curr Opin Chem. Biol. 1998, 2:597; Sundberg, S A, Curr Opin Biotechnol. 2000, 11:47). High throughput screens (HTS) often involve testing large numbers of compounds with high efficiency, e.g., in parallel. For example, tens or hundreds of thousands of compounds can be routinely screened in short periods of time, e.g, hours to days. In some embodiments, HTS refers to testing of between 1,000 and 100,000 compounds per day. In some embodiments, ultra high throughput refers to screening in excess of 100,000 compounds per day, e.g., up to 1 million or more compounds per day. The screening assays of the invention may be carried out in a multi-well format, for example, a 96-well, 384-well format, 1,536-well format, or 3,456-well format and are suitable for automation. In some embodiments, each well of a microwell plate can be used to run a separate assay against a different test compound or, if concentration or incubation time effects are to be observed, a plurality of wells can contain test samples of a single compound, with at least some wells optionally being left empty or used as controls or replicates. Typically, HTS implementations of the assays disclosed herein involve the use of automation. In some embodiments, an integrated robot system including one or more robots transports assay microwell plates between multiple assay stations for compound, cell and/or reagent addition, mixing, incubation, and readout or detection. In some aspects, an HTS system of the invention may prepare, incubate, and analyze many plates simultaneously. Suitable data processing and control software may be employed. High throughput screening implementations are well known in the art. Without limiting the invention in any way, certain general principles and techniques that may be applied in embodiments of a HTS of the present invention are described in Macarron R & Hertzberg R P. Design and implementation of high-throughput screening assays. Methods Mol Biol., 565:1-32, 2009 and/or An W F & Tolliday N J., Introduction: cell-based assays for high-throughput screening. Methods Mol Biol. 486:1-12, 2009, and/or references in either of these. Exemplary methods are also disclosed in High Throughput Screening: Methods and Protocols (Methods in Molecular Biology) by William P. Janzen (2002) and High-Throughput Screening in Drug Discovery (Methods and Principles in Medicinal Chemistry) (2006). An additional compound may, for example, have one or more improved pharmacokinetic and/or pharmacodynamic properties as compared with an initial hit or may simply have a different structure. An "improved property" may, for example, render a compound more effective or more suitable for one or more purposes described herein. In some embodiments, for example, a compound may have higher affinity for the molecular target of interest (e.g., TR activator or TR inhibitor gene products), lower affinity for a non-target molecule, greater solubility (e.g., increased aqueous solubility), increased stability (e.g., in blood, plasma, and/or in the gastrointestinal tract), increased half-life in the body, increased bioavailability, and/or reduced side effect(s), etc. Optimization can be accomplished through empirical modification of the hit structure (e.g., synthesizing compounds with related structures and testing them in cell-free or cell-based assays or in non-human animals) and/or using computational approaches. Such modification can, in some embodiments, make use of established principles of medicinal chemistry to predictably alter one or more properties. In some embodiments, one or more compounds that are "hit" are identified and subjected to systematic structural alteration to create a second library of compounds (e.g., refined lead compounds) structurally related to the hit. The second library can then be screened using any of the methods described herein. In some embodiments, an iTR factor is modified or incorporates a moiety that enhances cstability (e.g., in serum), increases half-life, reduces toxicity or immunogenicity, or otherwise confers a desirable property on the compound.

### Uses of iTR, iTM, and ICM Factors

### Pharmaceutical Compositions

iTR, iTM, senolytic, iS-CSC, and iCM factors have a variety of different uses. Nonlimiting examples of such uses are discussed herein. In some embodiments, an iTR factor is used to enhance regeneration of an organ or tissue. In some embodiments, an iTR factor is used to enhance regeneration of a limb, digit, cartilage, heart, blood vessel, bone, esophagus, stomach, liver, gallbladder, pancreas, intestines, rectum, anus, endocrine gland (e.g., thyroid, parathyroid, adrenal, endocrine portion of pancreas), skin, hair follicle, thymus, spleen, skeletal muscle, focal damaged cardiac muscle, smooth muscle, brain, spinal cord, peripheral nerve, ovary, fallopian tube, uterus, vagina, mammary gland, testes, vas deferens, seminal vesicle, prostate, penis, pharynx, larynx, trachea, bronchi, lungs, kidney, ureter, bladder, urethra, eye (e.g., retina, cornea), or ear (e.g., organ of Corti). In some embodiments, an iTR factor is used to enhance regeneration of a stromal layer, e.g., a connective tissue supporting the parenchyma of a tissue. In some embodiments, an iTR factor is used to enhance regeneration following surgery, e.g., surgery that entails removal of at least a portion of a diseased or damaged tissue, organ, or other structure such as a limb, digit, etc. For example, such surgery might remove at least a portion of a liver, lung, kidney, stomach, pancreas, intestine, mammary gland, ovary, testis, bone, limb, digit, muscle, skin, etc. In some embodiments, the surgery is to remove a tumor. In some embodiments, an iTR factor is used to promote scarless regeneration of skin following trauma, surgery, disease, and burns.

Enhancing regeneration can include any one or more of the following, in various embodiments: (a) increasing the rate of regeneration; (b) increasing the extent of regeneration; (c) promoting establishment of appropriate structure (e.g., shape, pattern, tissue architecture, tissue polarity) in a regenerating tissue or organ or other body structure; (d) promoting growth of new tissue in a manner that retains and/or restores function. While use of an iTR factor to enhance regeneration is of particular interest, the invention encompasses use of an iTR factor to enhance repair or wound healing in general, without necessarily producing a detectable enhancement of regeneration. Thus, the invention provides methods of enhancing repair or wound healing, wherein an iTR factor is administered to a subject in need thereof according to any of the methods described herein.

Numerous aspects of aging and age-related disease are taught in the present invention to addressable with iTR therapy. These manifestations of aging include age-related vascular dysfunction including peripheral vascular, coronary, and cerebrovascular disease; musculoskeletal disorders including osteoarthritis, intervertebral disc degeneration, bone fractures, tendon and ligament tears, and limb regeneration; neurological disorders including stroke and spinal cord injuries; muscular disorders including muscular dystrophy, sarcopenia, myocardial infarction, and heart failure; endocrine disorders including Type I diabetes, Addison's disease, hypothyroidism, and pituitary insufficiency; digestive disorders including pancreatic exocrine insufficiency; ocular disorders including macular degeneration, retinitis pigmentosa, and neural retinal degeneration disorders; dermatological conditions including skin burns, lacerations, surgical incisions, alopecia, graying of hair, and skin aging; pulmonary disorders including emphysema and interstitial fibrosis of the lung; auditory disorders including hearing loss; and hematological disorders such as aplastic anemia and failed hematopoietic stem cell grafts.

In some embodiments, the invention provides the use of an effective amount of an iTR factor to the subject in a method of enhancing regeneration in a subject in need thereof. In some embodiments, an effective amount of a compound (e.g., an iTR factor) is an amount that results in an increased rate or extent of regeneration of damaged tissue as compared with a reference value (e.g., a suitable control value). In some embodiments, the reference value is the expected (e.g., average or typical) rate or extent of regeneration in the absence of the compound (optionally with administration of a placebo). In some embodiments, an effective amount of an iTR factor is an amount that results in an improved structural and/or functional outcome as compared with the expected (e.g., average or typical) structural or functional outcome in the absence of the compound. In some embodiments, an effective amount of a compound, e.g., an iTR factor, results in enhanced blastema formation and/or reduced scarring. Extent or rate of regeneration can be assessed based on dimension(s) or volume of regenerated tissue, for example. Structural and/or functional outcome can be assessed based on, e.g., visual examination (optionally including use of microscopy or imaging techniques such as X-rays, CT scans, MRI scans, PET scans) and/or by evaluating the ability of the tissue, organ, or other body part to perform one or more physiological processes or task(s) normally performed by such tissue, organ, or body part. Typically, an improved structural outcome is one that more closely resembles normal structure (e.g., structure that existed prior to tissue damage or structure as it exists in a normal, healthy individual) as compared with the structural outcome that would be expected (e.g., average or typical outcome) in the absence of treatment with an iTR factor. One of ordinary skill in the art can select an appropriate assay or test for function. In some embodiments, an increase in the rate or extent of regeneration as compared with a control value is statistically significant (e.g., with a p value of <0.05, or with a p value of <0.01) and/or clinically significant. In some embodiments, an improvement in structural and/or functional outcome as compared with a control value is statistically significant and/or clinically significant. "Clinically significant improvement" refers to an improvement that, within the sound judgement of a medical or surgical practitioner, confers a meaningful benefit on a subject (e.g., a benefit sufficient to make the treatment worthwhile). It will be appreciated that in many embodiments an iTR modulator, e.g., an iTR factor, administered to a subject of a particular species (e.g., for therapeutic purposes) is a compound that modulates, e.g., inhibits, the endogenous TR genes expressed in subjects of that species. For example, if a subject is human, a compound that inhibits the activity of human TR inhibitor gene products and activates the activity of human TR activator gene products would typically be administered.

In some embodiments, the iTR factor is used to enhance skin regeneration, e.g., after a burn (thermal or chemical), scrape injury, or other situations involving skin loss, e.g., infections such as necrotizing fasciitis or purpura fulminans. In some embodiments, a burn is a second or third degree burn. In some embodiments, a region of skin loss has an area of at least 10 cm². In one aspect, an iTR factor enhances regeneration of grafted skin. In one aspect, an iTR factor reduces excessive and/or pathological wound contraction or scarring.

In some embodiments, an iTR factor is used to enhance bone regeneration, e.g., in a situation such as non-union fracture, implant fixation, periodontal or alveolar ridge augmentation, craniofacial surgery, or other conditions in which generation of new bone is considered appropriate. In some embodiments, an iTR factor is applied to a site where bone regeneration is desired. In some embodiments, an iTR factor is incorporated into or used in combination with a bone graft material. Bone graft materials include a variety of ceramic and proteinaceous materials. Bone graft materials include autologous bone (e.g., bone harvested from the iliac crest, fibula, ribs, etc.), allogeneic bone from cadavers, and xenogeneic bone. Synthetic bone graft materials include a variety of ceramics such as calcium phosphates (e.g. hydroxyapatite and tricalcium phosphate), bioglass, and calcium sulphate, and proteinaceous materials such as demineralized bone matrix (DBM). DBM can be prepared by grinding cortical bone tissues (generally to 100-500 µm sieved particle size), then treating the ground tissues with hydrochloric acid (generally 0.5 to 1 N). In some embodiments, an iTR factor is administered to a subject together with one or more bone graft materials. The iTR factor may be combined with the bone graft material (in a composition comprising an iTR factor and a bone graft material) or administered separately, e.g., after placement of the graft. In some embodiments, the invention provides a bone paste comprising an iTR factor. Bone pastes are products that have a suitable consistency and composition such that they can be introduced into bone defects, such as voids, gaps, cavities, cracks etc., and used to patch or fill such defects, or applied to existing bony structures. Bone pastes typically have sufficient malleability to permit them to be manipulated and molded by the user into various shapes. The desired outcome of such treatments is that bone formation will occur to replace the paste, e.g., retaining the shape in which the paste was applied. The bone paste provides a supporting structure for new bone formation and may contain substance(s) that promote bone formation. Bone pastes often contain one or more components that impart a paste or putty-like consistency to the material, e.g., hyaluronic acid, chitosan, starch components such as amylopectin, in addition to one or more of the ceramic or proteinaceous bone graft materials (e.g., DBM, hydroxyapatite) mentioned above.
In some embodiments, an iTR factor enhances the formation and/or recruitment of osteoprogenitor cells from undifferentiated mesechymal cells and/or enhances the differentiation of osteoprogenitor cells into cells that form new bone (osteoblasts).
In some embodiments, an iTR factor is administered to a subject with osteopenia or osteoporosis, e.g., to enhance bone regeneration in the subject.

In some embodiments, an iTR factor is used to enhance regeneration of a joint (e.g., a fibrous, cartilaginous, or synovial joint). In some embodiments, the joint is an intervertebral disc. In some embodiments, a joint is a hip, knee, elbow, or shoulder joint. In some embodiments, an iTR factor is used to enhance regeneration of dental and/or periodontal tissues or structures (e.g., pulp, periodontal ligament, teeth, periodontal bone).
In some embodiments, an iTR factor is used to reduce glial scarring in CNS and PNS injuries.
In some embodiments, an iTR factor is used to reduce adhesions and stricture formation in internal surgery.

In some embodiments, an iTR factor is used to decrease scarring in tendon and ligament repair improving mobility.

In some embodiments, an iTR factor is used to reduce vision loss following eye injury.

In some embodiments, an iTR factor is administered to a subject in combination with cells. The iTR factor and the cells may be administered separately or in the same composition. If administered separately, they may be administered at the same or different locations. The cells can be autologous, allogeneic, or xenogeneic in various embodiments. The cells can comprise progenitor cells or stem cells, e.g., adult stem cells. As used herein, a stem cell is a cell that possesses at least the following properties: (i) self-renewal, i.e., the ability to go through numerous cycles of cell division while still maintaining an undifferentiated state; and (ii) multipotency or multidifferentiative potential, i.e., the ability to generate progeny of several distinct cell types (e.g., many, most, or all of the distinct cell types of a particular tissue or organ). An adult stem cell is a stem cell originating from non-embryonic tissues (e.g., fetal, post-natal, or adult tissues). As used herein, the term "progenitor cell" encompasses cells multipotencand cells that are more differentiated than pluripotent stem cells but not fully differentiated. Such more differentiated cells (which may arise from embryonic progenitor cells) have reduced capacity for self-renewal as compared with embryonic progenitor cells. In some embodiments, an iTR factor is administered in combination with mesenchymal progenitor cells, neural progenitor cells, endothelial progenitor cells, hair follicle progenitor cells, neural crest progenitor cells, mammary stem cells, lung progenitor cells (e.g., bronchioalveolar stem cells), muscle progenitor cells (e.g., satellite cells), adipose-derived progenitor cells, epithelial progenitor cells (e.g., keratinocyte stem cells), and/or hematopoietic progenitor cells (e.g., hematopoietic stem cells). In some embodiments, the cells comprise induced pluripotent stem cells (iPS cells), or cells that have been at least partly differentiated from iPS cells. In some embodiments, the progenitor cells comprise adult stem cells. In some embodiments, at least some of the cells are differentiated cells, e.g., chondrocytes, osteoblasts, keratinocytes, hepatocytes. In some embodiments, the cells comprise myoblasts.

In some embodiments, an iTR factor is administered in a composition (e.g., a solution) comprising one or more compounds that polymerizes or becomes cross-linked or undergoes a phase transition *in situ* following administration to a subject, typically forming a hydrogel. The composition may comprise monomers, polymers, initiating agents, cross-linking agents, etc. The composition may be applied (e.g., using a syringe) to an area where regeneration is needed, where it forms a gel *in situ,* from which an iTR factor is released over time. Gelation may be triggered, e.g., by contact with ions in body fluids or by change in temperature or pH, or by light, or by combining reactive precursors (e.g., using a multi-barreled syringe). (See, e.g., U.S. Pat. No. 6,129,761; Yu L, Ding J. Injectable hydrogels as unique biomedical materials. Chem Soc Rev. 37(8):1473-81 (2008)). In some embodiments, the hydrogel is a hyaluronic acid or hyaluronic acid and collagen I-containing hydrogel such as HyStem-C described herein. In some embodiments, the composition further comprises cells.

In some embodiments, an iTR factor is administered to a subject in combination with vectors expressing the catalytic component of telomerase. The vector may be administered separately or in the same composition. If administered separately, they may be administered at the same or different locations. The vector may express the telomerase catalytic component from the same species as the treated tissue or from another species. Said co-administration of the iTR factor with the telomerase catalytic component is particularly useful wherein the target tissue is from an aged individual and said individual is from the human species. Other inventive methods comprise use of an iTR factor in the *ex vivo* production of living, functional tissues, organs, or cell-containing compositions to repair or replace a tissue or organ lost due to damage. For example, cells or tissues removed from an individual (either the future recipient, an individual of the same species, or an individual of a different species) may be cultured *in vitro,* optionally with an matrix, scaffold (e.g., a three dimensional scaffold) or mold (e.g., comprising a biocompatible, optionally biodegradable, material, e.g., a polymer such as HyStem-C), and their development into a functional tissue or organ can be promoted by contacting an iTR factor. The scaffold, matrix, or mold may be composed at least in part of naturally occurring proteins such as collagen, hyaluronic acid, or alginate (or chemically modified derivatives of any of these), or synthetic polymers or copolymers of lactic acid, caprolactone, glycolic acid, etc., or self-assembling peptides, or decellularized matrices derived from tissues such as heart valves, intestinal mucosa, blood vessels, and trachea. In some embodiments, the scaffold comprises a hydrogel. The scaffold may, in certain embodiments, be coated or impregnated with an iTR factor, which may diffuse out from the scaffold over time. After production *ex vivo,* the tissue or organ is grafted into or onto a subject. For example, the tissue or organ can be implanted or, in the case of certain tissues such as skin, placed on a body surface. The tissue or organ may continue to develop *in vivo.* In some embodiments, the tissue or organ to be produced at least in part *ex vivo* is a bladder, blood vessel, bone, fascia, liver, muscle, skin patch, etc. Suitable scaffolds may, for example, mimic the extracellular matrix (ECM). Optionally, an iTR factor is administered to the subject prior to, during, and/or following grafting of the *ex vivo* generated tissue or organ. In some aspects, a biocompatible material is a material that is substantially non-toxic to cells *in vitro* at the concentration used or, in the case of a material that is administered to a living subject, is substantially nontoxic to the subject's cells in the quantities and at the location used and does not elicit or cause a significant deleterious or untoward effect on the subject, e.g., an immunological or inflammatory reaction, unacceptable scar tissue formation, etc. It will be understood that certain biocompatible materials may elicit such adverse reactions in a small percentage of subjects, typically less than about 5%, 1%, 0.5%, or 0.1%.

In some embodiments, a matrix or scaffold coated or impregnated with an iTR factor or combinations of factors including those capable of causing a global pattern of iTR gene expression is implanted, optionally in combination with cells, into a subject in need of regeneration. The matrix or scaffold may be in the shape of a tissue or organ whose regeneration is desired. The cells may be stem cells of one or more type(s) that gives rise to such tissue or organ and/or of type(s) found in such tissue or organ.

In some embodiments, an iTR factor or combination of factors is administered directly to or near a site of tissue damage. "Directly to a site of tissue damage" encompasses injecting a compound or composition into a site of tissue damage or spreading, pouring, or otherwise directly contacting the site of tissue damage with the compound or composition. In some embodiments, administration is considered "near a site of tissue damage" if administration occurs within up to about 10 cm away from a visible or otherwise evident edge of a site of tissue damage or to a blood vessel (e.g., an artery) that is located at least in part within the damaged tissue or organ. Administration "near a site of tissue damage" is sometimes administration within a damaged organ, but at a location where damage is not evident. In some embodiments, following damage or loss of a tissue, organ, or other structure, an iTR factor is applied to the remaining portion of the tissue, organ, or other structure. In some embodiments, an iTR factor is applied to the end of a severed digit or limb) that remains attached to the body, to enhance regeneration of the portion that has been lost. In some embodiments, the severed portion is reattached surgically, and an iTR factor is applied to either or both faces of the wound. In some embodiments, an iTR factor is administered to enhance engraftment or healing or regeneration of a transplanted organ or portion thereof. In some embodiments, an iTR factor is used to enhance nerve regeneration. For example, an iTR factor may be infused into a severed nerve, e.g., near the proximal and/or distal stump. In some embodiments, an iTR factor is placed within an artificial nerve conduit, a tube composed of biological or synthetic materials within which the nerve ends and intervening gap are enclosed. The factor or factors may be formulated in a matrix to facilitate their controlled release over time. Said matrix may comprise a biocompatible, optionally biodegradable, material, e.g., a polymer such as that comprised of hyaluronic acid, including crosslinked hyaluronic acid or carboxymethyl hyaluronate crosslinked with PEGDA, or a mixture of carboxymethyl hyaluronate crosslinked by PEGDA with carboxymethyl-modified gelatin (HyStem-C).
In some embodiments, the iTR factor is AgeX1547 described herein and may or may not be formulated for localization and slow release in carboxymethyl hyaluronate crosslinked by PEGDA with carboxymethyl-modified gelatin (HyStem-C) to induce iTR. iTM and iCM factors such as exosomes derived from fetal or adult cells can be administered in physiological solutions such as saline, or slow-released in carboxymethyl hyaluronate crosslinked by PEGDA with carboxymethyl-modified gelatin (HyStem-C) to induce iTM or iCM.

In some embodiments, an iTR factor or combinations of factors is used to promote production of hair follicles and/or growth of hair. In some embodiments, an iTR factor triggers regeneration of hair follicles from epithelial cells that do not normally form hair. In some embodiments, an iTR factor is used to treat hair loss, hair sparseness, partial or complete baldness in a male or female. In some embodiments, baldness is the state of having no or essentially no hair or lacking hair where it often grows, such as on the top, back, and/or sides of the head. In some embodiments, hair sparseness is the state of having less hair than normal or average or, in some embodiments, less hair than an individual had in the past or, in some embodiments, less hair than an individual considers desirable. In some embodiments, an iTR factor is used to promote growth of eyebrows or eyelashes. In some embodiments, an iTR factor is used to treat androgenic alopecia or "male pattern baldness" (which can affect males and females). In some embodiments, an iTR factor is used to treat alopecia areata, which involves patchy hair loss on the scalp, alopecia totalis, which involves the loss of all head hair, or alopecia universalis, which involves the loss of all hair from the head and the body. In some embodiments, an iTR factor is applied to a site where hair growth is desired, e.g., the scalp or eyebrow region. In some embodiments, an iTR factor is applied to or near the edge of the eyelid, to promote eyelash growth. In some embodiments, an iTR factor is applied in a liquid formulation. In some embodiments, an iTR factor is applied in a cream, ointment, paste, or gel. In some embodiments, an iTR factor is used to enhance hair growth after a burn, surgery, chemotherapy, or other event causing loss of hair or hear-bearing skin. In some embodiments, an iTR factor or combination of factors are administered to tissues afflicted with age-related degenerative changes to regenerate youthful function. Said age-related degenerative changes includes by way of nonlimiting example, age-related macular degeneration, coronary disease, osteoporosis, osteonecrosis, heart failure, emphysema, peripheral artery disease, vocal cord atrophy, hearing loss, Alzheimer's disease, Parkinson's disease, skin ulcers, and other age-related degenerative diseases. In some embodiments, said iTR factors are co-administered with a vector expressing the catalytic component of telomerase to extend cell lifespan.

In some embodiments, an iTR factor or factors are administered to enhance replacement of cells that have been lost or damaged due to insults such as chemotherapy, radiation, or toxins. In some embodiments, such cells are stromal cells of solid organs and tissues. Inventive uses can include a step of identifying or providing a subject suffering from or at risk of a disease or condition in which in which enhancing regeneration would be of benefit to the subject. In some embodiments, the subject has experienced injury (e.g., physical trauma) or damage to a tissue or organ. In some embodiments, the damage is to a limb or digit. In some embodiments, a subject suffers from a disease affecting the cardiovascular, digestive, endocrine, musculoskeletal, gastrointestinal, hepatic, integumentary, nervous, respiratory, or urinary system. In some embodiments, tissue damage is to a tissue, organ, or structure such as cartilage, bone, heart, blood vessel, esophagus, stomach, liver, gallbladder, pancreas, intestines, rectum, anus, endocrine gland, skin, hair follicle, tooth, gum, lip, nose, mouth, thymus, spleen, skeletal muscle, smooth muscle, joint, brain, spinal cord, peripheral nerve, ovary, fallopian tube, uterus, vagina, mammary gland, testes, vas deferens, seminal vesicle, prostate, penis, pharynx, larynx, trachea, bronchi, lungs, kidney, ureter, bladder, urethra, eye (e.g., retina, cornea), or ear (e.g., organ of Corti).

In some embodiments a compound or composition is administered to a subject at least once within approximately 2, 4, 8, 12, 24, 48, 72, or 96 hours after a subject has suffered tissue damage (e.g., an injury or an acute disease-related event such as a myocardial infarction or stroke) and, optionally, at least once thereafter. In some embodiments, a compound or composition is administered to a subject at least once within approximately 1-2 weeks, 2-6 weeks, or 6-12 weeks, after a subject has suffered tissue damage and, optionally, at least once thereafter.

In some embodiments of the invention, it may useful to stimulate or facilitate regeneration or de novo development of a missing or hypoplastic tissue, organ, or structure by, for example, removing the skin, removing at least some tissue at a site where regeneration or de novo development is desired, abrading a joint or bone surface where regeneration or de novo development is desired, and/or inflicting another type of wound on a subject. In the case of regeneration after tissue damage, it may be desirable to remove (e.g., by surgical excision or debridement) at least some of the damaged tissue. In some embodiments, an iTR factor is administered at or near the site of such removal or abrasion. In some embodiments, an iTR factor is used to enhance generation of a tissue or organ in a subject in whom such tissue or organ is at least partially absent as a result of a congenital disorder, e.g., a genetic disease. Many congenital malformations result in hypoplasia or absence of a variety of tissues, organs, or body structures such as limbs or digits. In other instances, a developmental disorder resulting in hypoplasia of a tissue, organ, or other body structure becomes evident after birth. In some embodiments, an iTR factor is administered to a subject suffering from hypoplasia or absence of a tissue, organ, or other body structure, in order to stimulate growth or development of such tissue, organ, or other body structure. In some aspects, the invention provides a method of enhancing generation of a tissue, organ, or other body structure in a subject suffering from hypoplasia or congenital absence of such tissue, organ, or other body structure, the method comprising administering an iTR factor to the subject. In some embodiments, an iTR factor is administered to the subject prior to birth, i.e., in utero. The various aspects and embodiments of the invention described herein with respect to regeneration are applicable to such de novo generation of a tissue, organ, or other body structure and are encompassed within the invention.

In some aspects, an iTR factor is used to enhance generation of tissue in any of a variety of situations in which new tissue growth is useful at locations where such tissue did not previously exist. For example, generating bone tissue between joints is frequently useful in the context of fusion of spinal or other joints.

iTR factors may be tested in a variety of animal models of regeneration. In one aspect, a modulator of iTR is tested in murine species. For example, mice can be wounded (e.g., by incision, amputation, transection, or removal of a tissue fragment). An iTR factor is applied to the site of the wound and/or to a removed tissue fragment and its effect on regeneration is assessed. The effect of a modulator of vertebrate TR can be tested in a variety of vertebrate models for tissue or organ regeneration. For example, fin regeneration can be assessed in zebrafish, e.g., as described in (Mathew L K, Unraveling tissue regeneration pathways using chemical genetics. J Biol Chem. 282(48):35202-10 (2007)), and can serve as a model for limb regeneration. Rodent, canine, equine, caprine, fish, amphibian, and other animal models useful for testing the effects of treatment on regeneration of tissues and organs such as heart, lung, limbs, skeletal muscle, bone, etc., are widely available. For example, various animal models for musculoskeletal regeneration are discussed in Tissue Eng Part B Rev. 16(1) (2010). A commonly used animal model for the study of liver regeneration involves surgical removal of a larger portion of the rodent liver. Other models for liver regeneration include acute or chronic liver injury or liver failure caused by toxins such as carbon tetrachloride. In some embodiments, a model for hair regeneration or healing of skin wounds involves excising a patch of skin, e.g., from a mouse. Regeneration of hair follicles, hair growth, re-epithelialization, gland formation, etc., can be assessed.

The compounds and compositions disclosed herein and/or identified using a method and/or assay system described herein may be administered by any suitable means such as orally, intranasally, subcutaneously, intramuscularly, intravenously, intra-arterially, parenterally, intraperitoneally, intrathecally, intratracheally, ocularly, sublingually, vaginally, rectally, dermally, or by inhalation, e.g., as an aerosol. The particular mode selected will depend, of course, upon the particular compound selected, the particular condition being treated and the dosage required for therapeutic efficacy. The methods of this invention, generally speaking, may be practiced using any mode of administration that is medically or veterinarily acceptable, meaning any mode that produces acceptable levels of efficacy without causing clinically unacceptable (e.g., medically or veterinarily unacceptable) adverse effects. Suitable preparations, e.g., substantially pure preparations, of one or more compound(s) may be combined with one or more pharmaceutically acceptable carriers or excipients, etc., to produce an appropriate pharmaceutical composition suitable for administration to a subject. Such pharmaceutically acceptable compositions are an aspect of the invention. The term "pharmaceutically acceptable carrier or excipient" refers to a carrier (which term encompasses carriers, media, diluents, solvents, vehicles, etc.) or excipient which does not significantly interfere with the biological activity or effectiveness of the active ingredient(s) of a composition and which is not excessively toxic to the host at the concentrations at which it is used or administered. Other pharmaceutically acceptable ingredients can be present in the composition as well. Suitable substances and their use for the formulation of pharmaceutically active compounds are well-known in the art (see, for example, "Remington's Pharmaceutical Sciences", E. W. Martin, 19th Ed., 1995, Mack Publishing Co.: Easton, Pa., and more recent editions or versions thereof, such as Remington: The Science and Practice of Pharmacy. 21st Edition. Philadelphia, Pa. Lippincott Williams & Wilkins, 2005, for additional discussion of pharmaceutically acceptable substances and methods of preparing pharmaceutical compositions of various types). Furthermore, compounds and compositions of the invention may be used in combination with any compound or composition used in the art for treatment of a particular disease or condition of interest. A pharmaceutical composition is typically formulated to be compatible with its intended route of administration. For example, preparations for parenteral administration include sterile aqueous or non-aqueous solutions, suspensions, and emulsions. Aqueous carriers include water, alcoholic/aqueous solutions, emulsions or suspensions, including saline and buffered media, e.g., sodium chloride solution, Ringer's dextrose, dextrose and sodium chloride, lactated Ringer's. Examples of non-aqueous solvents are propylene glycol, polyethylene glycol, vegetable oils such as olive oil, and injectable organic esters such as ethyl oleate. fixed oils, polyethylene glycols, glycerine, propylene glycol or other synthetic solvents; preservatives, e.g., antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. pH can be adjusted with acids or bases, such as hydrochloric acid or sodium hydroxide. Such parenteral preparations can be enclosed in ampoules, disposable syringes or multiple dose vials made of glass or plastic.

For oral administration, compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like. Suitable excipients for oral dosage forms are, e.g., fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl cellulose, sodium carboxymethylcellulose, and/or polyvinylpyrrolidone (PVP).

For administration by inhalation, inventive compositions may be delivered in the form of an aerosol spray from a pressured container or dispenser that contains a suitable propellant, e.g., a gas such as carbon dioxide, a fluorocarbon, or a nebulizer. Liquid or dry aerosol (e.g., dry powders, large porous particles, etc.) can be used. The present invention also contemplates delivery of compositions using a nasal spray or other forms of nasal administration. For topical applications, pharmaceutical compositions may be formulated in a suitable ointment, lotion, gel, or cream containing the active components suspended or dissolved in one or more pharmaceutically acceptable carriers suitable for use in such comporisition. For local delivery to the eye, the pharmaceutically acceptable compositions may be formulated as solutions or micronized suspensions in isotonic, pH adjusted sterile saline, e.g., for use in eye drops, or in an ointment, or for intra-ocularly administration, e.g., by injection. Pharmaceutical compositions may be formulated for transmucosal or transdermal delivery. For transmucosal or transdermal administration, penetrants appropriate to the barrier to be permeated may be used in the formulation. Such penetrants are generally known in the art. Inventive pharmaceutical compositions may be formulated as suppositories (e.g., with conventional suppository bases such as cocoa butter and other glycerides) or as retention enemas for rectal delivery.

In some embodiments, a composition includes one or more agents intended to protect the active agent(s) against rapid elimination from the body, such as a controlled release formulation, implants, microencapsulated delivery system, etc. Compositions may incorporate agents to improve stability (e.g., in the gastrointestinal tract or bloodstream) and/or to enhance absorption. Compounds may be encapsulated or incorporated into particles, e.g., microparticles or nanoparticles. Biodegradable, biocompatible polymers can be used, such as ethylene vinyl acetate, polyanhydrides, polyglycolic acid, PLGA, collagen, polyorthoesters, polyethers, and polylactic acid. Methods for preparation of such formulations will be apparent to those skilled in the art. For example, and without limitation, a number of particle, lipid, and/or polymer-based delivery systems are known in the art for delivery of siRNA. The invention contemplates use of such compositions. Liposomes or other lipid-based particles can also be used as pharmaceutically acceptable carriers.

Pharmaceutical compositions and compounds for use in such compositions may be manufactured under conditions that meet standards, criteria, or guidelines prescribed by a regulatory agency. For example, such compositions and compounds may be manufactured according to Good Manufacturing Practices (GMP) and/or subjected to quality control procedures appropriate for pharmaceutical agents to be administered to humans and can be provided with a label approved by a government regulatory agency responsible for regulating pharmaceutical, surgical, or other therapeutically useful products.

Pharmaceutical compositions of the invention, when administered to a subject for treatment purposes, are preferably administered for a time and in an amount sufficient to treat the disease or condition for which they are administered. Therapeutic efficacy and toxicity of active agents can be assessed by standard pharmaceutical procedures in cell cultures or experimental animals. The data obtained from cell culture assays and animal studies can be used in formulating a range of dosages suitable for use in humans or other subjects. Different doses for human administration can be further tested in clinical trials in humans as known in the art. The dose used may be the maximum tolerated dose or a lower dose. A therapeutically effective dose of an active agent in a pharmaceutical composition may be within a range of about 0.001 mg/kg to about 100 mg/kg body weight, about 0.01 to about 25 mg/kg body weight, about 0.1 to about 20 mg/kg body weight, about 1 to about 10 mg/kg. Other exemplary doses include, for example, about 1 µg/kg to about 500 mg/kg, about 100 µg/kg to about 5 mg/kg. In some embodiments, a single dose is administered while in other embodiments multiple doses are administered. Those of ordinary skill in the art will appreciate that appropriate doses in any particular circumstance depend upon the potency of the agent(s) utilized, and may optionally be tailored to the particular recipient. The specific dose level for a subject may depend upon a variety of factors including the activity of the specific agent(s) employed, the particular disease or condition and its severity, the age, body weight, general health of the subject, etc. It may be desirable to formulate pharmaceutical compositions, particularly those for oral or parenteral compositions, in unit dosage form for ease of administration and uniformity of dosage. Unit dosage form, as that term is used herein, refers to physically discrete units suited as unitary dosages for the subject to be treated; each unit containing a predetermined quantity of active agent(s) calculated to produce the desired therapeutic effect in association with an appropriate pharmaceutically acceptable carrier. It will be understood that a therapeutic regimen may include administration of multiple doses, e.g., unit dosage forms, over a period of time, which can extend over days, weeks, months, or years. A subject may receive one or more doses a day, or may receive doses every other day or less frequently, within a treatment period. For example, administration may be biweekly, weekly, etc. Administration may continue, for example, until appropriate structure and/or function of a tissue or organ has been at least partially restored and/or until continued administration of the compound does not appear to promote further regeneration or improvement. In some embodiments, a subject administers one or more doses of a composition of the invention to him or herself.

In some embodiments, two or more compounds or compositions are administered in combination, e.g., for purposes of enhancing regeneration. Compounds or compositions administered in combination may be administered together in the same composition, or separately. In some embodiments, administration "in combination" means, with respect to administration of first and second compounds or compositions, administration performed such that (i) a dose of the second compound is administered before more than 90% of the most recently administered dose of the first agent has been metabolized to an inactive form or excreted from the body; or (ii) doses of the first and second compound are administered within 48, 72, 96, 120, or 168 hours of each other, or (iii) the agents are administered during overlapping time periods (e.g., by continuous or intermittent infusion); or (iv) any combination of the foregoing. In some embodiments, two or more iTR factors, or vectors expressing the catalytic component of telomerase and an iTR factor, are administered. In some embodiments an iTR factor is administered in combination with a combination with one or more growth factors, growth factor receptor ligands (e.g., agonists), hormones (e.g., steroid or peptide hormones), or signaling molecules, useful to promote regeneration and polarity. Of particular utility are organizing center molecules useful in organizing regeneration competent cells such as those produced using the methods of the present invention. In some embodiments, a growth factor is an epidermal growth factor family member (e.g., EGF, a neuregulin), a fibroblast growth factor (e.g., any of FGF1-FGF23), a hepatocyte growth factor (HGF), a nerve growth factor, a bone morphogenetic protein (e.g., any of BMP1-BMP7), a vascular endothelial growth factor (VEGF), a wnt ligand, a wnt antagonist, retinoic acid, NOTUM, follistatin, sonic hedgehog, or other organizing center factors.

### Sources of iTM and iCM Factors

iTM and iCM factors may be identified by exposing embryonic cells lacking markers of the EFT (such as, by way of nonlimiting example, stromal cells not expressing *COX7A1*) to a variety of agents and assaying for the induction of said markers such as *COX7A1* or reporter constructs such as GFP expressed using the *COX7A1* gene promoter. Since exosomes carry potent protein and RNA factors capable of reprogramming cells to confer new growth, migration and differentiation properties, we examined whether they are capable of reprogramming the developmental state of a cell, i.e. iTM and iCM. We therefore tested exosomes from adult cells for induction of adult genes in embryonic cells. We assessed total RNA expression profile using Illumina microarray analysis of a series of 15 hESC derived clonal embryonic progenitor cell lines and compared these to 18 primary endothelial cell lines (newborn to adult) obtained from various anatomical sites (not shown). We determined that exosomes from cells that have passed the EFT are capable of inducing the expression of *COX7A1* in embryonic cells previously lacking such expression, as well as maturing the cells using other markers described herein.

Those skilled in the art will recognize, or be able to ascertain using no more than routine experimentation, many equivalents to the specific embodiments of the invention described herein. The scope of the present invention is not intended to be limited to the Description or the details set forth therein. Articles such as "a", "an" and "the" may mean one or more than one unless indicated to the contrary or otherwise evident from the context. Certain of the inventive methods are often practiced using populations of cells, e.g., *in vitro* or *in vivo.* Thus, references to "a cell" should be understood as including embodiments in which the cell is a member of a population of cells, e.g., a population comprising or consisting of cells that are substantially genetically identical. However, the invention encompasses embodiments in which inventive methods is/are applied to an individual cell. Thus, references to "cells" should be understood as including embodiments applicable to individual cells within a population of cells and embodiments applicable to individual isolated cells.

Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process. It is contemplated that all embodiments described herein are applicable to all different aspects of the invention. It is also contemplated that any of the embodiments can be freely combined with one or more other such embodiments whenever appropriate. Furthermore, it is to be understood that the invention encompasses all variations, combinations, and permutations in which one or more limitations, elements, clauses, descriptive terms, etc., from one or more of the claims (whether original or subsequently added claims) is introduced into another claim (whether original or subsequently added). For example, any claim that is dependent on another claim can be modified to include one or more elements or limitations found in any other claim that is dependent on the same base claim, and any claim that refers to an element present in a different claim can be modified to include one or more elements or limitations found in any other claim that is dependent on the same base claim as such claim. Furthermore, where the claims recite a composition, the invention provides methods of making the composition, e.g., according to methods disclosed herein, and methods of using the composition, e.g., for purposes disclosed herein. Where the claims recite a method, the invention provides compositions suitable for performing the method, and methods of making the composition. Also, where the claims recite a method of making a composition, the invention provides compositions made according to the inventive methods and methods of using the composition, unless otherwise indicated or unless one of ordinary skill in the art would recognize that a contradiction or inconsistency would arise.

Where elements are presented as lists, e.g., in Markush group format, each subgroup of the elements is also disclosed, and any element(s) can be removed from the group. For purposes of conciseness, only some of these embodiments have been specifically recited herein, but the invention includes all such embodiments. It should also be understood that, in general, where the invention, or aspects of the invention, is/are referred to as comprising particular elements, features, etc., certain embodiments of the invention or aspects of the invention consist, or consist essentially of, such elements, features, etc.
Where numerical ranges are mentioned herein, the invention includes embodiments in which the endpoints are included, embodiments in which both endpoints are excluded, and embodiments in which one endpoint is included and the other is excluded. It should be assumed that both endpoints are included unless indicated otherwise. Furthermore, unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise. Where phrases such as "less than X", "greater than X", or "at least X" is used (where X is a number or percentage), it should be understood that any reasonable value can be selected as the lower or upper limit of the range. It is also understood that where a list of numerical values is stated herein (whether or not prefaced by "at least"), the invention includes embodiments that relate to any intervening value or range defined by any two values in the list, and that the lowest value may be taken as a minimum and the greatest value may be taken as a maximum. Furthermore, where a list of numbers, e.g., percentages, is prefaced by "at least", the term applies to each number in the list. For any embodiment of the invention in which a numerical value is prefaced by "about" or "approximately", the invention includes an embodiment in which the exact value is recited. For any embodiment of the invention in which a numerical value is not prefaced by "about" or "approximately", the invention includes an embodiment in which the value is prefaced by "about" or "approximately". "Approximately" or "about" generally includes numbers that fall within a range of 1% or in some embodiments 5% or in some embodiments 10% of a number in either direction (greater than or less than the number) unless otherwise stated or otherwise evident from the context (e.g., where such number would impermissibly exceed 100% of a possible value). A "composition" as used herein, can include one or more than one component unless otherwise indicated. For example, a "composition comprising an activator or a TR activator" can consist or consist essentially of an activator of a TR activator or can contain one or more additional components. It should be understood that, unless otherwise indicated, an inhibitor or a TR inhibitor (or other compound referred to herein) in any embodiment of the invention may be used or administered in a composition that comprises one or more additional components including the presence of an activator of a TR activator.

### Examples

Example 1. Low Throughput Screening of Adult Human Skin Fibroblasts for iTR factors using PCR to Assay Reduction of *COX7A1* expression as an iTR Marker.

Approximately 200 combinations of candidate iTR factors described herein were screened in low throughput conditions assaying for *COX7A1* expression by qPCR as a first assay. Conditions that showed marked reduction in *COX7A1* expression were used to prepare RNA for Illumina bead array-or RNA-sequencing-based transcriptomic analysis. The optimum condition that caused the greatest reduction in levels of *COX7A1* while maintaining viable cells was a treatment for the first 17 days of valproic acid 0.5mM, CHIR99021 at 10uM, RepSox at 10uM, Tranylpromine (Parnate) at 10uM, Forskolin at 50uM, TTNPB at 5uM - then for 14 more days with 3-Deazaneplanocin A (DZNep) at 100nM was added to the aforementioned cocktail - then for last seven days only PD0325901 at 1.0 uM and CHIR99021 at 10 uM were used. All factors were used in cell growth Medium (high glucose Dulbecco's Modified Eagle's Medium (DMEM) with 10% fetal bovine serum (FBS)).

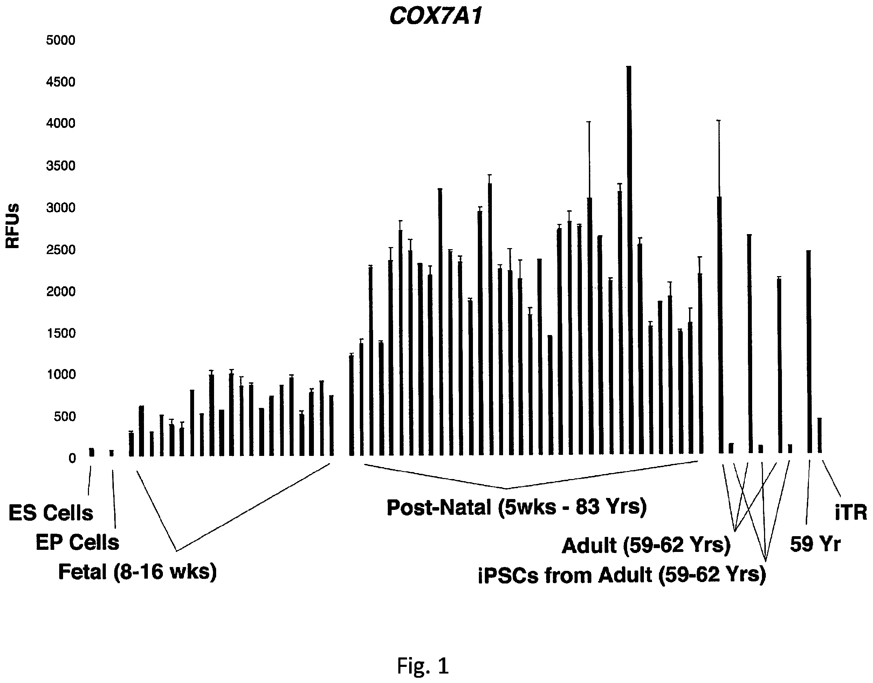

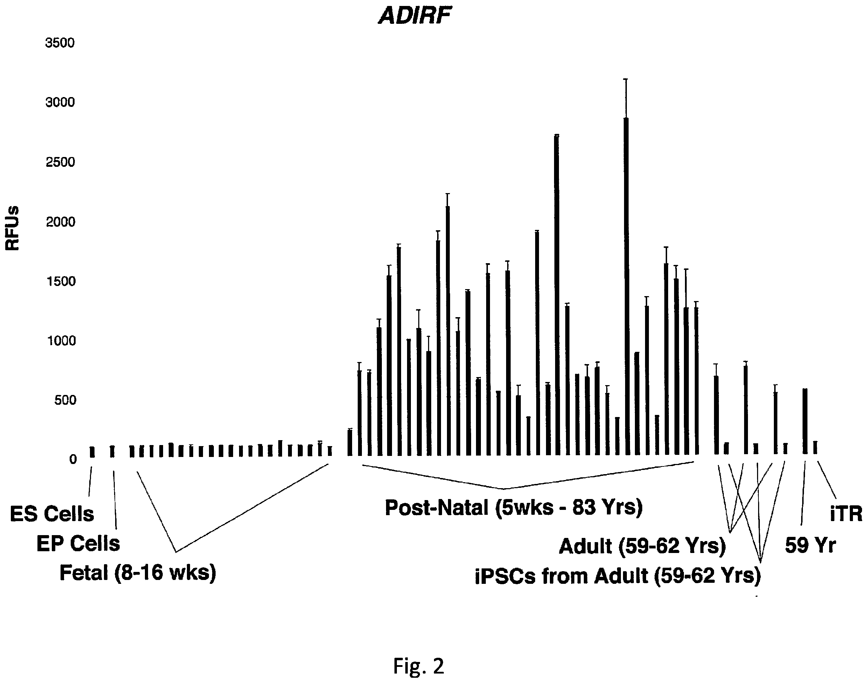

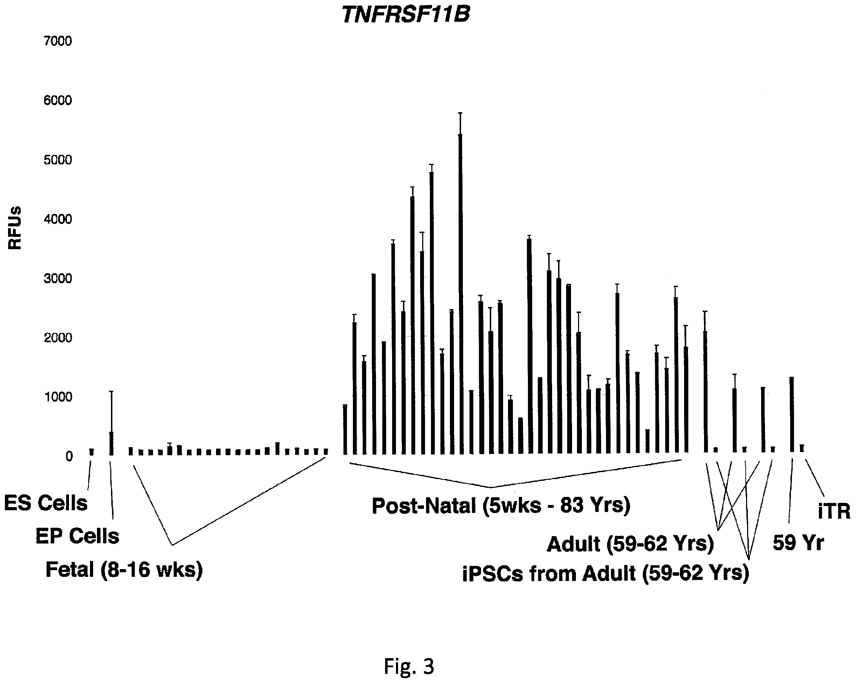

RNA was prepared from the cells in this iTR condition designated AgeX1547 and Illumina bead array analysis was performed. RFU values <100 were considered no expression. As shown in Figure 1, Illumina bead array analysis showed no *COX7A1* expression in hES cells or their derived hEP cell lines. However, in dermal skin fibroblast cultures at early passage, there was a progressive increase in *COX7A1* expression beginning at the earliest in vivo date tested (8 weeks of gestational development) that progressively increased even after the PPT to adulthood. Levels of COX7A1 were reversed back to essentially hES cell level (no expression) following iPS cell reprogramming, and the iTR cocktail AgeX1547 described above led to a reprogramming of *COX7A1* levels back to levels approximating 8 week (pre-fetal) development. As shown in Figure 2, *ADIRF* provided a marker of the PPT and again, AgeX1547 reverted the 59 year-old adult dermal fibroblasts back to a pre-fetal pattern of *ADIRF* gene expression. As shown in Figure 3, *TNFRSF11B* also provided a marker of PPT and and again, AgeX1547 reverted the 59 year-old adult dermal fibroblasts back to a pre-fetal pattern of *TNFRSF11B* gene expression. Since *TNFRSF11B* expression is implicated in blocking osteogenesis, the reversion of *TNFRSF11B* expression is consistent with a restoration of osteogenic potential in cells capable of forming new bone.

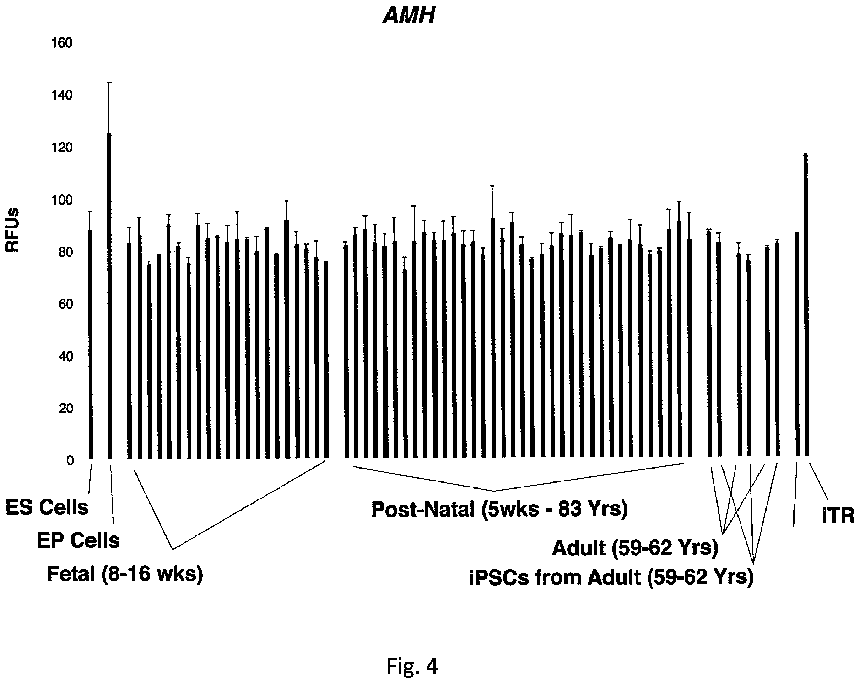

As shown in Figure 4, the embryonic marker *AMH* is expressed in hEP cell lines but is not expressed in fetal or adult skin fibroblasts, but following AgeX1547 treatement as described above, *AMH* expression is restored to an embryonic pattern of expression.

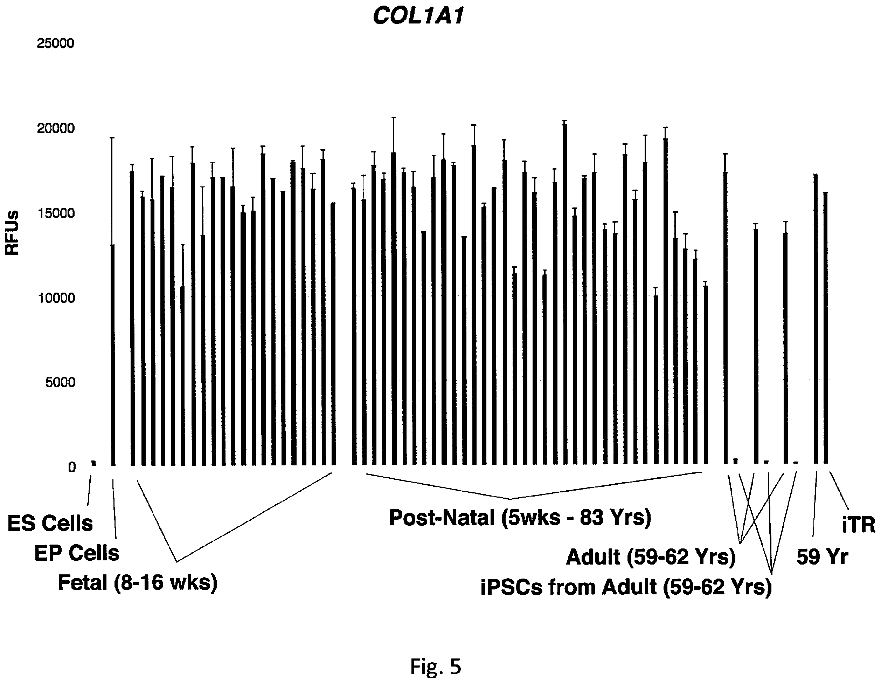

To determine whether the 59 year-old dermal fibroblasts reprogrammed by the iTR factor AgeX1547 were reverting to pluripotency which would be undesirable since the goal of iTR is to induce the regenerative senolytic phenotype without altering the cell differentiated state, we examined *COL1A1* expression. As shown in Figure 5, while hES cells showed markedly lower *COL1A1* expression compared to fetal and adult skin fibroblasts, and while the 59 year-old skin fibroblasts did repress *COL1A1* expression back to hES cell levels when reprogrammed to their iPSC counterpart, the 59 year-old skin fibroblasts treated with AgeX1547 did not repress *COL1A1* to the hES cell lives. Instead, the cells retained a fibroblastic morphology with no apparent signs of toxicity and *COL1A1* levels were comparable to normal dermal fibroblasts cultured in vitro.

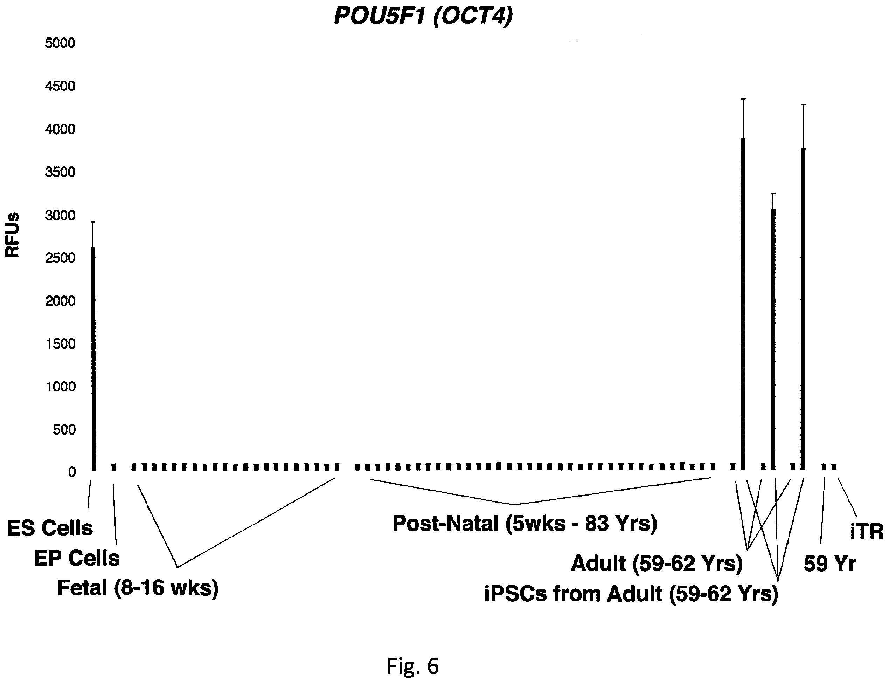

As shown in Figure 6, iPS cell reprogramming restored *OCT4* expression back to hES cell levels while AgeX1547 did not induce the pluripotency marker *OCT4* or other markers unique to pluripotent cells (not shown). Therefore, we conclude that AgeX1547 appears to be tolerated by human cells for the treatment period and appears to be capable of restoring the adult-derived cells back to a pattern of prenatal, indeed prefetal pattern of gene expression without otherwise altering their differentiated state (iTR).

Example 2. Screening for iTR factors capable of inducing senolysis in aged cells or CSCs.

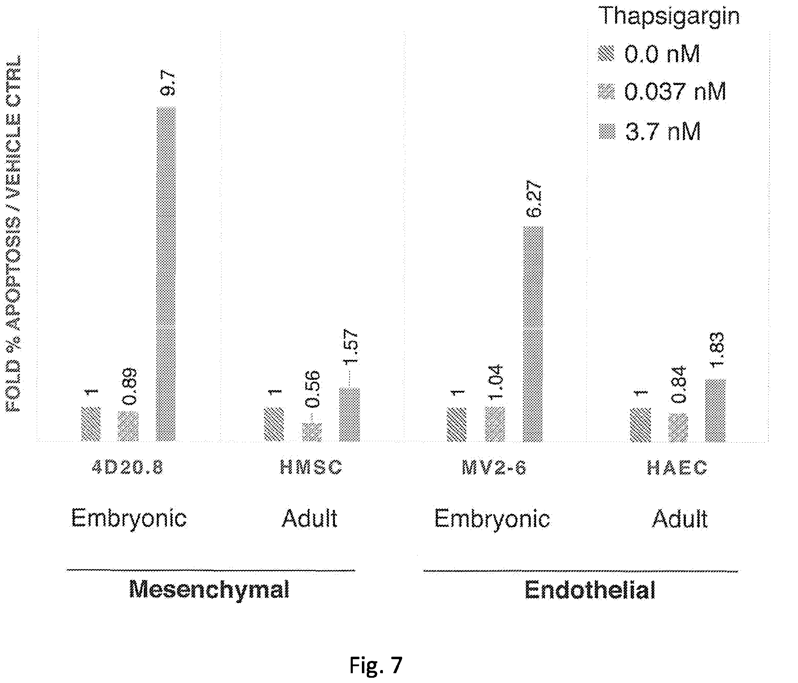

Optimized conditions were identified for distinguishing the increased apoptosis anticipated by cells with a pre-fetal pattern of gene expression. Embryonic and adult-derived mesenchymal (4D20.8 and MSCs respectively) and vascular endothelial cells (30MV2-6 and HAEC respectively) were treated with diverse stimuli for apoptosis including: Camptothecin (CPT), H₂O₂, and Thapsigargin (TG) at two concentrations each as well as a vehicle control. The compounds were applied for 24 hours prior to analysis. After treatment apoptosis was monitored via TUNEL staining using the In Situ Cell Death Detection kit (Roche, cat# 12156792910) following the manufacturer's instructions. As shown in Figure 7, thapsigargin at 3.7nM provided a robust statistically-significant increase in apoptosis in embryonic mesenchymal as well as endothelial cells compared to their adult counterparts. This assay is therefore useful in determining the effectiveness of iTR factors in inducing senolysis in aged cells and in CSCs.

Example 3. Method to prepare EGFP reporter for *COX7A1* expression used in high throughput iTR screens.

An IRES-EGFP was inserted downstream of *COX7A1* locus in *TERT-immortalized* human foreskin fibroblasts. To achieve this, two guide RNAs (gRNA) candidates were designed and cloned to target the *COX7A1* locus. A gRNA mediated CRISPR/Cas9 modification led to a targeted double-stranded break (DSB). A donor plasmid carrying the IRES-EGFP cassette was constructed based on the location of active gRNA, to serve as a DNA repair template. Co-transfection of gRNA and donor plasmid into *TERT*-immortalized human foreskin fibroblasts mediated homology-directed repair (HDR), allowing knock-in of IRES-EGFP at targeted region of *COX7A1* (Figure 7).

### Example 4.

In a first set of samples, human dermal fibroblasts (MDW1) at passages 5-8 were seeded at about 50,000 cells per well in 6 well Corning tissue culture plates in growth medium DMEM + 10% FBS.

The following day cells were treated with various cocktails shown below for various durations (27-42 days as shown).

Cocktails (in media: KnockOut DMEM supplemented with 10% KSR 10% FBS, 2mM GlutaMAX, 1% NEAA, 0.055 mM 2-mercaptoethanol, 10,000U/ml penicillin-streptomycin) and descriptive below.
1. MDW-1 P8 D41 CHIR99021 10uM + RepSox (10uM) + Tranylcypromine (parnate) 10uM + Forskolin 50uM + TTNPB 5uM + DZNep 100nM + PD0325901 1uM + FGF2 50ng/ml + EPZ004777 5uM + SGC0946 5uM + RSC133 10uM + CI994 1uM
2. MDW-1 P5 D39 CHIR99021 10uM + RepSox (10uM) + Tranylcypromine (parnate) 10uM + Forskolin 50uM + TTNPB 5uM + DZNep 100nM + PD0325901 1uM + FGF2 50ng/ml + OAC3 1uM + EPZ004777 5uM + SGC0946 5uM + RSC133 10uM + CI994 1uM + SB216763 20uM + SAHA 1uM + OCT4 250ng/ml + AM580 50nM
3. MDW-1 P5 D39 CHIR99021 10uM + RepSox (10uM) + Tranylcypromine (parnate) 10uM + Forskolin 50uM + TTNPB 5uM + DZNep 100nM + PD0325901 1uM + FGF2 50ng/ml + OAC3 1uM + EPZ004777 5uM + SGC0946 5uM + RSC133 10uM + CI994 1uM + SB216763 20uM + SAHA 1uM + OCT4 250ng/ml + AM580 50nM + PP1 10uM
4. MDW-1 P5 D42 CHIR99021 10uM + RepSox (10uM) + Tranylcypromine (parnate) 10uM + Forskolin 50uM + TTNPB 5uM + DZNep 100nM + PD0325901 1uM + FGF2 50ng/ml + EPZ004777 5uM + SGC0946 5uM + RSC133 10uM + CI994 1uM + SB216763 20uM + SAHA 1uM + AM580 50nM + PP1 10uM
5. MDW-1 P8 D42 MS-275 1uM + CHIR99021 10uM + RepSox (10uM) + Tranylcypromine (parnate) 10uM + Forskolin 50uM + TTNPB 5uM + DZNep 100nM + PD0325901 1uM + FGF2 50ng/ml + OAC3 1uM + EPZ004777 5uM + SGC0946 5uM + RSC133 10uM + CI994 1uM
6. MDW-1 P8 D27 MS-275 1uM + CHIR99021 10uM + RepSox (10uM) + Tranylcypromine (parnate) 10uM + Forskolin 50uM + TTNPB 5uM + DZNep 100nM + PD0325901 1uM + FGF 50ng/ml + OAC3 1uM + EPZ004777 5uM

Two controls were run (without cocktail) using "fasting medium" which DMEM with 0.5% FBS. The seeded cells were grown to confluence and then fed fasting medium for 3 days, then refed fasting medium for 2 more days and then lysed for RNA.

In a second set of samples, human dermal fibroblasts (MDW1) at passages 6-7 were seeded at about 50,000 cells per well in 6 well Corning tissue culture plates in growth medium DMEM + 10% FBS.

The following day the cells were treated with a cocktail containing BIX01294 2mM, then after 7 days Repsox 10uM, Dastinib 0.5uM and Kenpaullone 5uM were added for a total of 14 days in growth medium.

Also, a control was run (without cocktail) using "fasting medium" which is DMEM with 0.5% FBS. The seeded cells were grown to confluence and then fed fasting medium for 3 days, then refed fasting medium for 2 more days and then lysed for RNA.

For both the first and second sets of samples, the cells were then lysed with RLT (Qiagen, Valencia CA Cat #79216) and total RNA is extracted using Qiagen RNeasy mini kits (Qiagen, Cat # 74104) following manufacturers instructions. cDNA is prepared using SuperScript III first strand kits with random hexamers (Invitrogen, Carlsbad CA, Cat. 18080-051), following manufacturer's instructions. cDNA clean-up to remove nucleotides, primers, salts and polymerases is carried out using QIAquick PCR purification kits (Qiagen, Valencia CA Cat. #28104) following manufacturer's instructions.

Samples for testing (template) are prepared in standard Optical 96-well reaction plates (Applied Biosystems Carlsbad, CA, PN 4306737) consisting of 30ng of RNA equivalent of cDNA, 0.8uM per gene-specific custom oligonucleotide primer set (Invitrogen), ultra-pure distilled water (Invitrogen Cat. # 10977015), diluted 1:1 with 12.5ul of Power SYBR Green PCR Master Mix (Applied Biosystems Carlsbad, CA, Cat. # 4367659) incorporating AmpliTaq Gold DNA polymerase in a total reaction volume of 25ul. Real-Time qPCR was run using Applied Biosystems 7500 Real-Time PCR System employing SDSv2.3 software. Amplification conditions were set at 50□C for 2 min. (stage 1), 95□C for 10 min. (stage 2), 40 cycles of 95□C for 15 sec then 60□C for 1 min (stage 3), with a dissociation stage (stage 4) at 95□C for 15 sec, 60□C for 1 min, and 95□C for 15 sec. Ct values of amplicons were normalized to the average Ct value of GAPDH.

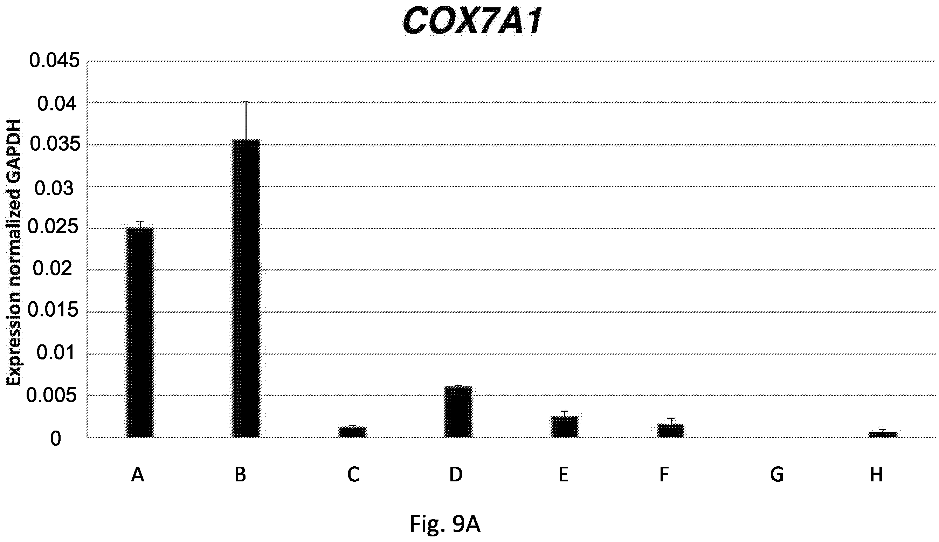

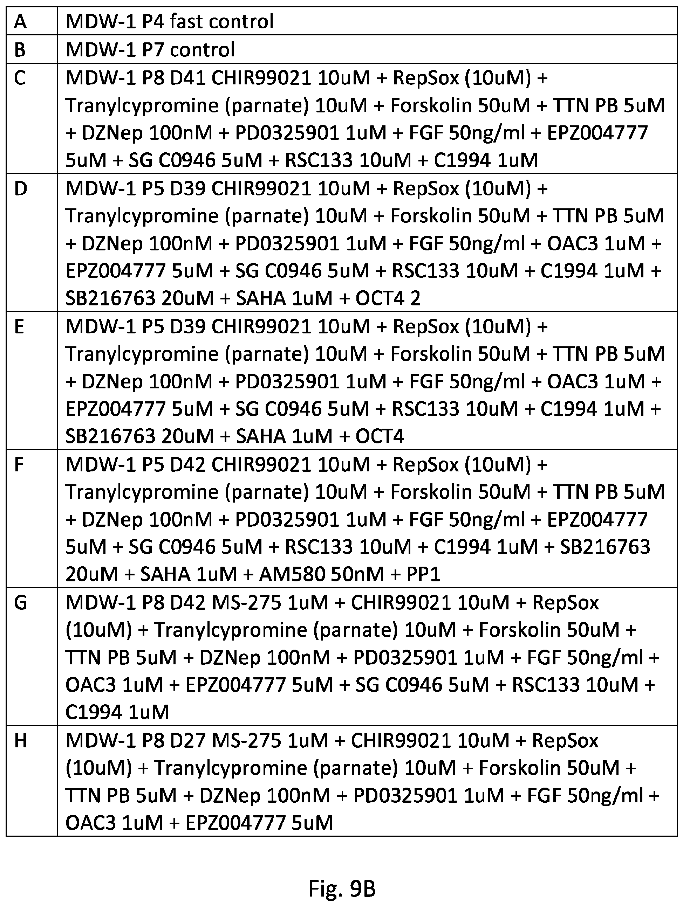

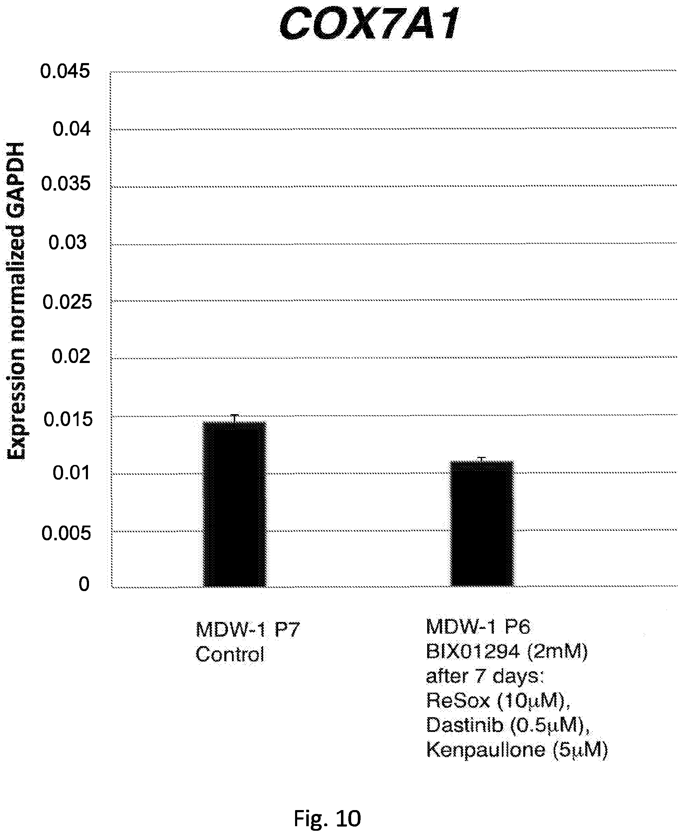

Results for the first set of samples are shown in Figure 9. Results for the second set of samples are shown in Figure 10. All combinations of iTR factors shown in Figures 9 and 10 significantly reduced *COX7A1* and other markers of EFT. The optimum combination was #5 above.

### Example 5. Scratch assay shows faster migration following cocktail treatment

BJ-TERT fibroblast cells at passage 8 were seeded at 50,000 cells/well on 6 well plates in DMEM 10% FBS. The following day the cells were treated with various cocktails listed below for 2 weeks:

### Control: Growth Medium only

A: LIN28A-11R (LD Biopharma HTF 0046) 4ug/ml
B: VALPROIC ACID (Cayman 13033) 0.5mM
C: LIN28A-11R 4ug/ml + VALPROIC ACID 0.5mM
D: MS-275 (Cayman 13284) 1uM + CHIR99021 (Cayman 13122) 10uM + RepSox (Sigma R0158) 10uM + Tranylcypromine (parnate) (Sigma 616431) 10uM + Forskolin (Cayman 11018) 50uM + TTNPB (Cayman 16144) 5uM + DZNep (Cayman 13128) 100nM + PD0325901 (Cayman 13034) 1uM + FGF2 (Peprotech AF-100-18B) 50ng/ml + OAC3 (Cayman 14104) 1uM + EPZ004777 (Cayman 16173) 5uM + SGC0946 (Cayman 13967) 5uM + RSC133 (Cayman 90018139) 10uM
E: LIN28A 4ug/ml + VPA 0.5mM, CHIR99021 10uM, RepSox 10uM, Parnate 10uM, Forskolin 50uM, TTNPB 5uM, + DZNep 100nM + PD0325901 1uM in hES medium (DMEM/F12 10% KSR, 10% FBS, 1% glutamax, 1% NEAA, 0.055mM 2-mercaptoethanol, bFGF 50ng/ml)

Following treatment, at confluence an 800um scratch was made and % filling evaluated 16 hours later. Filling of scratch of treated cells were compared to that of control. Data is shown below.

| | **A** | **B** | **C** | **D** | **E** |
|---|---|---|---|---|---|
| **Fold Increase** | | | | | |
| **Over Control** | **1.2** | **1.4** | **1.8** | **1.9** | **1.5** |

This study indicates that the reprogramming factors included here aid in cell migration and may be useful in healing injuries or in the regeneration of damaged tissue. In particular, conditions C, D and E showed 50% or more filling than control.

Example 6. Fibroblasts treated with reprogramming agents show senolytic activity toward late passage cells and reduced cell numbers.

Xgene fibroblasts (human foreskin, obtained from Xgene, Sausilito CA) were seeded at early (P9) and late (P33) passage at 20,000 cells/well (0.1% gelatin coated 24 well plate) in growth medium (DMEM + 10% FBS). The following day they were refed medium with the additive listed below for 1 week and fed every other day and then counted.

### Control No Additive,

A. LIN28A-11R (LD Biopharma HTF 0046) 4ug/ml,
B. Valproic Acid (Cayman 13033) 0.5mM,
C. LIN28A 4ug/ml + Valproic Acid 0.5mM
D. LIN28A 4ug/ml + VPA 0.5mM, CHIR99021 10uM (Cayman 13122), Repsox 10uM (Sigma R0158), Parnate 10uM (Sigma 616431), Forskolin 50uM (Cayman 11018), TTNPB 5uM (Cayman 16144), DZNep 100nM (Cayman 13128) and PD0325901 1uM (Cayman 13034).

The data is shown below:

| | **control** | | **Cond A** | | **Cond. B** | | **Cond. C** | |
|---|---|---|---|---|---|---|---|---|
| | **Xgene P9** | **Xgene P33** | **P9** | **P33** | **P9** | **P33** | **P9** | **P33** |
| | 184,000 | 56,000 | 220,000 | 46,000 | **228,000** | 62,000 | **296,000** | 55,000 |
| | 200,000 | 72,000 | 288,000 | **50,000** | **224,000** | 72,000 | **228,000** | 46,000 |
| **Avg** | **192,000** | **64,000** | **254,000** | **48,000** | **226,000** | 67,000 | **262,000** | **50,500** |
| **SD** | **11313** | **11313** | **48083** | **2828** | **2828** | **7071** | **48083** | **6363** |
| **Fold change** | | | **1.3** | **0.8** | **1.2** | **1.0** | **1.4** | **0.8** |

| | **Cond. D** | |
|---|---|---|
| | P9 | P33 |
| | 60,000 | 23,000 |
| | 52,000 | 20,000 |
| | **56,000** | **21,500** |
| | **5656.854249** | 2121.320344 |
| **CELL NUMBER:** | | |
| **FINAL/SEEDED** | **2.8** | **1.1** |

As expected, in growth medium alone the population doubling time of early passage fibroblasts (P9) is nearly 4x faster than at late passage (P33). Compared to controls (growth medium alone) the late passage cells displayed a reduced cell number in 1 week in when exposed to factors found in condition A (LIN28A) and condition C (LIN28A + VPA). This reduction, in the late passage cells, upon exposure to LIN28A with and without valproic acid, is due, at least in part, to senescent specific cell death (senolysis). In contrast, at early passage exposure to these agents led to heightened cell number. Treatment of cells with condition D, consisting of a more comprehensive cocktail of agents, caused a profound reduction in cell number which was greater at late passage, in part due to senolysis. Here we show that the cell number of the early passage cells increased 2.8 fold from that number seeded, while the late passage cells increased only 1.1 fold over that number seeded.

The reprogramming additives Valproic acid and LIN28A-11R used alone and together have a differential effect on late passage cells due to their senolytic behavior, and similarly, treatment with the inclusive reprogramming cocktail containing LIN28A 4ug/ml + VPA 0.5mM, CHIR99021 10uM, Repsox 10uM, Parnate 10uM, Forskolin 50uM, TTNPB, DZNep 100nM, and PD0325901 1uM led to a dramatic reduction of cells at late passage owing to their senolytic nature.

### Example 7. The use of Valproic Acid at lmM for iTR

Adult derived dermal fibroblasts were cultured in DMEM 10% FBS in wells of 6 well plates and treated with valproic acid at 0.5mM and ImM for 28 days. On day 28 the media was removed, the cells were washed with PBS, and then lysed using RLT with beta-mercaptoethanol (Qiagen Cat 79216). Total RNA was extracted using Qiagen RNeasy mini-kits (Qiagen Cat 74106) following manufacturers instructions. Then cDNA was prepared using ThermoFisher superscript III (Cat. 18080044). qPCR was run on an Applied Biosystems 7500 Real-Time PCR System employing SDSv2.3 software to evaluate the expression of COX7A1 (which is a definitive indicator of adult phenotype) relative to GAPDH of the treated line relative to the untreated line.

Unexpectedly, while a valproic acid concentration of 0.5mM had a negligible effect on COX7A1 expression (normalized to GAPDH) compared to control (untreated), there was a dramatic reduction of expression observed to near zero (0.001) noted at the ImM concentration.

## Claims

1. A set of compositions for altering the genetic profile of post-natal mammalian cells comprising:
a. a first composition comprising: 0.5 - 5.0mM valproic acid, 7 - l0µM 6- [[2-[[4-(2,4-Dichlorophenyl)-5-(5- methyl- lH-imidazol-2-yl)-2- pyrimidinyl] amino] ethyl] amino] -3 -pyridinecarbonitrile (CHIR99021), 4.0 - l0µM 2 -(3- (6-Methylpyridine-2-yl)-1H-pyrazol-4-yl)-1,5-naphthyridine (RepSox), 2.0 - l0µM Tranylpromine (Parnate), 0.5 - 50µM Forskolin and 1.0 - 5µM 4-[(E)-2-(5, 5,8,8-tetramethyl-6,7-dihydronaphthalen-2-yl)prop-l-enylbenzoic acid (TTNPB);
b. a second composition comprising: 0.5 - 5.0mM valproic acid, 7 - l0µM CHIR99021, 4.0 - l0µM RepSox, 2.0 - l0µM Tranylpromine (Parnate), 0.5 - 50µM Forskolin, 1.0 - 5µM TTNPB and 50nM - 240nM 3-Deazaneplanocin A (DZNep);
c. a third composition comprising: 0.1 - 1.0 µM N-[(2R)-2,3-dihydroxypropoxy] -3 ,4-difluoro-2-(2-fluoro-4-iodoanilino)benzamide (PD0325901) and 7.0 -10 µM 6-[[2-[[4-(2,4-Dichlorophenyl)-5-(5- methyl- lH-imidazol-2-yl)-2-pyrimidinyl] amino] ethyl] amino] -3 -pyridinecarbonitrile (CHIR99021 ); and
d. a fourth composition comprising an expression vector for expressing the gene TERT in mammalian cells.

2. The compositions of Claim 1, wherein said first, second and third compositions are in a cell culture medium.

3. The compositions of Claim 1, wherein said first, second and third compositions are in a hydrogel containing hyaluronic acid.

4. A set of compositions for altering the genetic profile of post-natal mammalian cells comprising:
a. a first composition comprising: 0.5 - 5.0mM valproic acid, 7 - l0µM 6- [ [2-[ [4-(2,4-Dichlorophenyl)-5 -(5- methyl- 1 H-imidazol-2-yl)-2- pyrimidinyl] amino] ethyl] amino] -3 -pyridinecarbonitrile (CHIR99021), 4.0 - l0µM 2 -(3- (6-Methylpyridine-2-yl)-lH-pyrazol-4-yl)-1,5-naphthyridine (RepSox), 2.0 - l0µM Tranylpromine (Parnate), 0.5 - 50µM Forskolin and 1.0 - 5µM 4-[(E)-2-(5, 5,8,8-tetramethyl-6,7-dihydronaphthalen-2-yl)prop-l-enyl]benzoic acid (TTNPB);
b. a second composition comprising: 0.5 - 5.0mM valproic acid, 7 - l0µM CHIR99021, 4.0 - l0µM RepSox, 2.0 - l0µM Tranylpromine (Parnate), 0.5 - 50µM Forskolin, 1.0 - 5µM TTNPB and 50nM - 240nM 3-Deazaneplanocin A (DZNep); and,
c. a third composition comprising: 0.1 - 1.0 µM N-[(2R)-2,3-dihydroxypropoxy] -3 ,4-difluoro-2-(2-fluoro-4-iodoanilino)benzamide (PD0325901) and 7.0 -10 µM 6-[[2-[[4-(2,4-Dichlorophenyl)-5-(5- methyl- lH-imidazol-2-yl)-2-pyrimidinyl] amino] ethyl] amino] -3 -pyridinecarbonitrile (CHIR99021 ).

5. The composition of Claim 4, wherein said first, second and third compositions are in a cell culture medium.

6. The composition of Claim 5, wherein said first, second and third compositions are in a hyaluronic acid-containing hydrogel.

7. A method to identify agents capable of inducing tissue regeneration (iTR), the method comprising: 1) culturing post-natal mammalian cells in a format that facilitates the exposure of said cells to one or more exogenous agents suspected of inducing tissue regeneration, 2) contacting said cells with said one or more exogenous agents and, 3) measuring the expression of genetic markers of embryonic-fetal transition (EFT) in said cells, wherein alteration of said expression is indicative of the ability of the agent to cause iTR.

8. The method of Claim 7, wherein genetic markers of embryonic-fetal transition (EFT) is selected from one of more of COX7AJ and ADIRF.

9. The method of Claim 7, wherein said genetic marker in COX7A1.

10. The method of Claim 7, wherein said post-natal mammalian cells are ZERr-immortalized adult human dermal fibroblast cells carrying a reporter gene signaling the EFT.

11. A method to identify agents capable of inducing tissue regeneration (iTR), the method comprising: 1) culturing post-natal mammalian cells in a format that facilitates the exposure of said cells to one or more exogenous agents suspected of inducing tissue regeneration, 2) contacting said cells with said one or more exogenous agents and, 3) preparing RNA from said cells and measuring the levels of embryonic-fetal transition (EFT) transcripts in said cells to determine whether said one or more agents cause iTR.

12. The method of Claim 11, wherein:
i.) genetic markers of embryonic-fetal transition (EFT) is selected from one of more of COX7AJ and ADIRF;
ii.) said genetic marker in COX7A1; or
iii.) said post-natal mammalian cells are TERT-immortalized adult human dermal fibroblast cells carrying a reporter gene signaling the EFT.

13. A method of altering the genetic profile of post-natal cells, the method comprising:
a. contacting one or more post-natal mammalian cells with a first composition comprising 0.5 - 5.0 mM valproic acid, 7 - l0µM CHIR99021, 4 - l0µM RepSox, 2.0 - l0µM Tranylpromine (Parnate), 0.5 - 50µM Forskolin, 1.0 - 5µM TTNPB for approximately 17 days;
b. contacting the one or more cells of step (a) with a second composition comprising 0.5 - 5.0 mM valproic acid, 7.0 -10µM CHIR99021, 4 - l0µM RepSox, 2.0 - l0µM Tranylpromine (Parnate), 0.5 - 50µM Forskolin, 1.0 - 5µM TTNPB and 50 - 240nM 3- Deazaneplanocin A (DZNep) for 14 days; and
c. contacting the one or more cells of step (b) with a third composition comprising 0.1 - 1.0 µM PD0325901 and 7.0 - 10 µM CHIR99021 for approximately 7 days.

14. The method of Claim 13, wherein:
i.) said one or more cells are considered to have an altered genetic profile when the expression of one of more of COX7A1, ADIRF and TNFRSF11B is decreased to expression levels associated with embryonic stem cells;
ii.) said one or more cells are considered to have an altered genetic profile when the expression of COX7A1 is decreased to expression levels associated with embryonic stem cells;
iii.) said first, second and third compositions are in a cell culture medium; or
iv.) said first, second and third compositions are in a hyaluronic acid-containing hydrogel.

## Patentansprüche

1. Satz von Zusammensetzungen zum Ändern des genetischen Profils von postnatalen Säugetierzellen, umfassend:
a. eine erste Zusammensetzung, die Folgendes umfasst: 0,5 - 5,0 mM Valproinsäure, 7 - 10 µM 6-[[2-[[4-(2,4-Dichlorphenyl)-5-(5-methyl-1H-imidazol-2-yl)-2-pyrimidinyl]amino]ethyl]amino]-3-pyridincarbonitril (CHIR99021), 4,0 - 10 µM 2-(3-(6-Methylpyridin-2-yl)-1H-pyrazol-4-yl)-1,5-naphthyridin (RepSox), 2,0 - 10 µM Tranylpromin (Parnat), 0,5 - 50 µM Forskolin und 1,0 - 5 µM 4-[(E)-2-(5,5,8,8-Tetramethyl-6,7-dihydronaphthalen-2-yl)prop-1-enyl]benzoesäure (TTNPB);
b. eine zweite Zusammensetzung, die Folgendes umfasst: 0,5 - 5,0 mM Valproinsäure, 7 - 10 µM CHIR99021, 4,0 - 10 µM RepSox, 2,0 - 10 µM Tranylpromin (Parnat), 0,5 -50 µM Forskolin, 1,0 - 5 µM TTNPB und 50 nM - 240 nM 3-Deazaneplanocin A (DZNep);
c. eine dritte Zusammensetzung, die Folgendes umfasst: 0,1 - 1,0 µM N-[(2R)-2,3-Dihydroxypropoxy]-3,4-difluor-2-(2-fluor-4-iodanilino)benzamid (PD0325901) und 7,0 - 10 µM 6-[[2-[[4-(2,4-Dichlorphenyl)-5-(5-methyl-1H-imidazol-2-yl)-2-pyrimidinyl]amino]ethyl]amino]-3-pyridincarbonitril (CHIR99021); und
d. eine vierte Zusammensetzung, die einen Expressionsvektor zum Exprimieren des Gens TERT in Säugetierzellen umfasst.

2. Zusammensetzungen nach Anspruch 1, wobei die erste, die zweite und die dritte Zusammensetzung in einem Zellkulturmedium vorliegen.

3. Zusammensetzungen nach Anspruch 1, wobei die erste, die zweite und die dritte Zusammensetzung in einem Hydrogel enthaltend Hyaluronsäure vorliegen.

4. Satz von Zusammensetzungen zum Ändern des genetischen Profils von postnatalen Säugetierzellen, umfassend:
a. eine erste Zusammensetzung, die Folgendes umfasst: 0,5 - 5,0 mM Valproinsäure, 7 - 10 µM 6-[[2-[[4-(2,4-Dichlorphenyl)-5-(5-methyl-1H-imidazol-2-yl)-2-pyrimidinyl]amino]ethyl]amino]-3-pyridincarbonitril (CHIR99021), 4,0 - 10 µM 2-(3-(6-Methylpyridin-2-yl)-1H-pyrazol-4-yl)-1,5-naphthyridin (RepSox), 2,0 - 10 µM Tranylpromin (Parnat), 0,5 - 50 µM Forskolin und 1,0 - 5 µM 4-[(E)-2-(5,5,8,8-Tetramethyl-6,7-dihydronaphthalen-2-yl)prop-1-enyl]benzoesäure (TTNPB);
b. eine zweite Zusammensetzung, die Folgendes umfasst: 0,5 - 5,0 mM Valproinsäure, 7 - 10 µM CHIR99021, 4,0 - 10 µM RepSox, 2,0 - 10 µM Tranylpromin (Parnat), 0,5 -50 µM Forskolin, 1,0 - 5 µM TTNPB und 50 nM - 240 nM 3-Deazaneplanocin A (DZNep); und
c. eine dritte Zusammensetzung, die Folgendes umfasst: 0,1 - 1,0 µM N-[(2R)-2,3-Dihydroxypropoxy]-3,4-difluor-2-(2-fluor-4-iodanilino)benzamid (PD0325901) und 7,0 - 10 µM 6-[[2-[[4-(2,4-Dichlorphenyl)-5-(5-methyl-1H-imidazol-2-yl)-2-pyrimidinyl]amino]ethyl]amino]-3-pyridincarbonitril (CHIR99021).

5. Zusammensetzung nach Anspruch 4, wobei die erste, die zweite und die dritte Zusammensetzung in einem Zellkulturmedium vorliegen.

6. Zusammensetzung nach Anspruch 5, wobei die erste, die zweite und die dritte Zusammensetzung in einem Hyaluronsäure enthaltenden Hydrogel vorliegen.

7. Verfahren zum Identifizieren von Agenzien, die in der Lage sind, eine Geweberegeneration zu induzieren (iTR), wobei das Verfahren Folgendes umfasst: 1) Kultivieren postnataler Säugetierzellen in einem Format, das die Exposition der Zellen gegenüber einem oder mehreren exogenen Agenzien, die im Verdacht stehen, eine Geweberegeneration zu induzieren, erleichtert, 2) Inkontaktbringen der Zellen mit dem einen oder den mehreren exogenen Agenzien und 3) Messen der Expression genetischer Marker des embryonal-fetalen Übergangs (EFT) in den Zellen, wobei eine Veränderung der Expression auf die Fähigkeit des Agens hinweist, iTR zu verursachen.

8. Verfahren nach Anspruch 7, wobei genetische Marker des embryonal-fetalen Übergangs (EFT) ausgewählt ist aus einem von mehreren von COX7AJ und ADIRF.

9. Verfahren nach Anspruch 7, wobei der genetische Marker COX7A1 in.

10. Verfahren nach Anspruch 7, wobei die postnatalen Säugetierzellen ZERr-immortalisierte adulte humane dermale Fibroblastenzellen sind, die ein Reportergen tragen, das den EFT signalisiert.

11. Verfahren zum Identifizieren von Agenzien, die in der Lage sind, eine Geweberegeneration zu induzieren (iTR), wobei das Verfahren Folgendes umfasst: 1) Kultivieren postnataler Säugetierzellen in einem Format, das die Exposition der Zellen gegenüber einem oder mehreren exogenen Agenzien, die im Verdacht stehen, eine Geweberegeneration zu induzieren, erleichtert, 2) Inkontaktbringen der Zellen mit dem einen oder den mehreren exogenen Agenzien und 3) Herstellen von RNA aus den Zellen und Messen der Niveaus von Transkripten des embryonal-fetalen Übergangs (EFT) in den Zellen, um zu bestimmen, ob das eine oder die mehreren Agenzien iTR verursachen.

12. Verfahren nach Anspruch 11, wobei:
i.) genetische Marker des embryonal-fetalen Übergangs (EFT) ausgewählt ist aus einem von mehreren von COX7AJ und ADIRF;
ii.) der genetische Marker COX7A1 in; oder
iii.) die postnatalen Säugetierzellen TERT-immortalisierte adulte humane dermale Fibroblastenzellen sind, die ein Reportergen tragen, das den EFT signalisiert.

13. Verfahren zum Ändern des genetischen Profils von postnatalen Zellen, wobei das Verfahren Folgendes umfasst:
a. Inkontaktbringen einer oder mehrerer postnataler Säugetierzellen mit einer ersten Zusammensetzung, die 0,5 - 5,0 mM Valproinsäure, 7 - 10 µM CHIR99021, 4 - 10 µM RepSox, 2,0 - 10 µM Tranylpromin (Parnat), 0,5 - 50 µM Forskolin, 1,0 - 5 µM TTNPB umfasst, für etwa 17 Tage;
b. Inkontaktbringen der einen oder mehreren Zellen aus Schritt (a) mit einer zweiten Zusammensetzung, die 0,5 - 5,0 mM Valproinsäure, 7.0 - 10 µM CHIR99021, 4 - 10 µM RepSox, 2,0 - 10 µM Tranylpromin (Parnat), 0,5 - 50 µM Forskolin, 1,0 - 5 µM TTNPB und 50 - 240 nM 3-Deazaneplanocin A (DZNep) umfasst, für etwa 14 Tage; und
c. Inkontaktbringen der einen oder mehreren Zellen aus Schritt (b) mit einer dritten Zusammensetzung, die 0,1 - 1,0 µM PD0325901 und 7,0 - 10 µM CHIR99021 umfasst, für etwa 7 Tage.

14. Verfahren nach Anspruch 13, wobei:
i.) die eine oder mehreren Zellen dafür angesehen werden, ein verändertes genetisches Profil aufzuweisen, wenn die Expression eines oder mehrerer von COX7A1, ADIRF und TNFRSF11B auf Expressionsniveaus verringert ist, die mit embryonalen Stammzellen assoziiert sind;
ii.) die eine oder mehreren Zellen dafür angesehen werden, ein verändertes genetisches Profil aufzuweisen, wenn die Expression von COX7A1 auf Expressionsniveaus verringert ist, die mit embryonalen Stammzellen assoziiert sind;
iii.) die erste, die zweite und die dritte Zusammensetzung in einem Zellkulturmedium vorliegen; oder
iv.) die erste, die zweite und die dritte Zusammensetzung in einem Hyaluronsäure enthaltenden Hydrogel vorliegen.

## Revendications

1. Ensemble de compositions permettant de modifier le profil génétique de cellules de mammifère post-natales comprenant :
a. une première composition comprenant : de l'acide valproïque 0,5 à 5,0 mM, du 6-[[2-[[4-(2,4-dichlorophényl)-5-(5-méthyl-1H-imidazol-2-yl)-2-pyrimidinyl]amino]éthyl]amino]-3-pyridinecarbonitrile (CHIR99021) 7 à 10 µM, de la 2-(3-(6-méthylpyridine-2-yl)-1H-pyrazol-4-yl)-1,5-naphthyridine (RepSox) 4,0 à 10 µM, de la tranylpromine (Parnate) 2,0 à 10 µM, de la forskoline 0,5 à 50 µM et de l'acide 4-[(E)-2-(5,5,8,8-tétraméthyl-6,7-dihydronaphthalén-2-yl)prop-1-ényl]benzoïque (TTNPB) 1,0 à 5 µM ;
b. une deuxième composition comprenant : de l'acide valproïque 0,5 à 5,0 mM, du CHIR99021 7 à 10 µM, de la RepSox 4,0 à 10 µM, de la tranylpromine (Parnate) 2,0 à 10 µM, de la forskoline 0,5 à 50 µM, du TTNPB 1,0 à 5 µM et de la 3-déazaneplanocine A (DZNep) 50 nM à 240 nM ;
c. une troisième composition comprenant : du N-[(2R)-2,3-dihydroxypropoxy]-3,4-difluoro-2-(2-fluoro-4-iodoanilino)benzamide (PD0325901) 0,1 à 1,0 µM et du 6-[[2-[[4-(2,4-dichlorophényl)-5-(5-méthyl-1H-imidazol-2-yl)-2-pyrimidinyl]amino]éthyl]amino]-3-pyridinecarbonitrile (CHIR99021) 7,0 à 10 µM ; et
d. une quatrième composition comprenant un vecteur d'expression destiné à exprimer le gène TERT dans des cellules de mammifère.

2. Compositions de la revendication 1, lesdites première, deuxième et troisième compositions étant dans un milieu de culture cellulaire.

3. Compositions de la revendication 1, lesdites première, deuxième et troisième compositions étant dans un hydrogel contenant de l'acide hyaluronique.

4. Ensemble de compositions permettant de modifier le profil génétique de cellules de mammifère post-natales comprenant :
a. une première composition comprenant : de l'acide valproïque 0,5 à 5,0 mM, du 6-[[2-[[4-(2,4-dichlorophényl)-5-(5-méthyl-1H-imidazol-2-yl)-2-pyrimidinyl]amino]éthyl]amino]-3-pyridinecarbonitrile (CHIR99021) 7 à 10 µM, de la 2-(3-(6-méthylpyridine-2-yl)-1H-pyrazol-4-yl)-1,5-naphthyridine (RepSox) 4,0 à 10 µM, de la tranylpromine (Parnate) 2,0 à 10 µM, de la forskoline 0,5 à 50 µM et de l'acide 4-[(E)-2-(5,5,8,8-tétraméthyl-6,7-dihydronaphthalén-2-yl)prop-1-ényl]benzoïque (TTNPB) 1,0 à 5 µM ;
b. une deuxième composition comprenant : de l'acide valproïque 0,5 à 5,0 mM, du CHIR99021 7 à 10 µM, de la RepSox 4,0 à 10 µM, de la tranylpromine (Parnate) 2,0 à 10 µM, de la forskoline 0,5 à 50 µM, du TTNPB 1,0 à 5 µM et de la 3-déazaneplanocine A (DZNep) 50 nM à 240 nM ; et,
c. une troisième composition comprenant : du N-[(2R)-2,3-dihydroxypropoxy]-3,4-difluoro-2-(2-fluoro-4-iodoanilino)benzamide (PD0325901) 0,1 à 1,0 µM et du 6-[[2-[[4-(2,4-dichlorophényl)-5-(5-méthyl-1H-imidazol-2-yl)-2-pyrimidinyl]amino]éthyl]amino]-3-pyridinecarbonitrile (CHIR99021) 7,0 à 10 µM.

5. Composition de la revendication 4, dans laquelle lesdites première, deuxième et troisième compositions sont dans un milieu de culture cellulaire.

6. Composition de la revendication 5, dans laquelle lesdites première, deuxième et troisième compositions sont dans un hydrogel contenant de l'acide hyaluronique.

7. Procédé d'identification d'agents capables d'induire une régénération tissulaire (iTR), le procédé comprenant : 1) la culture de cellules de mammifère post-natales dans un format qui facilite l'exposition desdites cellules à un ou plusieurs agents exogènes soupçonnés d'induire une régénération tissulaire, 2) la mise en contact desdites cellules avec lesdits un ou plusieurs agents exogènes et, 3) la mesure de l'expression de marqueurs génétiques de transition embryonnaire-fœtale (EFT) dans lesdites cellules, dans lequel la modification de ladite expression est indicative de la capacité de l'agent à provoquer l'iTR.

8. Procédé de la revendication 7, dans lequel les marqueurs génétiques de la transition embryonnaire-fœtale (EFT) est choisi parmi un ou plusieurs de COX7AJ et ADIRF.

9. Procédé de la revendication 7, dans lequel ledit marqueur génétique dans COX7A1.

10. Procédé de la revendication 7, dans lequel lesdites cellules de mammifère post-natales sont des cellules de fibroblastes dermiques humains adultes immortalisées par ZERr portant un gène rapporteur signalant l'EFT.

11. Procédé d'identification d'agents capables d'induire une régénération tissulaire (iTR), le procédé comprenant : 1) la culture de cellules de mammifère post-natales dans un format qui facilite l'exposition desdites cellules à un ou plusieurs agents exogènes soupçonnés d'induire une régénération tissulaire, 2) la mise en contact desdites cellules avec lesdits un ou plusieurs agents exogènes et, 3) la préparation d'ARN à partir desdites cellules et la mesure des niveaux de transcrits de transition embryonnaire-fœtale (EFT) dans lesdites cellules pour déterminer si lesdits un ou plusieurs agents provoquent l'iTR.

12. Procédé de la revendication 11, dans lequel :
i.) les marqueurs génétiques de la transition embryonnaire-fœtale (EFT) est choisi parmi un ou plusieurs de COX7AJ et ADIRF ;
ii.) ledit marqueur génétique dans COX7A1 ; ou
iii.) lesdites cellules de mammifère post-natales sont des cellules de fibroblastes dermiques humains adultes immortalisées par TERT portant un gène rapporteur signalant l'EFT.

13. Procédé de modification du profil génétique de cellules post-natales, le procédé comprenant :
a. la mise en contact d'une ou plusieurs cellules de mammifère post-natales avec une première composition comprenant de l'acide valproïque 0,5 à 5,0 mM, du CHIR99021 7 à 10 µM, de la RepSox 4,0 à 10 µM, de la tranylpromine (Parnate) 2,0 à 10 µM, de la forskoline 0,5 à 50 µM, du TTNPB 1,0 à 5 µM pendant environ 17 jours ;
b. la mise en contact de la ou des cellules de l'étape (a) avec une deuxième composition comprenant de l'acide valproïque 0,5 à 5,0 mM, du CHIR99021 7 à 10 µM, de la RepSox 4,0 à 10 µM, de la tranylpromine (Parnate) 2,0 à 10 µM, de la forskoline 0,5 à 50 µM, du TTNPB 1,0 à 5 µM et de la 3-déazaneplanocine A (DZNep) 50 à 240 nM pendant 14 jours ; et
c. la mise en contact de la ou des cellules de l'étape (b) avec une troisième composition comprenant du PD0325901 0,1 à 1,0 µM et du CHIR99021 7,0 à 10 µM pendant environ 7 jours.

14. Procédé de la revendication 13, dans lequel :
i.) lesdites une ou plusieurs cellules sont considérées comme possédant un profil génétique modifié lorsque l'expression d'un ou plusieurs parmi COX7A1, ADIRF et TNFRSF11B est réduite à des niveaux d'expression associés à des cellules souches embryonnaires ;
ii.) lesdites une ou plusieurs cellules sont considérées comme possédant un profil génétique modifié lorsque l'expression de COX7A1 est réduite à des niveaux d'expression associés à des cellules souches embryonnaires ;
iii.) lesdites première, deuxième et troisième compositions sont dans un milieu de culture cellulaire ; ou
iv.) lesdites première, deuxième et troisième compositions sont dans un hydrogel contenant de l'acide hyaluronique.
